# EUROPEAN PATENT SPECIFICATION

(11) **EP 3 538 559 B1**
(45) Date of publication and mention of the grant of the patent: **09.04.2025**
(21) Application number: 17870043.1
(22) Date of filing: 08.11.2017
(51) Int. Cl.: C07K 14/74, A61P 37/02, A61K 47/69, A61K 47/55, A61K 38/00, A61P 37/06, A61K 39/00, A61K 38/16, A61P 29/00, A61P 37/00

(54) **RECOMBINANT PMHC CLASS II MOLECULES**
REKOMBINANTE PMHC-KLASSE-II-MOLEKÜLE
MOLÉCULES DE CLASSE II DE PMHC DE RECOMBINAISON

(30) Priority: 09.11.2016 US 201662419947 P
(43) Date of publication of application: 18.09.2019
(73) Proprietor: UTI Limited Partnership, Calgary, AB T2L 1Y8 (CA)
(72) Inventor: SANTAMARIA, Pedro, Calgary, Alberta T3H 2S3 (CA)
(74) Representative: Sagittarius IP
(86) International application number: PCT/IB2017/001508
(87) International publication number: WO 2018/087597

(56) References cited:
- WO-A2-2014/080286
- WO-A2-2014/080286
- WO-A2-2015/063616
- WO-A2-2016/198932
- WO-A2-98/06749
- WO-A2-98/06749
- US-A1- 2015 209 446
- US-A1- 2015 344 586
- US-A1- 2015 344 586
- JI-HEE HA ET AL: "Immunoglobulin Fc Heterodimer Platform Technology: From Design to Applications in Therapeutic Antibodies and Proteins", FRONTIERS IN IMMUNOLOGY, vol. 7, 6 October 2016 (2016-10-06), pages 1 - 16, XP055377975, DOI: 10.3389/fimmu.2016.00394

## Description

### BACKGROUND

Major histocompatibility complex (MHC) class II molecules are normally found on antigen-presenting cells where they present antigen to cognate CD4+ T-cells helping to regulate immune responses. MHC class II molecules are formed by dimerization of an alpha and beta chain, and are stabilized in the presence of a polypeptide antigen that associates in a biding groove formed by the alpha and beta chain. However, production of MHC class II in engineered systems *in vitro* has been challenging due to the intrinsic instability of the protein heterodimers, even in the presence of a polypeptide.
WO 98/06749 discloses monovalent and multivalent fusion proteins comprising human Class II MHC binding domains in which substantially all of the C-terminal transmembrane and cytoplasmic domains have been replaced by coiled-coil dimerization domains, such as leucine zipper domains.
Hee Ha *et al.* (2016) discloses concepts and strategies for the generation of heterodimeric Fc proteins, and their application in the development of bispecific antibodies.
US 2015/344586 discloses a disulfine-linked multivalent multi-function protein containing MHC class I extracellular domain and β-microglobulin.
WO 2014/080286 discloses a method of treating multiple sclerosis (MS) or a related disorder by administering an antigen-MHC-nanoparticle complex, wherein the antigen is MS-related.
US 2015/209446 also discloses a method of treating multiple sclerosis (MS) or a related disorder by administering an antigen-MHC-nanoparticle complex, wherein the antigen is MS-related.
WO 2015/063616 discloses compositions and methods for promoting the formation, expansion and recruitment of TR1 cells and /or Breg cells in an antigen-specific manner and treating autoimmune diseases and disorders.

### SUMMARY

Disclosed herein are isolated and purified antigen-MHC heterodimers and efficient methods of making the same. The heterodimers are engineered to facilitate ease of production and isolation. Multiple methods are employed to increase production of isolated heterodimers. First, the alpha and beta chains are both separately fused to a portion of an IgG heavy chain, then the IgG is engineered with a knob-in-hole architecture (with one of the alpha or beta-chain IgG comprising a knob and the opposite alpha or beta-chain IgG comprising the hole). In some instances the heterodimer can be further stabilized by a cysteine trap between the antigen and a residue of the MHC class II binding groove. To employ a cysteine trap, the antigen can comprise an endogenous or engineered cysteine that forms a disulfide bond with an engineered cysteine on the alpha or beta chain of the MHC molecule that is in close proximity to the binding groove. This cysteine trap can further increase the stability of a peptide MHC molecule. Unexpectedly, the knob-in-hole architecture has been found to work in the absence of a heterologous dimerization domains that have been previously employed. In fact, a heterologous dimerization domain was found to be detrimental to formation of heterodimers.

Thus, according to the present invention there is provided a composition comprising at least one isolated heterodimer conjugated to a nanoparticle,
wherein the nanoparticle is non-liposomal and/or has a solid core and
wherein the isolated heterodimer comprises at least one first polypeptide, at least one second polypeptide and an autoimmune disease-relevant antigen, wherein the first polypeptide and the second polypeptide meet at an interface, wherein the interface of the first polypeptide comprises an engineered protuberance which is positionable in an engineered cavity in the interface of the second polypeptide,
wherein the engineered protuberance comprises an amino acid sequence that is at least 90% identical to the amino acid sequence of SEQ ID NO: 53,
wherein the engineered cavity comprises an amino acid sequence that is at least 90% identical to the amino acid sequence of SEQ ID NO: 51; wherein
   (i) the first polypeptide comprises a soluble MHC class II α1 domain, a soluble MHC class II α2 domain, or a combination thereof; and the second polypeptide comprises a soluble MHC class II β1 domain, a soluble MHC class II β2 domain, or a combination thereof; or
   (ii) the first polypeptide comprises a soluble MHC class II β1 domain, a soluble MHC class II β2 domain, or a combination thereof; and the second polypeptide comprises a soluble MHC class II α1 domain, a soluble MHC class II α2 domain, or a combination thereof; and

wherein the autoimmune disease-relevant antigen is a polypeptide which is at least 11 amino acids in length connected to the MHC class II α1 domain or the MHC class II β1 domain by a flexible linker; and
wherein neither the first polypeptide nor the second polypeptide comprises a leucine zipper domain, or an extraneous biotinylation site or purification tag.

In a further aspect of the invention, there is provided a pharmaceutical composition comprising the composition described herein, and a pharmaceutical excipient, stabilizer, or diluent.

In a yet further aspect, there is provided the pharmaceutical composition described herein for use in a method of treating an autoimmune disease or inflammatory condition.

In a still further aspect, there is provided the pharmaceutical composition described herein for use in a method of treating the human or animal body by therapy.

Provided herein, in one aspect, are isolated heterodimers comprising at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof. In some embodiments, the protuberance comprises one or more non-naturally occurring amino acid residues. In some embodiments, the protuberance comprises one or more naturally occurring amino acid residues. In some embodiments, the protuberance comprises one or more amino acids selected from phenylalanine, arginine, tyrosine, tryptophan, and cysteine. In some embodiments, the protuberance comprises an arginine residue. In some embodiments, the protuberance comprises a phenylalanine residue. In some embodiments, the protuberance comprises a tyrosine residue. In some embodiments, the protuberance comprises a tryptophan residue. In some embodiments, the protuberance comprises a cysteine residue. In some embodiments, the protuberance comprises a cysteine residue and a tryptophan residue. In some embodiments, the cavity comprises a non-naturally occurring amino acid residue. In some embodiments, the cavity comprises a naturally occurring amino acid residue. In some embodiments, the cavity comprises one or more amino acids selected from alanine, serine, threonine, valine, and cysteine. In some embodiments, the cavity comprises an alanine residue. In some embodiments, the cavity comprises a serine residue. In some embodiments, the cavity comprises a threonine residue.

In some embodiments, the cavity comprises a valine residue. In some embodiments, the cavity comprises a cysteine residue. In some embodiments, the cavity comprises a cysteine residue, a serine residue, an alanine residue, and a valine residue. In some embodiments, the first polypeptide and the second polypeptide interface via a C_{H}3 domain of an antibody. In some embodiments, the C_{H}3 domain is from an IgG. In some embodiments, the IgG is of the IgG1 subtype. In some embodiments, the first polypeptide and/or the second polypeptide further comprise a C-terminal cysteine residue. In some embodiments of the disclosure and not of the invention, the first polypeptide and/or the second polypeptide further comprise a biotinylation site. In some embodiments of the disclosure and not of the invention, the first polypeptide and/or the second polypeptide further comprise a Strep tag. In some embodiments, the isolated heterodimer comprises at least one polypeptide encoded by **SEQ ID NO: 1.** In some embodiments, the isolated heterodimer comprises at least one polypeptide encoded by **SEQ ID NO: 2.** In some embodiments, the isolated heterodimer comprises at least one polypeptide encoded by **SEQ ID NO: 3.** In some embodiments, the isolated heterodimer comprises at least one polypeptide encoded by **SEQ ID NO: 4.** In some embodiments, the isolated heterodimer comprises at least one polypeptide encoded by any one of **SEQ ID NOS: 5-8.** In some embodiments, the isolated heterodimer comprises at least one polypeptide encoded by a DNA sequence comprising any one of **SEQ ID NOS: 1-26, 64, or 65.** In some embodiments, the isolated heterodimer comprises at least one polypeptide comprising an amino acid sequence of any one of **SEQ ID NOS: 27-63,** or a fragment thereof.

Provided herein, in another aspect, are multimers comprising two or more isolated heterodimers described herein. In some embodiments, the multimer further comprises avidin.

In some embodiments, each of the two or more heterodimers is connected to the avidin. In some embodiments, the multimer further comprises a polymeric backbone, wherein each of the two or more heterodimers is connected to the polymeric backbone. In some embodiments, the polymeric backbone is dextran or polyethylene glycol (PEG).

Provided herein, in another aspect, are polypeptides comprising an MHC class II α1 domain, an MHC class II α2 domain, or a combination thereof; and at least one engineered protuberance. In some embodiments, the at least one engineered protuberance is not located at the MHC class II α1 domain or the MHC class II α2 domain. In some embodiments, the engineered protuberance is located at an antibody C_{H}3 domain fused to the polypeptide. In some embodiments, the polypeptide optionally comprises an antibody C_{H}2 domain located between an MHC class II α2 domain and the C_{H}3 domain with an engineered protuberance. In certain embodiments, the polypeptide comprises an antibody C_{H}3 domain, and the antibody C_{H}3 domain comprises at least one mutation selected from the list consisting of S354C, T366W, and both S354C and T366W (EU numbering).

Provided herein, in another aspect, are polypeptides comprising an MHC class II β1 domain, an MHC class II β2 domain, or a combination thereof; and at least one engineered protuberance. In some embodiments, the at least one engineered protuberance is not located at the MHC class II β1 domain or the MHC class II β2 domain. In some embodiments, the engineered protuberance is located at an antibody C_{H}3 domain fused to the polypeptide. In some embodiments, the polypeptide optionally comprises an antibody C_{H}2 domain located between an MHC class II β2 domain and the C_{H}3 domain with an engineered protuberance. In certain embodiments, the polypeptide comprises an antibody C_{H}3 domain, and the antibody C_{H}3 domain comprises at least one mutation selected from the list consisting of S354C, T366W, and both S354C and T366W (EU numbering).

In some embodiments, the protuberance comprises one or more non-naturally occurring amino acid residues. In some embodiments, the protuberance comprises one or more naturally occurring amino acid residues. In some embodiments, the protuberance comprises one or more amino acids selected from phenylalanine, arginine, tyrosine, tryptophan, and cysteine. In some embodiments, the protuberance comprises an arginine residue. In some embodiments, the protuberance comprises a phenylalanine residue. In some embodiments, the protuberance comprises a tyrosine residue. In some embodiments, the protuberance comprises a tryptophan residue. In some embodiments, the protuberance comprises a cysteine residue. In some embodiments, the protuberance comprises a cysteine residue and a tryptophan residue.

Provided herein, in another aspect, are polypeptides comprising an MHC class II α1 domain, an MHC class II α2 domain, or a combination thereof; and at least one engineered cavity. In some embodiments, the at least one engineered cavity is not located at the MHC class II α1 domain or the MHC class II α2 domain. In some embodiments, the engineered cavity is located at an antibody C_{H}3 domain fused to the polypeptide. In some embodiments, the polypeptide optionally comprises an antibody C_{H}2 domain located between an MHC class II α2 domain and the C_{H}3 domain with an engineered cavity. In certain embodiments, the polypeptide comprises an antibody C_{H}3 domain, and the antibody C_{H}3 domain comprises at least one mutation selected from the list consisting of Y349C, T366S, L368A, Y407V (EU numbering), and combinations thereof.

Provided herein, in another aspect, are polypeptides comprising an MHC class II β1 domain, an MHC class II β2 domain, or a combination thereof; and at least one engineered cavity. In some embodiments, the at least one engineered cavity is not located at the MHC class II β1 domain or the MHC class II β2 domain. In some embodiments, the engineered cavity is located at an antibody C_{H}3 domain fused to the polypeptide. In some embodiments, the polypeptide optionally comprises an antibody C_{H}2 domain located between an MHC class II β2 domain and the C_{H}3 domain with an engineered cavity. In certain embodiments, the polypeptide comprises an antibody C_{H}3 domain, and the antibody C_{H}3 domain comprises at least one mutation selected from the list consisting of Y349C, T366S, L368A, Y407V (EU numbering), and combinations thereof.

In some embodiments, the cavity comprises a non-naturally occurring amino acid residue. In some embodiments, the cavity comprises a naturally occurring amino acid residue. In some embodiments, the cavity comprises one or more amino acids selected from alanine, serine, threonine, valine, and cysteine. In some embodiments, the cavity comprises an alanine residue. In some embodiments, the cavity comprises a serine residue. In some embodiments, the cavity comprises a threonine residue. In some embodiments, the cavity comprises a valine residue. In some embodiments, the cavity comprises a cysteine residue. In some embodiments, the cavity comprises a cysteine residue, a serine residue, an alanine residue, and a valine residue.

In some embodiments, the protuberance is located at a C_{H}3 antibody constant domain. In some embodiments, the cavity is located at a C_{H}3 antibody constant domain. In some embodiments, the polypeptide further comprises a C-terminal cysteine residue.

Provided herein, in another aspect, are polypeptides encoded by a DNA sequence comprising any one of **SEQ ID NOS: 1-26, 64, or 65.**

Provided herein, in another aspect, are polypeptides comprising an amino acid sequence of any one of **SEQ ID NOS: 27-63,** or a fragment thereof.

Provided herein, in another aspect, are heterodimer-nanoparticle conjugates comprising at least one heterodimer described herein; and a nanoparticle, wherein the nanoparticle is non-liposomal and/or has a solid core. In some embodiments, the solid core is a gold or iron oxide core. In some embodiments, the at least one heterodimer is covalently or non-covalently linked to the nanoparticle. In some embodiments, the at least one heterodimer is covalently linked to the nanoparticle through a linker. In some embodiments, the linker comprises polyethylene glycol.

Also disclosed herein are methods of preparing a heterodimer comprising a first polypeptide and a second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof;
comprising the steps of: (a) culturing a host cell comprising nucleic acid encoding the first polypeptide and second polypeptide including the interfaces thereof, wherein the nucleic acid encoding the interface of the first polypeptide has been altered from nucleic acid encoding an original interface of the first polypeptide to encode the protuberance or the nucleic acid encoding the interface of the second polypeptide has been altered from nucleic acid encoding an original interface of the second polypeptide to encode the cavity or both, and wherein the culturing is such that the first polypeptide and second polypeptide are expressed; and (b) recovering the heterodimer from the host cell culture.

In some embodiments of the disclosure, the nucleic acid encoding the first polypeptide has been altered from the original nucleic acid to encode the protuberance and the nucleic acid encoding the second polypeptide has been altered from the original nucleic acid to encode the cavity.

In some embodiments of the disclosure, step (a) is preceded by a step wherein each of one or more nucleic acid encoding an original amino acid residue from the interface of the first polypeptide is replaced with nucleic acid encoding an import amino acid residue, wherein the protuberance comprises one or more import residues. In some embodiments, the import residue is selected from phenylalanine, arginine, tyrosine, tryptophan, and cysteine. In some embodiments, the import residue is arginine. In some embodiments, the import residue is phenylalanine.

In some embodiments, the import residue is tyrosine. In some embodiments, the import residue is tryptophan. In some embodiments, the import residue is cysteine.

In some embodiments of the disclosure, step (a) is preceded by a step wherein each of one or more nucleic acid encoding an original amino acid residue in the interface of the second polypeptide is replaced with nucleic acid encoding an import amino acid residue, wherein the cavity comprises one or more import residues. In some embodiments, the import residue is selected from cysteine, alanine, serine, threonine, or valine. In some embodiments, the import residue is cysteine.
In some embodiments, the import residue is alanine. In some embodiments, the import residue is serine. In some embodiments, the import residue is threonine. In some embodiments, the import residue is valine.

In some embodiments, the first polypeptide and the second polypeptide each comprise an antibody constant domain. In some embodiments, the antibody constant domain is a C_{H}3 domain. In some embodiments, the antibody constant domain is from an IgG. In some embodiments, the IgG is human IgG1. In some embodiments, the antibody constant domain is a C_{H}2 and a C_{H}3 domain. In certain embodiments, the C_{H}3 further comprises an engineered protuberance (knob) or cavity (hole).

Also disclosed herein are methods of preparing a heterodimer-nanoparticle conjugate comprising linking at least one heterodimer described herein to a nanoparticle, wherein the nanoparticle is non-liposomal and/or has a solid core. In some embodiments, the solid core is a gold or iron oxide core. In some embodiments of the disclosure, the linking step comprises covalently or non-covalently linking the at least one heterodimer to the nanoparticle. In some embodiments of the disclosure, the linking step comprises covalently linking the at least one heterodimer to the nanoparticle via a linker. In some embodiments, the linker comprises polyethylene glycol.

In another aspect, described herein is an isolated heterodimer comprising at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof. In certain embodiments, the protuberance comprises one or more non-naturally occurring amino acid residues. In certain embodiments, the protuberance comprises one or more amino acids selected from phenylalanine, arginine, tyrosine, tryptophan, and cysteine. In certain embodiments, the first or second polypeptide of the isolated heterodimer comprises an amino acid sequence that is at least 80% identical to the amino acid sequence set forth in **SEQ ID NO: 53.** In certain embodiments, the first or second polypeptide of the isolated heterodimer comprises an amino acid sequence at least 80% identical to **SEQ ID NO: 54.** In certain embodiments, the first or second polypeptide of the isolated heterodimer comprises a C_{H}3 domain and the C_{H}3 domain comprises at least one mutation selected from the list consisting of S354C, T366W, and both S354C and T366W (EU numbering). In certain embodiments, the cavity comprises a non-naturally occurring amino acid residue. In certain embodiments, the cavity comprises one or more amino acids selected from alanine, serine, threonine, valine, and cysteine. In certain embodiments, the first or second polypeptide of the isolated heterodimer comprises an amino acid sequence that is at least 80% identical to the amino acid sequence set forth in **SEQ ID NO: 51.** In certain embodiments, the first or second polypeptide of the isolated heterodimer comprises an amino acid sequence at least 80% identical to the amino acid sequence set forth in **SEQ ID NO: 52.** In certain embodiments, the first or second polypeptide of the isolated heterodimer comprises a C_{H}3 domain and the C_{H}3 domain comprises at least one mutation selected from the list consisting of Y349C, T366S, L368A, Y407V (EU numbering), and combinations thereof.

In certain embodiments of the disclosure, one or both of the first polypeptide or the second polypeptide does not comprise a heterologous dimerization domain. In certain embodiments of the disclosure, one or both of the first polypeptide or the second polypeptide does not comprise a leucine zipper. In certain embodiments of the disclosure, one or both of the first polypeptide or the second polypeptide does not comprise a site specific biotinylation site. In certain embodiments, the isolated heterodimer further comprises an autoimmune disease-relevant antigen. In certain embodiments, the disease-relevant antigen comprises a polypeptide at least 11 amino acids in length. In certain embodiments, the autoimmune disease-relevant antigen is connected to the MHC class II α1 domain or the MHC class II β1 domain by a flexible linker. In certain embodiments, the antigen is covalently connected to the MHC class II α1 domain or the MHC class II β1 domain by a disulfide bond formed between a cysteine amino acid associated with the antigenic peptide and a cysteine amino acid of the MHC class II α1 domain or the MHC class II β1 domain. In certain embodiments, the cysteine amino acid of the MHC class II α1 domain or the MHC class II β1 domain is within 10 amino acids of a residue that forms a part of an MHC class II binding groove. In certain embodiments, the cysteine residue of the MHC class II α1 domain or the MHC class II β1 domain is within 3 amino acids of a residue that forms a part of an MHC class II binding groove. In certain embodiments, the cysteine amino acid of the MHC class II α1 domain or the MHC class II β1 domain has been introduced into the naturally occurring sequence of the MHC class II α1 domain or the MHC class II β1 domain. In certain embodiments, the isolated heterodimer is for use in treating an individual diagnosed or suspected of having an autoimmune disease. In certain embodiments, the isolated heterodimer is encoded by a polynucleotide encoding the first or the second polypeptide described herein. In certain embodiments of the disclosure, a host cell comprises the polynucleotide. In certain embodiments of the disclosure, the polynucleotide is stably integrated into the genome. In certain embodiments, at least one heterodimer is conjugated to a nanoparticle to form a heterodimer-nanoparticle conjugate, wherein the nanoparticle is non-liposomal and/or has a solid core. In certain embodiments, the solid core is a gold, iron, or iron oxide core. In certain embodiments, the solid core has a diameter of less than 100 nanometers. In certain embodiments, the at least one heterodimer is covalently linked to the nanoparticle. In certain embodiments, the at least one heterodimer is covalently linked to the nanoparticle through a linker comprising polyethylene glycol (PEG). In certain embodiments, the polyethylene glycol is functionalized with maleimide. In certain embodiments, polyethylene glycol is less than 5 kD. In certain embodiments, a pharmaceutical composition comprising the heterodimer-nanoparticle conjugate is formed with a pharmaceutical excipient, stabilizer, or diluent. In certain embodiments, the isolated heterodimer or the pharmaceutical composition is for use in a method of treating an autoimmune disease or inflammatory condition. In certain embodiments of the disclosure, a method of treating an autoimmune disease or inflammatory condition comprises administering to an individual the isolated heterodimer-nanoparticle conjugate or the pharmaceutical composition.

Also disclosed herein is a method of preparing a heterodimer comprising a first polypeptide and a second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof; comprising the steps of: (a) culturing a host cell comprising a nucleic acid encoding the first polypeptide and second polypeptide including the interfaces thereof, wherein the nucleic acid encoding the interface of the first polypeptide has been altered from nucleic acid encoding an original interface of the first polypeptide to encode the protuberance or the nucleic acid encoding the interface of the second polypeptide has been altered from nucleic acid encoding an original interface of the second polypeptide to encode the cavity or both, and wherein the culturing is such that the first polypeptide and second polypeptide are expressed; and (b) recovering the heterodimer from the host cell culture. In certain embodiments of the disclosure, the nucleic acid encoding the first polypeptide and the second polypeptide is stably integrated into the genome of the host cell. In certain embodiments of the disclosure, the host cell comprises a Chinese hamster ovary (CHO) cell. In certain embodiments of the disclosure, recovering the heterodimer from the host cell culture or host cell cultures comprises applying a liquid comprising the heterodimer to liquid chromatography column. In certain embodiments of the disclosure, the liquid chromatography column comprises Protein A, Protein G, Protein L, or a combination thereof.

Also disclosed herein is a method of preparing a heterodimer comprising a first polypeptide and a second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof; comprising the steps of: (a) culturing a first host cell comprising a nucleic acid encoding the first polypeptide; (b) culturing a second host cell comprising a nucleic acid encoding the second polypeptide; (c) recovering the polypeptides from the host cell cultures; and (d) forming the heterodimer by incubating the first and second polypeptides together; wherein the nucleic acid encoding the interface of the first polypeptide has been altered from nucleic acid encoding an original interface of the first polypeptide to encode the protuberance or the nucleic acid encoding the interface of the second polypeptide has been altered from nucleic acid encoding an original interface of the second polypeptide to encode the cavity or both. In certain embodiments of the disclosure, the nucleic acid encoding the first polypeptide and the second polypeptide is stably integrated into the genome of the host cell. In certain embodiments of the disclosure, the first and second host cells comprise a Chinese hamster ovary (CHO) cell. In certain embodiments of the disclosure, the method further comprises recovering the heterodimer or the polypeptides from the host cell culture or host cell cultures comprises applying a liquid comprising the heterodimer or the polypeptides to a liquid chromatography column. In certain embodiments of the disclosure, the liquid chromatography column comprises Protein A, Protein G, Protein L, or a combination thereof.

Provided herein, in one aspect, are isolated heterodimers comprising at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, an IgG C_{H}2 domain, and an IgG C_{H}3 domain, wherein the protuberance is formed by mutations in the IgG C_{H}3 domain corresponding to S354C and T366W (EU numbering); and (ii) the second polypeptide comprises an MHC class II β1 domain, an MHC class II β2 domain, an IgG C_{H}2 domain, and an IgG C_{H}3 domain, wherein the cavity is formed by mutations in the IgG C_{H}3 domain corresponding to Y349C, T366S, L368A and Y407V (EU numbering).

Provided herein, in one aspect, are isolated heterodimers comprising at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II β1 domain, an MHC class II β2 domain, an IgG C_{H}2 domain, and an IgG C_{H}3 domain, wherein the protuberance is formed by mutations in the IgG C_{H}3 domain corresponding to S354C and T366W (EU numbering); and (ii) the second polypeptide comprises an MHC class II α1 domain, an MHC class II α2 domain, an IgG C_{H}2 domain, and an IgG C_{H}3 domain, wherein the cavity is formed by mutations in the IgG C_{H}3 domain corresponding to Y349C, T366S, L368A and Y407V (EU numbering).

### BRIEF DESCRIPTION OF THE DRAWINGS

**FIGS. 1A-B** show a lentiviral vector in both linearized **(A)** and circular form **(B).**
**FIG. 2** shows an example of pMHC engineering using a leucine zipper.

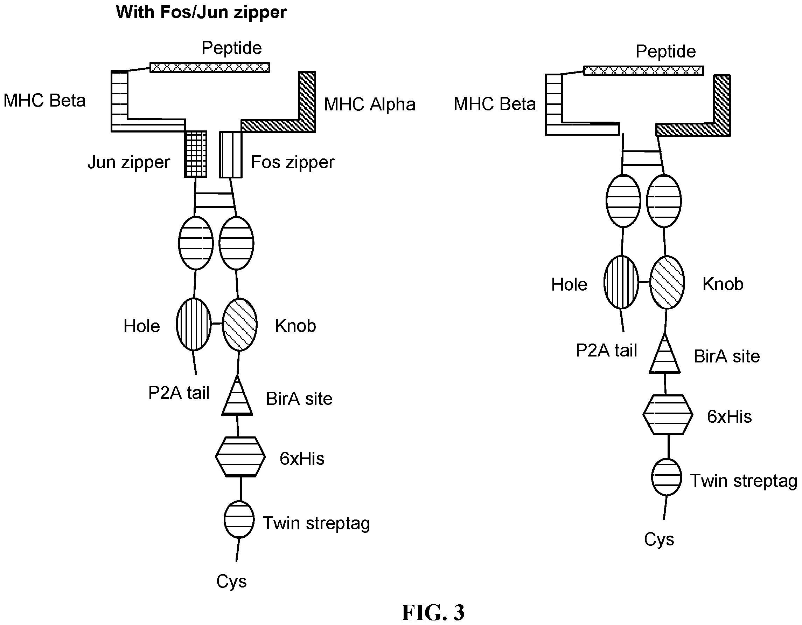

**FIG. 3** shows schematic knob-in-hole pMHC designs (with and without the leucine zipper).

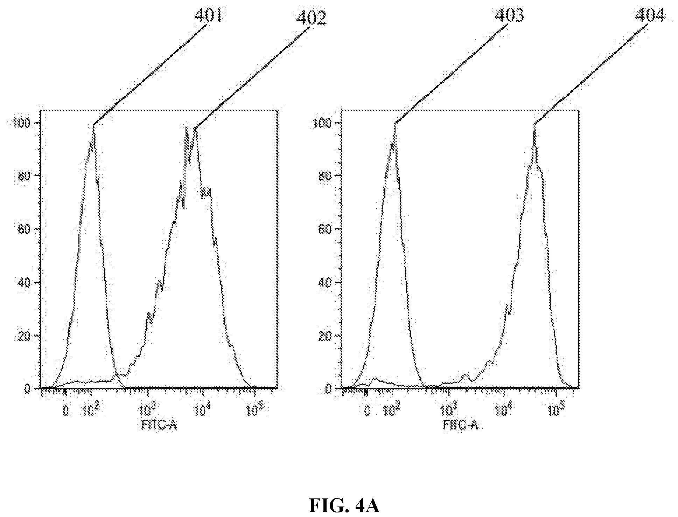

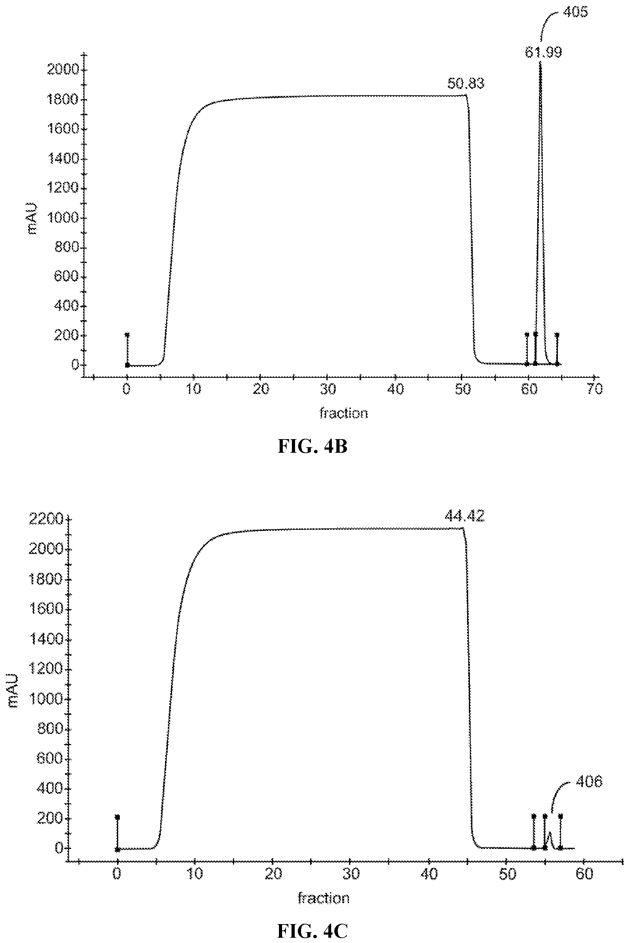

**FIGS. 4A-C** **FIG. 4A** shows flow cytometric analysis of eGFP expression in CHO cells transduced with lentiviruses encoding BDC2.5mi/IA^{g7}-knob-in-hole pMHCs with (left) or without (right) the leucine zipper. **FIG. 4B** and **FIG. 4C** show protein G affinity chromatography elution profiles of culture supernatants from CHO cells expressing knob-in-hole-based pMHCs without the leucine zipper **(****FIG. 4B****),** and with the leucine zipper **(****FIG. 4C****).**

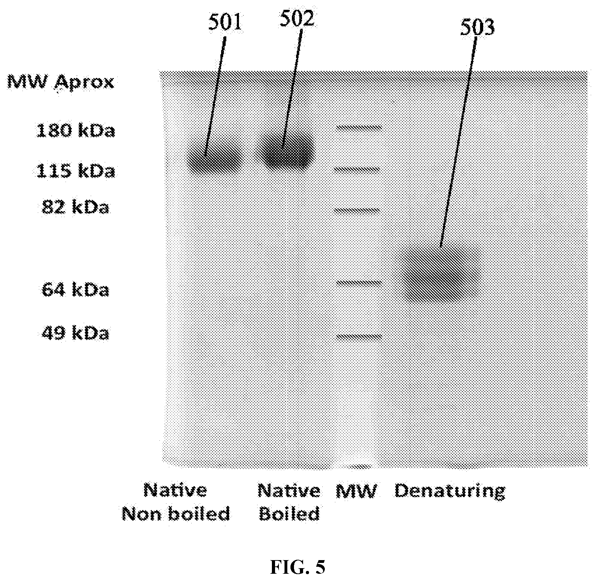

**FIG. 5** shows electrophoretic mobility of zipperless knob-in-hole-based pMHCs by native (left) and denaturing (right) SDS-PAGE.

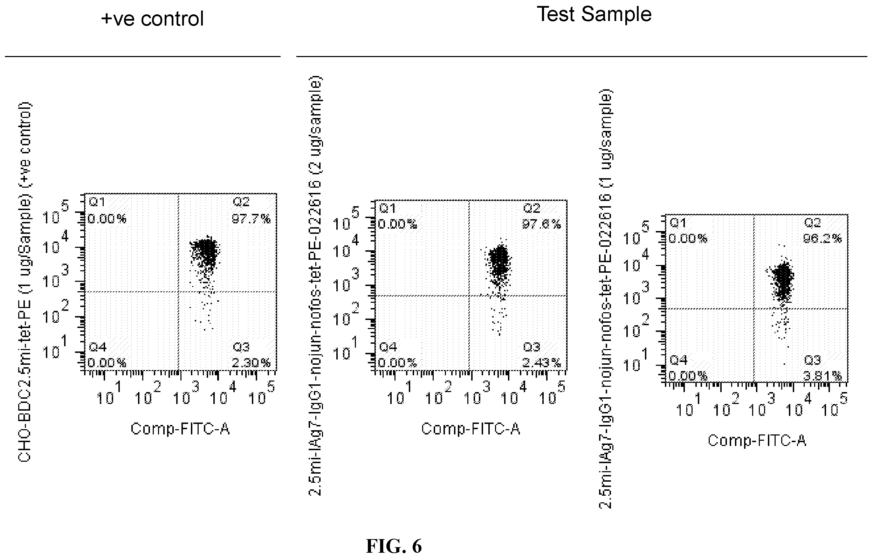

**FIG. 6** shows FACS staining profiles of BDC2.5-CD4+ T-cells using tetramers made with conventional pMHC monomers (left panel), or a zipperless knob-in-hole-based design (middle and right panels).

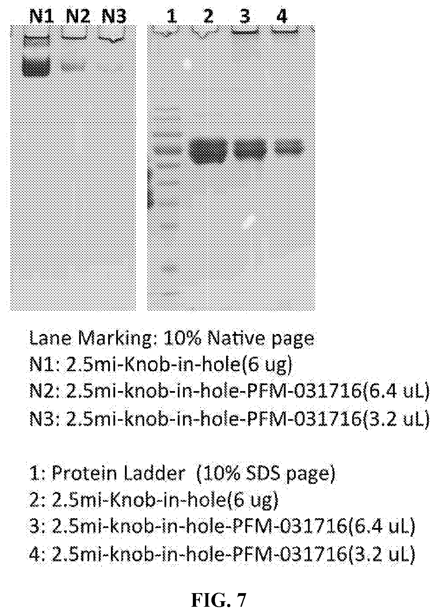

**FIG. 7** shows non-denaturing (left) and denaturing (right) SDS-PAGE gel images of PFM nanoparticles conjugated with zipperless BDC2.5mi/IA^{g7} knob-in-hole pMHCs.

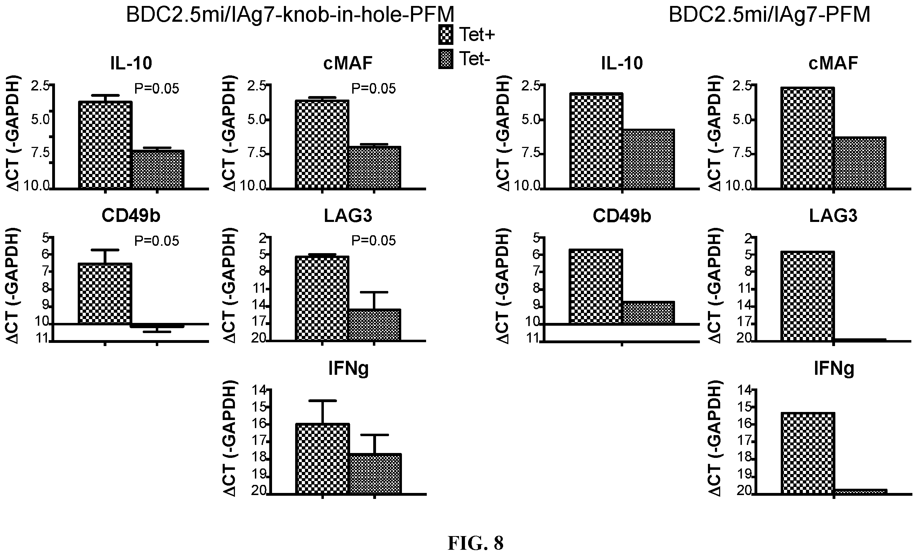

**FIG. 8** shows T-cell responses from mice treated with 2.5mi/IA^{g7}-zipperless knob-in-hole nanoparticles (left) or zippered conventional (non-knob-in-hole-based) 2.5mi/A^{g7}-nanoparticles (right). Mice were treated with 10 doses (2 doses per week for 5 weeks), after which splenic T-cells were isolated, sorted into 2.5mi/IA^{g7} tetramer positive and negative fractions, and stimulated with anti-CD3/anti-CD28 mAb coated beads. mRNA was assayed for the presence of TR₁ cell relevant transcripts from tetramer positive and negative fractions.

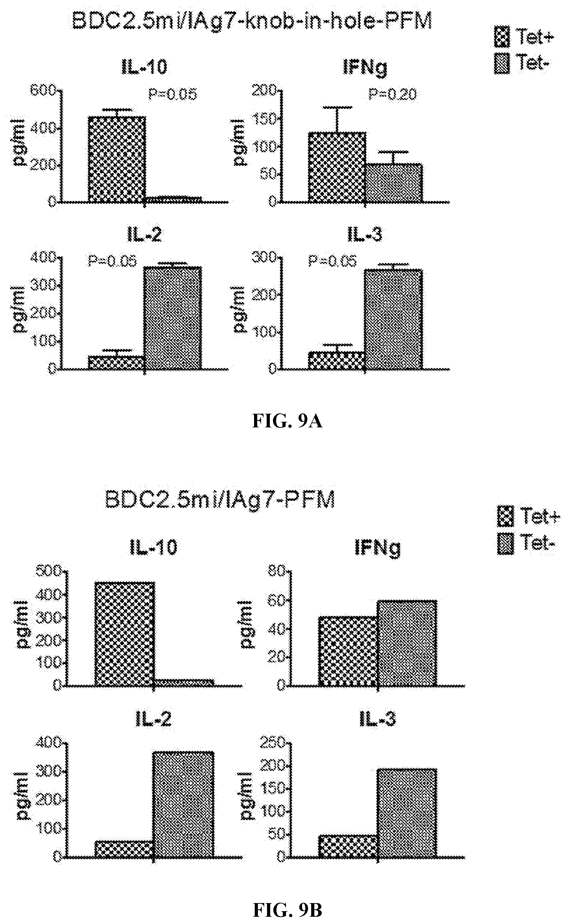

**FIGS. 9A-B** show cytokine release from T-cells isolated from mice treated as in **FIG. 8****.** Mice were treated with either 2.5mi/IA^{g7}-zipperless knob-in-hole nanoparticles **(A),** or zippered conventional (non-knob-in-hole-based) 2.5mi/A^{g7}-nanoparticles **(B).**

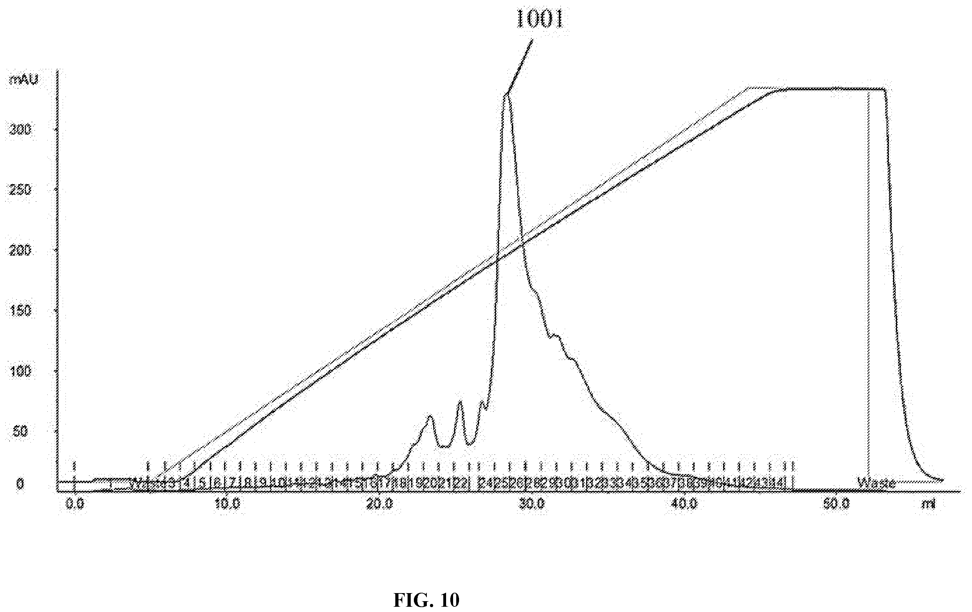

**FIG. 10** shows mono-Q purification profile of BDC-PEG-biotin protein. Fraction numbers 19 to 38 were collected for biotin screening ELISA and SDS page analysis.

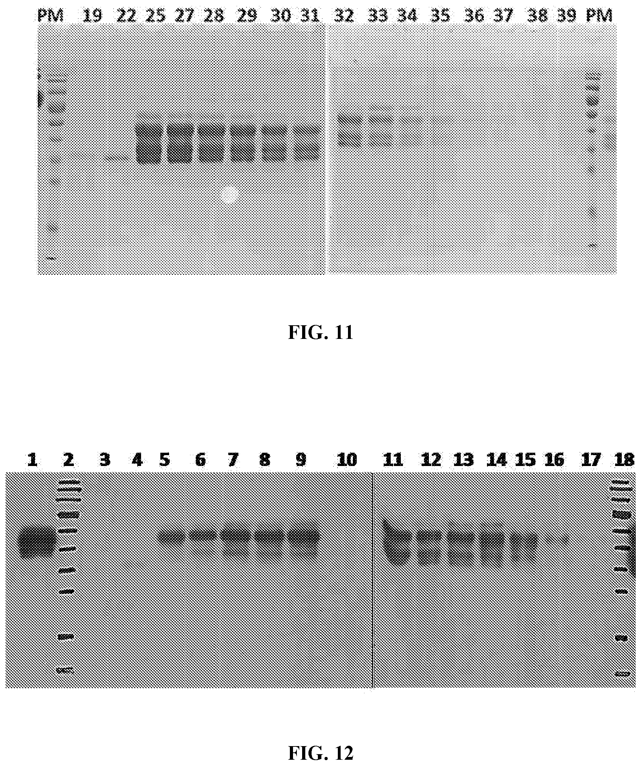

**FIG. 11** shows SDS-PAGE profiles of mono-Q purified fractions. The lanes are labeled with the number of each FPLC fraction. 20 µL of each fraction were loaded on the gel.
**FIG. 12** shows western blot analysis of fractions 22-25 (lanes 5-9) showed presence of biotin exclusively in the alpha chain of the pMHC monomer, as compared to BDC2.5mi/IA^{g7} pMHC monomer carrying a biotinylation sequence in the alpha chain and biotinylated using the BirA enzyme (lane 1). Fractions #25-29 were pooled and used to prepare pMHC tetramers. Lanes 11-17 show fractions 30-36.

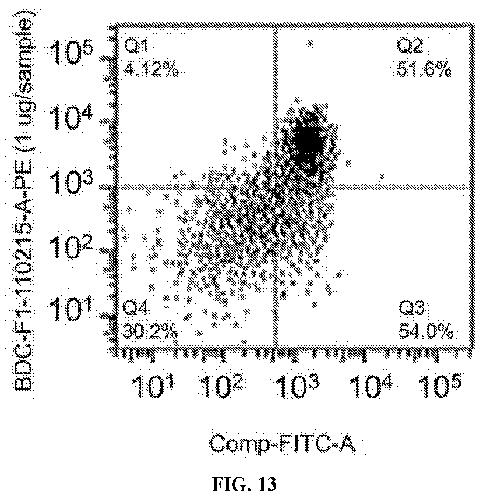

**FIG. 13** shows flow cytometry profile of BDC2.5-CD4+ T-cells stained with tetramers produced using BDC2.5mi/I-A^{g7}-maleimide-PEG2-biotin.

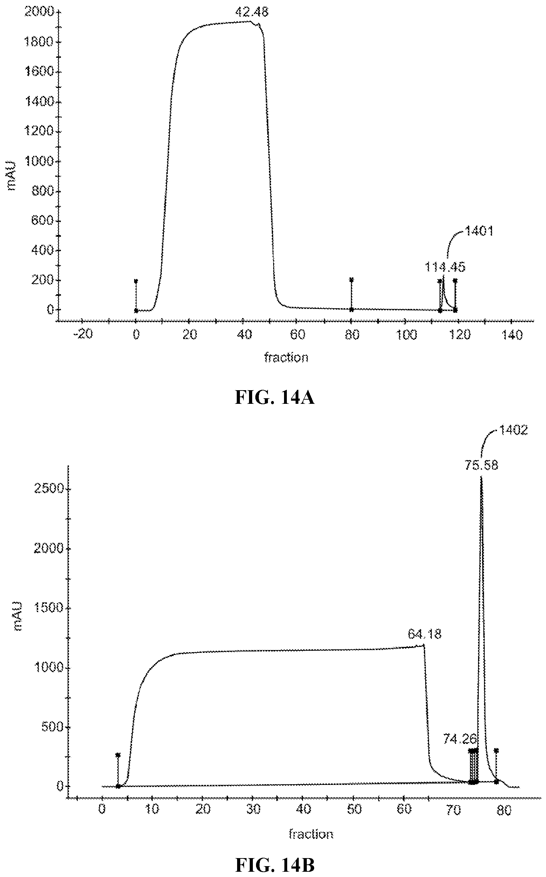

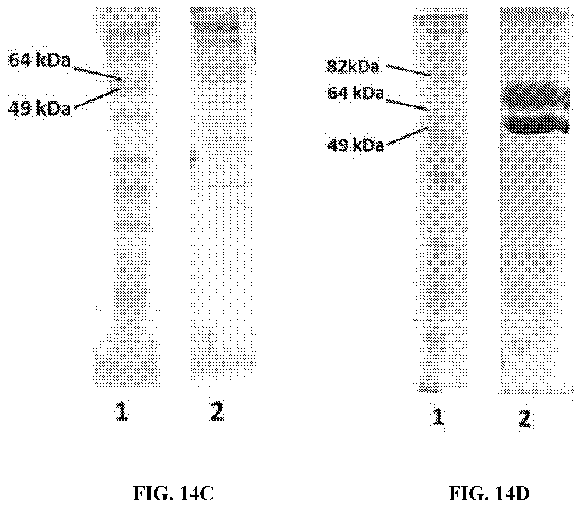

**FIG. 14A** and **FIG. 14B** show FPLC elution profiles, **FIG. 14C** and **FIG. 14D** show SDS-PAGE analysis of the eluted fractions indicated in **FIG. 14A** and **FIG. 14B****.**

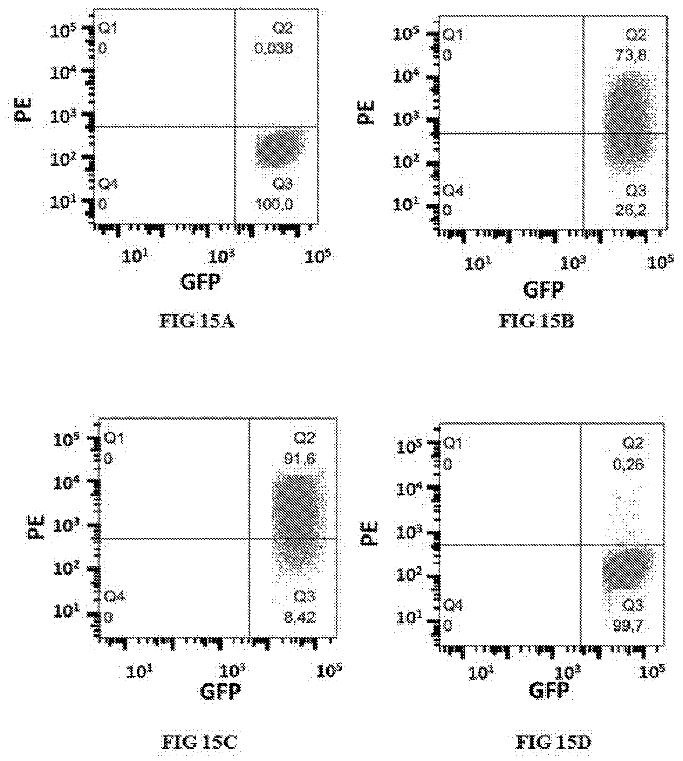

**FIG. 15A-D** shows flow cytometry of GFP labeled JURMA cells expressing a TCR specific for DR complexed with the IGRP₁₃₋₂₅ polypeptide. **FIG. 15A** shows cell line by itself; **FIG. 15B** shows cell line incubated with PE labeled DR3 IGRP₁₃₋₂₅ made by standard leucine zipper dimerization technology; **FIG. 15C** shows cell line incubated with PE labeled DR3 IGRP₁₃₋₂₅ made using knob-in-hole and cys-trap dimerization technology, lacking a leucine zipper; **FIG. 15D** shows cell line incubated with irrelevant PE labeled MHC class II heterodimers.

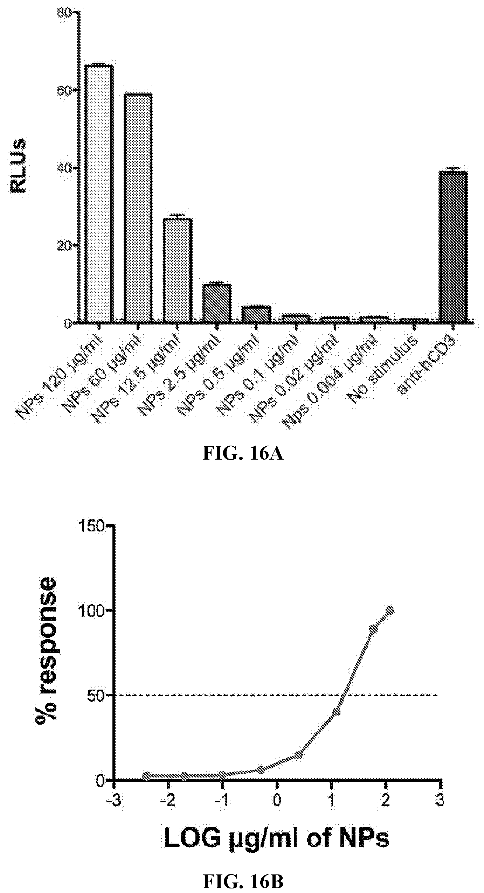

**FIG. 16A** and **FIG. 16B** show stimulation of JURMA cells expressing a TCR specific for DR complexed with the IGRP₁₃₋₂₅ polypeptide conjugated to a nanoparticle.

### DETAILED DESCRIPTION

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s).

Provided herein, in one aspect, are isolated heterodimers comprising at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof. Either the first polypeptide, the second polypeptide or both can comprise an antibody C_{H}3 domain fused to the polypeptide. Optionally, either the first polypeptide, the second polypeptide or both comprise an antibody C_{H}2 domain located between the MHC (α or β chain) and the C_{H}3 domain. In certain embodiments, the first polypeptide comprises an antibody C_{H}3 domain, and the antibody C_{H}3 domain comprises at least one mutation selected from the list consisting of S354C, T366W, and both S354C and T366W (EU numbering). In certain embodiments, the second polypeptide comprises an antibody C_{H}3 domain, and the antibody C_{H}3 domain comprises at least one mutation selected from the list consisting of Y349C, T366S, L368A, Y407V (EU numbering), and combinations thereof. In further embodiments, the isolated heterodimer comprises an autoimmune or inflammatory-disease relevant peptide, optionally covalently bound to either the first or the second polypeptide. Optionally, the autoimmune or inflammatory-disease relevant peptide comprises a cysteine residue that interacts with a cysteine residue in either the first or second polypeptide to create a cysteine trap.

In one aspect, one polypeptide of the heterodimer comprises an MHC class II α1 domain, an MHC class II α2 domain, or a combination thereof; and at least one engineered protuberance. In some embodiments, the at least one engineered protuberance is not located at the MHC class II α1 domain or the MHC class II α2 domain. In some embodiments, the engineered protuberance is located at an antibody C_{H}3 domain fused to the polypeptide. In some embodiments, the polypeptide optionally comprises an antibody C_{H}2 domain located between an MHC class II α2 domain and the C_{H}3 domain with an engineered protuberance. In certain embodiments, the polypeptide comprises an antibody C_{H}3 domain, and the antibody C_{H}3 domain comprises at least one mutation selected from the list consisting of S354C, T366W, and both S354C and T366W (EU numbering). In further embodiments, the polypeptide comprises an autoimmune or inflammatory-disease relevant peptide. Optionally, the autoimmune or inflammatory-disease relevant peptide comprises a cysteine residue that interacts with a cysteine residue in either the an MHC α1 or β1 domain to create a cysteine trap.

In one aspect, one polypeptide of the heterodimer comprises an MHC class II β1 domain, an MHC class II β2 domain, or a combination thereof; and at least one engineered protuberance. In some embodiments, the at least one engineered protuberance is not located at the MHC class II β1 domain or the MHC class II β2 domain. In some embodiments, the engineered protuberance is located at an antibody C_{H}3 domain fused to the polypeptide. In some embodiments, the polypeptide optionally comprises an antibody C_{H}2 domain located between an MHC class II β2 domain and the C_{H}3 domain with an engineered protuberance. In certain embodiments, the polypeptide comprises an antibody C_{H}3 domain, and the antibody C_{H}3 domain comprises at least one mutation selected from the list consisting of S354C, T366W, and both S354C and T366W (EU numbering). In further embodiments, the polypeptide comprises an autoimmune or inflammatory-disease relevant peptide. Optionally, the autoimmune or inflammatory-disease relevant peptide comprises a cysteine residue that interacts with a cysteine residue in either the an MHC α1 or β1 domain to create a cysteine trap.

In one aspect, one polypeptide of the heterodimer comprises an MHC class II α1 domain, an MHC class II α2 domain, or a combination thereof; and at least one engineered cavity. In some embodiments, the at least one engineered cavity is not located at the MHC class II α1 domain or the MHC class II α2 domain. In some embodiments, the engineered cavity is located at an antibody C_{H}3 domain fused to the polypeptide. In some embodiments, the polypeptide optionally comprises an antibody C_{H}2 domain located between an MHC class II α2 domain and the C_{H}3 domain with an engineered cavity. In certain embodiments, the polypeptide comprises an antibody C_{H}3 domain, and the antibody C_{H}3 domain comprises at least one mutation selected from the list consisting of Y349C, T366S, L368A, Y407V (EU numbering), and combinations thereof. In further embodiments, the polypeptide comprises an autoimmune or inflammatory-disease relevant peptide. Optionally, the autoimmune or inflammatory-disease relevant peptide comprises a cysteine residue that interacts with a cysteine residue in either the an MHC α1 or β1 domain to create a cysteine trap.

In one aspect, one polypeptide of the heterodimer comprises an MHC class II β1 domain, an MHC class II β2 domain, or a combination thereof; and at least one engineered cavity. In some embodiments, the at least one engineered cavity is not located at the MHC class II β1 domain or the MHC class II β2 domain. In some embodiments, the engineered cavity is located at an antibody C_{H}3 domain fused to the polypeptide. In some embodiments, the polypeptide optionally comprises an antibody C_{H}2 domain located between an MHC class II β2 domain and the C_{H}3 domain with an engineered cavity. In certain embodiments, the polypeptide comprises an antibody C_{H}3 domain, and the antibody C_{H}3 domain comprises at least one mutation selected from the list consisting of Y349C, T366S, L368A, Y407V (EU numbering), and combinations thereof. In further embodiments, the polypeptide comprises an autoimmune or inflammatory-disease relevant peptide. Optionally, the autoimmune or inflammatory-disease relevant peptide comprises a cysteine residue that interacts with a cysteine residue in either the an MHC α1 or β1 domain to create a cysteine trap.

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The use of the terms "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the embodiments and does not pose a limitation on the scope of the claims unless otherwise stated. No language in the specification should be construed as indicating any non-claimed element as essential.

As used herein, "heterodimer" refers to a molecule comprising a first polypeptide and a second polypeptide, wherein the second polypeptide differs in amino acid sequence from the first polypeptide by at least one amino acid residue. A "multimer" refers to a molecule comprising two or more heterodimers or comprising four or more polypeptides disclosed herein.

As used herein "individual" is synonymous with "subject". The individual can be diagnosed with a disease. The individual can suspected of having a particular disease based on manifesting at least one symptom of said disease, having a family history of said disease, having a genotype relevant to define risk for said disease, or having one or phenotypic measurements "lab tests" at or near a level that would place an individual at risk for the disease. The individual can be a mammal, such as a horse, cat, dog, pig, cow, goat, or sheep. The individual can in certain instances be a human person.

As used herein, "polypeptide" refers generally to peptides and proteins having more than about ten amino acids.

As used herein, the "first polypeptide" is any polypeptide which is to be associated with a second polypeptide. The first and second polypeptide meet at an "interface" (defined below). In addition to the interface, the first polypeptide may comprise one or more additional domains, such as "binding domains" (*e.g.*, a ligand binding domain) or antibody constant domains (or parts thereof) including C_{H}2, C_{H}1 and C_{L} domains. In some embodiments, the first polypeptide comprises at least one domain which is derived from an antibody. In further embodiments, the domain conveniently is a constant domain, such as the C_{H}3 domain of an antibody. In still further embodiments, the domain forms the interface of the first polypeptide.

As used herein, the "second polypeptide" is any polypeptide which is to be associated with the first polypeptide via an "interface". In addition to the interface, the second polypeptide may comprise additional domains such as a "binding domain" (*e.g.,* a ligand binding domain), or antibody constant domains (or parts thereof) including C_{H}2, C_{H}1 and C_{L} domains. In some embodiments, the second polypeptide comprises at least one domain which is derived from an antibody. In further embodiments, the domain conveniently is a constant region, such as the C_{H}3 domain of an antibody. In still further embodiments, the domain forms the interface of the second polypeptide.

As used herein, a "binding domain" comprises any region of a polypeptide which is responsible for selectively binding to a molecule of interest (e.g., an antigen).

As used herein "binding groove" refers to the molecular pocket or cleft that exists in an MHC class II heterodimer formed between an MHC class II α₁ domain and an MHC class II β₂ domain. This is the part of the MHC class II heterodimer that interacts with antigens. The antigens are generally polypeptide antigens, but can be further modified by lipid moieties, glycol moieties, or glycolipid moieties. The residues that make up the binding groove display polymorphism and account for the differences in antigen binding between different HLA alleles. An important feature of the binding groove for MHC class II molecules is that the ends of the groove are open and allow for the antigen to extend beyond either or both sides of the binding groove.

As used herein, the "interface" comprises those "contact" amino acid residues in the first polypeptide which interact with one or more "contact" amino acid residues in the interface of the second polypeptide. In some embodiments, the interface comprises the C_{H}3 domain of an immunoglobulin. In further embodiments, the immunoglobulin is derived from an IgG antibody. The IgG antibody may be of IgG₁, IgG₂, IgG₃, or IgG₄ isotype. In still further embodiments, the immunoglobulin is derived from a human IgG1 antibody.

As used herein, a "protuberance" refers to at least one amino acid side chain which projects from the interface of the first polypeptide and is therefore positionable in a compensatory cavity in the adjacent interface (i.e., the interface of the second polypeptide) so as to stabilize the heterodimer, and thereby favor heterodimer formation over homodimer formation, for example. The protuberance may exist in the original interface or may be introduced synthetically (e.g., by altering nucleic acid encoding the interface). An "engineered protuberance" is introduced synthetically. In some embodiments, a nucleic acid encoding the interface of the first polypeptide is altered to encode the protuberance. To achieve this, the nucleic acid encoding at least one "original" amino acid residue in the interface of the first polypeptide is replaced with a nucleic acid encoding at least one "import" amino acid residue which has a larger side chain volume than the original amino acid residue. It will be appreciated that there can be more than one original and corresponding import residue. In some embodiments, the upper limit for the number of original residues which are replaced is the total number of residues in the interface of the first polypeptide. In some embodiments, the import residues for the formation of a protuberance are generally naturally occurring amino acid residues. In some embodiments, the import residues are selected from arginine (R), cysteine (C), phenylalanine (F), tyrosine (Y), and tryptophan (W). In some embodiments, the import residues are selected from arginine (R), phenylalanine (F), tyrosine (Y), and tryptophan (W). In some embodiments, the import residues are tryptophan and tyrosine. In some embodiments, the import residues are tryptophan and cysteine. In some embodiments, the original residue for the formation of the protuberance has a small side chain volume, such as alanine, asparagine, aspartic acid, glycine, serine, threonine, or valine. In some embodiments, a serine residue is replaced with cysteine and a threonine residue is replaced with tryptophan. In some embodiments, the serine at position 354 is replaced with another amino acid residue to form part or all of the protuberance. In some embodiments, the threonine at position 366 is replaced with another amino acid residue to form part or all of the protuberance. In further embodiments, the amino acid replacements comprise S354C and T366W (EU numbering). All numbering according to Edelman, Gerald M. et al. PNAS 63(1) (1969): 78-85.

As used herein, a "cavity" refers to at least one amino acid side chain which is recessed from the interface of the second polypeptide and therefore accommodates a corresponding protuberance on the adjacent interface of the first polypeptide. The cavity may exist in the original interface or may be introduced synthetically (e.g., by altering nucleic acid encoding the interface). An "engineered cavity" is introduced synthetically. In some embodiments, nucleic acid encoding the interface of the second polypeptide is altered to encode the cavity. To achieve this, the nucleic acid encoding at least one "original" amino acid residue in the interface of the second polypeptide is replaced with DNA encoding at least one "import" amino acid residue which has a smaller side chain volume than the original amino acid residue. It will be appreciated that there can be more than one original and corresponding import residue. The upper limit for the number of original residues which are replaced is the total number of residues in the interface of the second polypeptide. In some embodiments, the import residues for the formation of a cavity are usually naturally occurring amino acid residues. In some embodiments, the import residues are selected from alanine (A), cysteine (C), serine (S), threonine (T), and valine (V). In some embodiments, the import residues are selected from alanine (A), serine (S), threonine (T), and valine (V). In some embodiments, the import residues are selected from alanine (A), cysteine (C), serine (S), and valine (V). In some embodiments, the import residues are serine, alanine, or threonine. In some embodiments, the import residues are alanine (A), cysteine (C), serine (S), and valine (V). In some embodiments, the tyrosine at position 349 is replaced with another amino acid residue to form part or all of the cavity. In some embodiments, the threonine at position 366 is replaced with another amino acid residue to form part or all of the cavity. In some embodiments, the leucine at position 368 is replaced with another amino acid residue to form part or all of the cavity. In some embodiments, the tyrosine at position 407 is replaced with another amino acid residue to form part or all of the cavity. In some embodiments, the original residue for the formation of the protuberance has a large side chain volume, such as tyrosine, arginine, phenylalanine, or tryptophan. In other embodiments, the original residue is selected from tyrosine, threonine, and leucine. In some embodiments, one or more tyrosine residues is replaced with cysteine or valine, a threonine residue is replaced with serine, and a leucine residue is replaced with alanine. In further embodiments, the amino acid replacements comprise Y349C, T366S, L368A, and Y407V (EU numbering). All numbering according to Edelman, Gerald M. et al. PNAS 63(1) (1969): 78-85.

As used herein, an "original" amino acid residue is one which is replaced by an "import" residue which can have a smaller or larger side chain volume than the original residue. The import amino acid residue can be a naturally occurring or non-naturally occurring amino acid residue, but preferably is the former. "Naturally occurring" amino acid residues are those residues encoded by the genetic code. By "non-naturally occurring" amino acid residue is meant a residue which is not encoded by the genetic code, but which is able to covalently bind adjacent amino acid residue(s) in the polypeptide chain. Examples of non-naturally occurring amino acid residues are nor leucine, ornithine, nor valine, homoserine, and other amino acid residue analogues such as those described in Ellman et al., Meth. Enzym. 202:301-336 (1991), for example. To generate such non-naturally occurring amino acid residues, the procedures of Noren et al. Science 244: 182 (1989) and Ellman et al., supra can be used. Briefly, this involves chemically activating a suppressor tRNA with a non-naturally occurring amino acid residue followed by in vitro transcription and translation of the RNA. The method of the instant invention involves replacing at least one original amino acid residue, but more than one original residue can be replaced. Normally, no more than the total residues in the interface of the first or second polypeptide will comprise original amino acid residues which are replaced. The preferred original residues for replacement are "buried". By "buried" is meant that the residue is essentially inaccessible to solvent. In certain embodiments, the import residue is not cysteine to prevent possible oxidation or mispairing of disulfide bonds.

The protuberance is "positionable" in the cavity which means that the spatial location of the protuberance and cavity on the interface of the first polypeptide and second polypeptide respectively and the sizes of the protuberance and cavity are such that the protuberance can be located in the cavity without significantly perturbing the normal association of the first and second polypeptides at the interface. Since protuberances comprising one of more amino acids such as, but not limited to, Cys, Tyr, Phe, and Trp do not typically extend perpendicularly from the axis of the interface and have preferred conformations, the alignment of a protuberance with a corresponding cavity relies on modeling the protuberance/cavity pair based upon a three-dimensional structure such as that obtained by X-ray crystallography or nuclear magnetic resonance (NMR). This can be achieved using widely accepted techniques in the art.

An "original nucleic acid" is a nucleic acid encoding a polypeptide of interest which can be "altered" (i.e. genetically engineered or mutated) to encode a protuberance or cavity. The original or starting nucleic acid may be a naturally occurring nucleic acid or may comprise a nucleic acid which has been subjected to prior alteration (e.g. a humanized antibody fragment). By "altering" the nucleic acid is meant that the original nucleic acid is mutated by inserting, deleting, or replacing at least one codon encoding an amino acid residue of interest. Normally, a codon encoding an original residue is replaced by a codon encoding an import residue. Techniques for genetically modifying a DNA in this manner have been reviewed in Mutagenesis: a Practical Approach, M. J. McPherson, Ed., (IRL Press, Oxford, UK. (1991), and include site-directed mutagenesis, cassette mutagenesis and polymerase chain reaction (PCR) mutagenesis, for example.

The protuberance or cavity can be "introduced" into the interface of the first or second polypeptide by synthetic means, e.g. by recombinant techniques, in vitro peptide synthesis, those techniques for introducing non-naturally occurring amino acid residues previously described, by enzymatic or chemical coupling of peptides or some combination of these techniques. Accordingly, the protuberance or cavity which is "introduced" is "non-naturally occurring" or "non-native", which means that it does not exist in nature or in the original polypeptide (e.g. a humanized monoclonal antibody).

Preferably the import amino acid residue for forming the protuberance has a relatively small number of "rotamers" (e.g., about 3-6). A "rotamer" is an energetically favorable conformation of an amino acid side chain. The number of rotamers of the various amino acid residues are reviewed in Ponders and Richards, J. Mol. Biol. 193: 775-791 (1987).

As used herein, "knob-in-hole" or "knob-into-hole" refers to a polypeptidyl architecture requiring a protuberance (or "knob") at an interface of a first polypeptide and a corresponding cavity (or a "hole") at an interface of a second polypeptide, such that the protuberance can be positioned in the cavity so as to promote heterodimer formation. Protuberances may be constructed by replacing small amino acid side chains from the interface of the first polypeptide with larger side chains (e.g., phenylalanine or tyrosine). Cavities of identical or similar size to the protuberances may be created in the interface of the second polypeptide by replacing large amino acid side chains with smaller ones (e.g., alanine or threonine). The protuberances and cavities can be made by synthetic means such as by altering the nucleic acid encoding the polypeptides or by peptide synthesis, using routine methods by one skilled in the art. In some embodiments, the interface of the first polypeptide is located on an Fc domain in the first polypeptide; and the interface of the second polypeptide is located on an Fc domain on the second polypeptide. Knob-in-hole heterodimers and methods of their preparation and use are disclosed in U.S. Patent Nos. 5,731,168; 5,807,706; 5,821,333; 7,642,228; 7,695,936; 8,216,805; and 8,679,785; and in Merchant et al., Nature Biotechnology, 1998, 16:677-681.

As used herein, "isolated" heterodimer means heterodimer which has been identified and separated and/or recovered from a component of its natural cell culture environment. Contaminant components of its natural environment are materials which would interfere with diagnostic or therapeutic uses for the heterodimer, and may include enzymes, hormones, and other proteinaceous or nonproteinaceous solutes. In preferred embodiments, the heterodimer will be purified (1) to greater than 95% by weight of protein as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, silver stain.

By "nanosphere," "NP," or "nanoparticle" herein is meant a small discrete particle that is administered singularly or plurally to a subject, cell specimen, or tissue specimen as appropriate. In certain embodiments, the term "nanoparticle" as used herein includes any layers around the nanoparticle core. In certain embodiments, the nanoparticles are substantially spherical in shape. In certain embodiments, the nanoparticle is not a liposome or a viral particle. In further embodiments, the nanoparticle is comprised of any appropriate material, *e.g*., a solid, a solid core, a metal, a dendrimer, a polymeric micelle, a metal oxide, or a protein or fragment or combinations thereof. The term "substantially spherical," as used herein, means that the shape of the particles does not deviate from a sphere by more than about 10%. Various known antigen or peptide complexes of the disclosure may be applied to the particles. The nanoparticles of this disclosure range in size from about 1 nm to about 1 µm, from about 1 nm to about 500 nm or alternatively from about 1 nm to about 100 nm, or alternatively from about 1 nm to about 50 nm, or alternatively from about 5 nm to about 100 nm, and in some aspects refers to the average or median diameter of a plurality of nanoparticles when a plurality of nanoparticles are intended. Smaller nanosize particles can be obtained, for example, by the process of fractionation whereby the larger particles are allowed to settle in an aqueous solution. The upper portion of the solution is then recovered by methods known to those of skill in the art. This upper portion is enriched in smaller size particles. The process can be repeated until a desired average size is generated. The term "nanostructure" is used generally to describe structures smaller than about 1 µm.

An antigen-MHC-nanoparticle complex ("NP-complex" or "complex" or pMHC-NP or "nanoparticle complex") refers to presentation of a peptide, carbohydrate, lipid, or other antigenic segment, fragment, or epitope of an antigenic molecule or protein (*i.e.*, self-peptide or autoantigen) on a surface, such as a nanoparticle core.

The "nanoparticle core" is the nanoparticle substrate that does or does not include layers or coatings. The nanoparticle complex comprises the core with at least the antigen-MHC complex coupled to the core.

"Density," when referring to pMHC per nanoparticle, is calculated as the surface area of the nanoparticle core with or without outer layers that can also include linkers. Surface area is the total available surface area of the construct used. In one aspect, when a PEG linker is used, this can increase the total diameter of the nanoparticle core by about 20 nm² of the nanoparticle which increases the surface area accordingly of the total available surface area of the nanoparticle. In other words, it is the final surface area of the nanoparticle without the addition of one or more of the pMHC, costimulatory molecules, and/or cytokines.

"Antigen" as used herein refers to all, part, fragment, or segment of a molecule that can induce an immune response in a subject or an expansion of an immune cell, preferably a T or B cell.

As used herein, the term "disease-relevant" antigen refers to an antigen or fragment thereof selected to treat a selected disease and is involved in the disease process. For example, a diabetes-relevant antigen is an antigen or fragment thereof that, when presented, produces an immune response that serves to treat diabetes; thus, a diabetes-relevant antigen producing such an effect is selected to treat diabetes. A multiple sclerosis (MS)-relevant antigen is selected to treat MS. A diabetes-relevant antigen would not be selected to treat MS. Similarly, an autoimmunity-related antigen is an antigen that is relevant to an autoimmune disease and would not be selected for the treatment of a disorder or disease other than autoimmunity, e.g., cancer. Non-limiting, exemplary disease-relevant antigens are disclosed herein and further, such antigens may be determined for a particular disease based on techniques, mechanisms, and methods documented in the literature.

### MHC class II heterodimers and knob-in-hole architecture

Expression of MHC class II molecules has been difficult, because secreted MHC class II α and β chains lacking the transmembrane and cytoplasmic domains do not form stable heterodimers, even in the presence of a peptide ligand. The transmembrane regions of the MHC class II α and β chains facilitate the proper assembly of the αβ heterodimer, presumably through the interaction of the two α-helical transmembrane segments.

Currently, the state of the art is to produce recombinant pMHC class II molecules to generate pMHC tetramers using a leucine zipper. This is also true for the NIH tetramer facility and commercial suppliers of pMHC tetramers.

This approach, however, offers its own limitations as a platform with which to build pMHC class II monomers for production of disease-relevant pMHC class II-nanoparticle compounds: 1) the possibility that the jun/fos sequences might be recognized as "foreign" and thus be immunogenic in pMHC-nanoparticle-treated individuals, which could potentially limit therapeutic activity, particularly after repeated administration; 2) chromatographic separation of pMHC complexes from eukaryotic cell culture (*i.e.,* Chinese hamster ovary -CHO-) supernatants is difficult if affinity separation tags are not introduced in the pMHC complex; and 3) affinity tags are also problematic due to the antigenicity of affinity tags such as FLAG or 6xHIS. Due to the possibility of antigenicity and their potential widespread use in purification of different proteins these tags are disfavored for use with human therapeutics. Unfortunately, addition of affinity tags to these proteins could trigger the generation of anti-drug antibodies (ADAs).

Provided herein, in one aspect, are isolated heterodimers comprising at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof.

In another aspect, provided herein are isolated heterodimers comprising a first polypeptide and a second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof.

In another aspect, provided herein are isolated heterodimers comprising a first polypeptide and a second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain and an MHC class II α2 domain; and the second polypeptide comprises an MHC class II β1 domain and an MHC class II β2 domain; or (ii) the first polypeptide comprises an MHC class II β1 domain and an MHC class II β2 domain; and the second polypeptide comprises an MHC class II α1 domain and an MHC class II α2 domain.

In some embodiments, the first polypeptide and the second polypeptide each comprise a C_{H}3 domain of an antibody and interface via the C_{H}3 domains. In some embodiments, the first polypeptide and the second polypeptide each comprise a C_{H}2 domain of an antibody and interface via the C_{H}2 domains. In some embodiments, the first polypeptide and the second polypeptide each comprise a C_{H}2 domain and a C_{H}3 domain of an antibody and interface via the C_{H}2 domains, the C_{H}3 domains, or a combination thereof. In some embodiments, the first polypeptide and the second polypeptide each comprise a C_{H}2 domain and a C_{H}3 domain of an antibody and interface via the C_{H}3 domains. In some embodiments, the C_{H}3 domain is from an IgG. In some embodiments, the C_{H}2 domain is from an IgG. The IgG may be of IgG1, IgG2, IgG3, or IgG4 subtype. In some embodiments, the IgG is of the IgG1 subtype.

In some embodiments, the first polypeptide and/or the second polypeptide further comprise a C-terminal cysteine residue. In some embodiments, the first polypeptide and the second polypeptide further comprise a C-terminal cysteine residue. In some embodiments, the first polypeptide or the second polypeptide further comprise a C-terminal cysteine residue. In some embodiments, the first polypeptide further comprises a C-terminal cysteine residue. In some embodiments, the second polypeptide further comprises a C-terminal cysteine residue.

Biotinylation of pMHCs via BirA is a technique routinely used to produce biotinylated pMHC monomers suitable for tetramerization using streptavidin. Such pMHC tetramers and their higher order derivatives (pentamers, dextramers, or other multimers) are useful as reagents capable of enumerating the frequency of antigen-specific T-cells in biological samples. In some embodiments of the disclosure and not of the invention, the first polypeptide and/or the second polypeptide further comprise a biotinylation site. In some embodiments of the disclosure and not of the invention, the first polypeptide and the second polypeptide further comprise a biotinylation site. In some embodiments of the disclosure and not of the invention, the first polypeptide or the second polypeptide further comprise a biotinylation site. In some embodiments of the disclosure and not of the invention, the first polypeptide further comprises a biotinylation site. In some embodiments of the disclosure and not of the invention, the second polypeptide further comprises a biotinylation site.

In some embodiments of the disclosure and not of the invention, the first polypeptide and/or the second polypeptide further comprise a Strep tag. In some embodiments of the disclosure and not of the invention, the first polypeptide and the second polypeptide further comprise a Strep tag. In some embodiments of the disclosure and not of the invention, the first polypeptide or the second polypeptide further comprise a Strep tag. In some embodiments of the disclosure and not of the invention, the first polypeptide further comprises a Strep tag. In some embodiments of the disclosure and not of the invention, the second polypeptide further comprises a Strep tag.

In some embodiments of the disclosure and not of the invention, the isolated heterodimer further comprises a leucine zipper. In some embodiments of the disclosure and not of the invention, the first polypeptide comprises a C-Jun fragment and the second polypeptide comprises a C-Fos fragment. In embodiments, the isolated heterodimer does not comprise a leucine zipper.

In embodiments, the isolated heterodimer is devoid of extraneous protein sequences that could be the target of immunoreactivity, such as the biotinylation sequence that the BirA enzyme targets to attach a biotin molecule or an epitope/affinity/purification tag (*e.g.,* c-Myc, FLAG, V5, polyhistidine, 6xHIS, etc.). Often MHC dimers are produced using heterologous dimerization domains. Some examples include leucine zippers (Fos/Jun, Acid-p1/Base-p1, or GCN4 dimers), Colicin E9/E9 DNase immunity protein dimers, or Kv1.2 T1 domain dimers. In some embodiments, the isolated heterodimer is devoid of a dimerization domain other than a knob-in-hole architecture. In embodiments, the isolated heterodimer is devoid of a leucine zipper dimerization domain. In some embodiments, the isolated heterodimer is devoid of c-FOS/c-JUN dimerization domains.

In certain embodiments, the MHC class II α1 domain and an MHC class II α2 domain and the MHC class II β1 domain and the MHC class II β2 domain of the heterodimer are derived from a human leukocyte antigen (HLA) molecule such as HLA-DR, HLA-DQ, or HLA-DP. In certain embodiments, the MHC class II α1 domain and the MHC class II α2 domain are derived from DRA, DQA1, or DPA1. In certain embodiments, the MHC class II α1 domain or the MHC class II α2 domain are derived from DRA, DQA1, or DPA1. In certain embodiments, the MHC class II β1 domain and the MHC class II β2 are derived from HLA-DRB1, HLA-DRB3, HLA-DRB4, HLA-DRB5, HLA-DQB1, or HLA-DPB1. In certain embodiments, the MHC class II β1 domain or the MHC class II β2 are derived from HLA-DRB1, HLA-DRB3, HLA-DRB4, HLA-DRB5, HLA-DQB1, or HLA-DPB1.

In some embodiments, two or more heterodimers are covalently attached to one another through a polymeric backbone to form a multimer. In some embodiments, the polymeric backbone is dextran or polyethylene glycol. In some embodiments, each of the polypeptides within the heterodimer is attached to the polymeric backbone. In some embodiments, each of the polypeptides within the heterodimer is attached to the polymeric backbone via a terminal cysteine residue on each of the polypeptides. In some embodiments, each of the polypeptides within the heterodimer is attached to the polymeric backbone via a biotinylation site on each of the polypeptides.

In some embodiments, two heterodimers are covalently attached to one another via avidin to form a multimer.

### Antigens

In certain embodiments, the heterodimers described herein further comprise an antigen. In certain embodiments, the antigen is a polypeptide antigen that is at least 10, 11, 12, 13, 14, 15, 16, 17, 18, 19, 20, 21, 22, 23, 24, 25, 26, 27, 28, 29, 30 or more amino acids in length, and, in certain embodiments, not longer than 50, 60, 70, 80, 90, or 100 amino acids in length. In certain embodiments, the antigen is a disease-relevant antigen. In certain embodiments, the antigen is an autoimmune disease-relevant antigen. In certain embodiments, the antigen is an inflammatory disease-relevant antigen. The antigens described herein are joined to the N-terminus of an MHC class IIα or MHC class IIβ polypeptide. In certain embodiments, the antigens are joined by a flexible peptide linker. The linker can, for instance, be a polypeptide linker and comprise glycine and serine.

In certain embodiments, the antigen is an autoimmune-relevant antigen. In certain embodiments, the autoimmune-relevant antigen is a type I diabetes, multiple sclerosis, Celiac disease, primary biliary cirrhosis, autoimmune hepatitis, primary sclerosing cholangitis, pemphigus, pemphigus folliaceus, pemphigus vulgaris, neuromyelitis optica spectrum disorder, arthritis (including rheumatoid arthritis), allergic asthma, inflammatory bowel disease (including Crohn's disease and ulcerative colitis), systemic lupus erythematosus, atherosclerosis, chronic obstructive pulmonary disease, emphysema, psoriasis, autoimmune hepatitis, uveitis, Sjögren's syndrome, scleroderma, anti-phospholipid syndrome, ANCA-associated vasculitis, or Stiff Man Syndrome-relevant antigen. In a further aspect, the disease-relevant antigen is a tumor- or cancer-relevant antigen. In certain embodiments, the autoimmune-relevant antigen is a type I diabetes-relevant antigen. In certain embodiments, the autoimmune-relevant antigen is a multiple sclerosis-relevant antigen. In certain embodiments, the autoimmune-relevant antigen is a rheumatoid arthritis-relevant antigen. In certain embodiments, the autoimmune-relevant antigen is a Crohn's disease-relevant antigen. In certain embodiments, the autoimmune-relevant antigen is an ulcerative colitis disease-relevant antigen. In certain embodiments, the autoimmune-relevant antigen is a celiac disease-relevant antigen. In certain embodiments, the autoimmune-relevant antigen is a hepato-biliary autoimmune disease-relevant antigen. In certain embodiments, the hepato-biliary autoimmune-relevant antigen is a primary biliary cirrhosis, autoimmune hepatitis, or primary sclerosing cholangitis disease-relevant antigen. In certain embodiments, the antigen is derived from a microbe of the gastrointestinal tract. In certain embodiments, the antigen derived from a microbe of the gastrointestinal tract is Flagellin, Fla-2, Fla-X, uncharacterized *E. coli* protein (YIDX), or Bacterioides integrase. In certain embodiments, the antigen is a food antigen. In certain embodiments, the food antigen is gliadin. In certain embodiments, the food antigen is ovalbumin. In certain embodiments, the food antigen is a peanut derived antigen.

### Cysteine trapping

A cysteine trap can be utilized to stabilize a heterodimer described herein. Cysteine trapping involves forming covalently joined polypeptide complexes from unbound polypeptide partners. In some embodiments, cysteine trapping comprises introducing a cysteine at a strategically selected position within the interaction interface of the polypeptide partners to form a stabilized polypeptide complex. In some embodiments, cysteine trapping may stabilize the polypeptide complex to favor a specific conformation and to prevent dissociation. Cysteine trapping is also referred to as disulfide trapping and disulfide crosslinking. Examples of methods and applications of cysteine trapping are reviewed in Kufareva, et al., Methods Enzymol. 570: 389-420 (2016). In the context of MHC, a cysteine is engineered into a polypeptide that is known or suspected to associate in the binding groove of an MHC class II dimer. A cysteine is then engineered in or near the binding groove such that, when the polypeptide associates with the binding groove, the binding groove cysteine can come into proximity and form a disulfide linkage with a polypeptide cysteine.

To employ a cysteine trap requires that two cysteines be engineered into the polypeptides that make up the heterodimers. The first cysteine is a "binding groove cysteine" that is within or near the MHC class II binding groove. The binding groove cysteine can be within 1, 2, 3, 4, 5, 6, 7, 8, 9, or 10 amino acids of the binding groove. The cysteine can be located on either of the alpha or beta chains and at either the N- or the C-terminus of the binding groove. The second cysteine is a "polypeptide cysteine" that is within or near an antigenic polypeptide that associates in the binding groove. The polypeptide cysteine can be within 1, 2, 3, 4, 5, 6, 7, 8, 9, or 10 amino acids of the antigenic polypeptide. The polypeptide cysteine can be at either the Nor the C-terminus of the antigenic polypeptide. Usually the antigenic polypeptide is joined to either an alpha chain or a beta chain by a flexible linker and a cysteine is placed between the linker and the antigenic polypeptide. In some embodiments, the arrangement of the polypeptide chain is antigenic polypeptide-polypeptide cysteine-linker-MHC class II α1 domain, MHC class II α2 domain. In some embodiments, the arrangement of the polypeptide chain is antigenic polypeptide-polypeptide cysteine-linker-MHC class II β1 domain, MHC class II β2 domain. In some embodiments, the arrangement of the polypeptide chain is polypeptide cysteine-antigenic polypeptide- linker-MHC class II α1 domain, MHC class II α2 domain. In some embodiments, the arrangement of the polypeptide chain is polypeptide cysteine-antigenic polypeptide- linker-MHC class II β1 domain, MHC class II β2 domain. These MHC class II polypeptides can further comprise a C_{H}2 and a C_{H}3 domain with an engineered knob on one polypeptide, and a C_{H}2 and a C_{H}3 domain with an engineered hole on the other polypeptide. In certain instances, an antigenic polypeptide may comprise a naturally occurring cysteine negating the necessity of adding a cysteine residue.

In some embodiments, the polypeptides of the heterodimers described herein comprise one or more mutations to introduce a cysteine. In some embodiments, the mutation may be in the MHC class II alpha chain. In some embodiments, the mutation may be in the MHC class II beta chain. In some embodiments, the mutation may be in the MHC class II α1 domain. In some embodiments, the mutation may be in the MHC class II β1 domain. In some embodiments, the mutation may be near the binding groove that is formed between the MHC class II α1 and β1 chains, on either or both the alpha and beta chains. In some embodiments, the mutation may be in a polypeptide antigen that is associated with the binding groove. In some embodiments, the mutation may be at the N-terminus of the antigenic polypeptide that associates with the binding groove. In some embodiments, the mutation may be at the C-terminus of the antigenic polypeptide that associates with the binding groove. In some embodiments, the cysteine trap may be formed between a cysteine residue at the N-terminus of an antigenic peptide that associates in the MHC binding groove and a residue at the N-terminus of the binding groove located on the MHC class II alpha chain. In some embodiments, the cysteine trap may be formed between a cysteine residue at the N-terminus of an antigenic peptide that associates in the MHC binding groove and a residue at the C-terminus of the binding groove located on the MHC class II alpha chain. In some embodiments, the cysteine trap may be formed between a cysteine residue at the C-terminus of an antigenic peptide that associates in the MHC binding groove and a residue at the N-terminus of the binding groove located on the MHC class II alpha chain. In some embodiments, the cysteine trap may be formed between a cysteine residue at the C-terminus of an antigenic peptide that associates in the MHC binding groove and a residue at the C-terminus of the binding groove located on the MHC class II alpha chain. In some embodiments, the cysteine trap may be formed between a cysteine residue at the N-terminus of an antigenic peptide that associates in the MHC binding groove and a residue at the N-terminus of the binding groove located on the MHC class II beta chain. In some embodiments, the cysteine trap may be formed between a cysteine residue at the N-terminus of an antigenic peptide that associates in the MHC binding groove and a residue at the C-terminus of the binding groove located on the MHC class II beta chain. In some embodiments, the cysteine trap may be formed between a cysteine residue at the C-terminus of an antigenic peptide that associates in the MHC binding groove and a residue at the N-terminus of the binding groove located on the MHC class II beta chain. In some embodiments, the cysteine trap may be formed between a cysteine residue at the C-terminus of an antigenic peptide that associates in the MHC binding groove and a residue at the C-terminus of the binding groove located on the MHC class II beta chain. In some embodiments, the mutation may be in a binding core polypeptide. In some embodiments, the mutation comprises replacing an amino acid residue with a cysteine. In some embodiments, the mutation comprises adding a cysteine residue in frame with other residues.

Non-limiting examples of polypeptides that comprise a cysteine trap include, but are not limited to, the polypeptides set forth in **SEQ ID NOS: 60** and **61.** In some embodiments, the polypeptides for cysteine trapping comprises an amino acid sequence with at least 80, 81, 82, 83, 84, 85, 86, 87, 88, 89, 90, 91, 92, 93, 94, 95, 96, 97, 98, or 99% identity to the polypeptides set forth in of any one of **SEQ ID NOS: 60** and **61.** In some embodiments, the mutation to cysteine may be at any one or more of the residues of the polypeptides set forth in **SEQ ID NOS: 62** and **63.** In some embodiments, the polypeptides for cysteine trapping comprises an amino acid sequence with at least 80, 81, 82, 83, 84, 85, 86, 87, 88, 89, 90, 91, 92, 93, 94, 95, 96, 97, 98, or 99% identity to the polypeptides set forth in of any one of **SEQ ID NOS: 62** and **63.** In some embodiments, the mutation to cysteine may be at any one or more of the residues of the polypeptides set forth in **SEQ ID NOS: 62** and **63.**

### Heterodimer-Nanoparticle Conjugates

In another aspect, provided herein are heterodimer-nanoparticle conjugates comprising at least one heterodimer described herein and a nanoparticle, wherein the nanoparticle is non-liposomal and/or has a solid core. In certain embodiments, the solid core can be a metal or a metal oxide. In certain embodiments, the solid core can be iron, iron oxide, or gold. The solid core can be a high density core such that the density is greater than about 2.0 g/cm³, about 3.0 g/cm³, about 4.0 g/cm³, about 5.0 g/cm³, about 6.0 g/cm³, or about 7.0 g/cm³. In certain embodiments, the density of the solid core is between about 4.0 g/cm³ and about 8.0 g/cm³. In certain embodiments, the density of the solid core is between about 5.0 g/cm³ and about 8.0 g/cm³. In certain embodiments, the density of the solid core is between about 5.0 g/cm³ and about 7.0 g/cm³. In certain embodiments, the density of the solid core is between about 5.0 g/cm³ and about 6.0 g/cm³.

In another aspect, provided herein are heterodimer-nanoparticle conjugates comprising at least one heterodimer described herein and a nanoparticle, wherein the nanoparticle is non-liposomal and has an iron oxide core.

In another aspect, provided herein are heterodimer-nanoparticle conjugates comprising at least one heterodimer described herein and a nanoparticle, wherein the nanoparticle is non-liposomal and has a gold core.

In another aspect, provided herein are heterodimer-nanoparticle conjugates comprising at least one heterodimer described herein and a nanoparticle, wherein the nanoparticle is non-liposomal and has an iron oxide core; and the at least one heterodimer is covalently linked to the nanoparticle through a linker.

In another aspect, provided herein are heterodimer-nanoparticle conjugates comprising at least one heterodimer described herein and a nanoparticle, wherein the nanoparticle is non-liposomal and has an iron oxide core; and the at least one heterodimer is covalently linked to the nanoparticle through a linker comprising polyethylene glycol. In some embodiments, the heterodimer-nanoparticle conjugate comprises at least one heterodimer described herein and a nanoparticle, wherein the nanoparticle is non-liposomal and has an iron oxide core; and the at least one heterodimer is covalently linked to the nanoparticle through a linker comprising polyethylene glycol with a molecular weight of less than 500 Daltons. In some embodiments, polyethylene glycol has a molecular weight of less than 1 kD, 2 kD, 3 kD, 4 kD, 5 kD, 6 kD, 7 kD, 8 kD, 9 kD, or 10 kD. In some embodiments, polyethylene glycol is functionalized with maleimide. In some embodiments, polyethylene glycol has a molecular weight of between about 1 kD and about 5 kD, between about 2 kD and about 5 kD, between about 3 kD and about 5 kD. In some embodiments, polyethylene glycol is functionalized with maleimide. In certain embodiments, the end of the linker that is in contact with the solid core is embedded in the solid core.

In some aspects, the nanoparticle core has a diameter selected from the group of from about 1 nm to about 100 nm; from about 1 nm to about 75 nm; from about 1 nm to about 50 nm; from about 1 nm to about 25 nm; from about 1 nm to about 25 nm; from about 5 nm to about 100 nm; from about 5 nm to about 50 nm; from about 5 nm to about 25 nm, from about 15 nm to about 25 nm, or from about 5 nm to about 20 nm. In some embodiments, the nanoparticles core has a diameter of from about 25 nm to about 60 nm, from about 25 nm to about 50 nm, from about 20 nm to about 40 nm, from about 15 nm to about 50 nm, from about 15 nm to about 40 nm, from about 15 nm to about 35 nm, from about 15 nm to about 30 nm, or from about 15 nm to about 25 nm, alternatively about 15 nm, about 20 nm, about 25 nm, about 30 nm, about 35 nm, or about 40 nm.

In some aspects, the number of pMHCs per nanoparticle core (referred to herein as the "valency" of the nanoparticle complex) may range between about 1 pMHC complex to 1 nanoparticle core to about 6000 pMHC complexes to 1 nanoparticle core, alternatively between about 10:1 to about 6000:1, alternatively between about 11:1 to about 6000:1, alternatively between about 12:1 to about 6000:1, alternatively at least 2:1, alternatively at least 8:1, alternatively at least 9:1, alternatively at least 10:1, alternatively at least 11:1, or alternatively at least 12:1. In some embodiments, the number of pMHCs per nanoparticle core is from about 10:1 to about 6000:1, from about 20:1 to about 5500:1, alternatively from about 10:1 to about 5000:1, alternatively from about 10:1 to about 4000:1, alternatively from about 10:1 to about 3500:1, alternatively from about 10:1 to about 3000:1, alternatively from about 10:1 to about 2500:1, alternatively from about 10:1 to about 2000:1, alternatively from about 10:1 to about 1500:1, alternatively from about 10:1 to 1000:1, alternatively from about 10:1 to about 500:1, alternatively from about 10:1 to about 100:1, alternatively from about 20:1 to about 50:1, alternatively from about 25:1 to about 60:1; alternatively from about 30:1 to about 50:1, alternatively from about 35:1 to about 45:1, or alternatively about 40:1. In some embodiments, the number of pMHCs per nanoparticle core is from about 10:1 to about 100:1, from about 10:1 to about 110:1, from about 10:1 to about 120:1, from about 10:1 to about 130:1, from about 10:1 to about 140:1, from about 10:1 to about 150:1, or from about 10:1 to about 160:1. In some embodiments, the number of pMHCs per nanoparticle core is from about 30:1 to about 100:1, from about 30:1 to about 110:1, from about 30:1 to about 120:1, from about 30:1 to about 130:1, from about 30:1 to about 140:1, from about 30:1 to about 150:1, or from about 30:1 to about 160:1. In some embodiments, the number of pMHCs per nanoparticle core is from about 32:1 to about 100:1, from about 32:1 to about 110:1, from about 32:1 to about 120:1, from about 30:1 to about 130:1, from about 32:1 to about 140:1, from about 32:1 to about 150:1, or from about 32:1 to about 160:1.

In some aspects, the nanoparticle core has a defined valency per surface area of the core, also referred to herein as "density." In these aspects, the pMHC density per nanoparticle is from about 0.025 pMHC/100 nm² to about 100 pMHC/100 nm² of the surface area of the nanoparticle core, alternatively from about 0.406 pMHC/100 nm² to about 50 pMHC/100 nm², or alternatively from about 0.05 pMHC/100 nm² to about 25 pMHC/100 nm². In certain aspects, the pMHC density per nanoparticle is from about 0.4 pMHC/100 nm² to about 25 pMHC/100 nm², from about 0.4 pMHC/100 nm² to about 20 pMHC/100 nm², from about 0.4 pMHC/100 nm² to about 15 pMHC/100 nm², from about 0.4 pMHC/100 nm² to about 14 pMHC/100 nm², from about 0.4 pMHC/100 nm² to about 13 pMHC/100 nm², from about 0.4 pMHC/100 nm² to about 12 pMHC/100 nm², from about 0.4 pMHC/100 nm² to about 11.6 pMHC/100 nm², from about 0.4 pMHC/100 nm² to about 11.5 pMHC/100 nm², from about 0.4 pMHC/100 nm² to about 11 pMHC/100 nm², from about 0.4 pMHC/100 nm² to about 10 pMHC/100 nm², from about 0.4 pMHC/100 nm² to about 9 pMHC/100 nm², from about 0.4 pMHC/100 nm² to about 8 pMHC/100 nm², from about 0.4 pMHC/100 nm² to about 7 pMHC/100 nm², from about 0.4 pMHC/100 nm² to about 6 pMHC/100 nm², from about 0.4 pMHC/100 nm² to about 5 pMHC/100 nm², from about 0.4 pMHC/100 nm² to about 4 pMHC/100 nm², from about 0.4 pMHC/100 nm² to about 3 pMHC/100 nm², from about 0.4 pMHC/100 nm² to about 2.5 pMHC/100 nm², from about 0.4 pMHC/100 nm² to about 2 pMHC/100 nm², or from about 0.4 pMHC/100 nm² to about 1.5 pMHC/100 nm².

In another aspect, the nanoparticle may have a pMHC density of from about 0.22 pMHC/100 nm² to about 10 pMHC/100 nm², from about 0.22 pMHC/100 nm² to about 9 pMHC/100 nm², from about 0.22 pMHC/100 nm² to about 8 pMHC/100 nm², from about 0.22 pMHC/100 nm² to about 7 pMHC/100 nm², from about 0.22 pMHC/100 nm² to about 6 pMHC/100 nm², from about 0.22 pMHC/100 nm² to about 5 pMHC/100 nm², from about 0.22 pMHC/100 nm² to about 4 pMHC/100 nm², from about 0.22 pMHC/100 nm² to about 3 pMHC/100 nm², from about 0.22 pMHC/100 nm² to about 2 pMHC/100 nm², or from about 0.22 pMHC/100 nm² to about 1.5 pMHC/100 nm². In some aspects, the nanoparticle has a pMHC density of from about 0.22 pMHC/100 nm² to about 10 pMHC/100 nm², 0.24 pMHC/100 nm² to about 9 pMHC/100 nm², from about 0.26 pMHC/100 nm² to about 8 pMHC/100 nm², from about 0.28 pMHC/100 nm² to about 7 pMHC/100 nm², from about 0.24 pMHC/100 nm² to about 4 pMHC/100 nm², from about 0.5 pMHC/100 nm² to about 3 pMHC/100 nm², or from about 0.6 pMHC/100 nm² to about 1.5 pMHC/100 nm². In a further aspect, the nanoparticle has a pMHC density of from about 0.4 pMHC/100 nm² to about 1.3 pMHC/100 nm², alternatively from about 0.5 pMHC/100 nm² to about 0.9 pMHC/100 nm², or alternatively from about 0.6 pMHC/100 nm² to about 0.8 pMHC/100 nm².

In some embodiments, the nanoparticle can have a pMHC density of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, or 12.0 pMHC/100 nm². In specific embodiments, the nanoparticle can have a pMHC density of from about 0.4 pMHC/100 nm² to about 1.5 pMHC/100 nm², 0.4 pMHC/100 nm² to about 2.5 pMHC/100 nm², from about 0.4 pMHC/100 nm² to about 6 pMHC/100 nm², or from about 0.4 pMHC/100nm² to about 12 pMHC/100 nm².

In yet another aspect, the nanoparticle has a pMHC density as defined herein of from about 0.4 pMHC/100 nm² to about 1.3 pMHC/100 nm², alternatively from about 0.5 pMHC/100 nm² to about 0.9 pMHC/100 nm², or alternatively from about 0.6 pMHC/100 nm² to about 0.8 pMHC/100 nm², and further wherein the nanoparticle core has a diameter from about from about 25 nm to about 60 nm, from about 25 nm to about 50 nm, from about 20 nm to about 40 nm, from about 15 nm to about 50 nm, from about 15 nm to about 40 nm, from about 15 nm to about 35 nm, from about 15 nm to about 30 nm, from about 15 nm to about 25 nm, alternatively about 15 nm, about 20 nm, about 25 nm, about 30 nm, about 35 nm, or about 40 nm, or about 50 nm.

### Co-Stimulatory Molecule Components

In certain aspects of the disclosure, the NPs additionally comprise, or alternatively consist essentially of, or yet further consist of at least one co-stimulatory molecule. Co-stimulatory molecules are molecules that produce a secondary signal *in vivo* that serves to activate naive T cells into antigen-specific T cells capable of producing an immune response to cells possessing said specific antigen. The present disclosure is not limited to any specific co-stimulatory molecule. The various co-stimulatory molecules are well-known in the art. Some non-limiting examples of co-stimulatory molecules are 4-IBBL, OX40L, CD40, IL-15/IL-15Ra, CD28, CD80, CD86, CD30L, and ICOSL. Only one specific co-stimulatory molecule may be coupled to one nanoparticle or a variety of co-stimulatory molecules may be coupled to the same nanoparticle. In certain embodiments, the co-stimulatory molecule is a protein such as an antibody that is capable of agonizing a co-stimulatory receptor on a T cell. In this case, the antibody is capable of inducing a co-stimulatory signal that is necessary to activate naive T cells and induce an immune response in an antigen-specific manner. Additionally or alternatively, the term "co-stimulatory molecule" as used herein may also refer to an agent capable of generating a co-stimulatory signal by having an agonistic effect on a native co-stimulatory signaling molecule, *e.g*. anti-CD28 or CD28 ligand generating a CD28 co-stimulatory response.

In specific embodiments, the co-stimulatory molecules of the present disclosure may be any one or more of the following molecules B7-1/CD80, BTLA, B7-2/CD86, CD28, B7-H1/PD-L1, CTLA-4, B7-H2, Gi24/VISTA/B7-H5, B7-H3, ICOS, B7-H4, PD-1, B7-H6, PD-L2/B7-DC, B7-H7, PDCD6, LILRA3/CD85e, LILRB2/CD85d/ILT4, LILRA4/CD85g/ILT7, LILRB3/CD85a/ILT5, LILRB1/CD85j/ILT2, LII,RB4/CD85k/ILT3, 4-1BB/TNFRSF9/CD137, GITR Ligand/TNFSF18, 4-1BB Ligand/TNFSF9, HVEM/TNFRSF14, BAFF/BLyS/TNFSF13B, LIGHT/TNFSF14, BAFF R/TNFRSF13C, Lymphotoxin-alpha/TNF-beta, CD27/TNFRSF7, OX40/TNFRSF4, CD27 Ligand/TNFSF7, OX40 Ligand/TNFSF4, CD30/TNFRSF8, RELT/TNFRSF19L, CD30 Ligand/TNFSF8, TACI/TNFRSF13B, CD40/TNFRSF5, TL1A/TNFSF15, CD40 Ligand/TNFSF5, TNF-alpha, DR3/TNFRSF25, TNF RII/TNFRSF1B, GITR/TNFRSF18, 2B4/CD244/SLAMF4, CD84/SLAMF5, BLAME/SLAMF8, CD229/SLAMF3, CD2, CRACC/SLAMF7, CD2F-10/SLAMF9, NTB-A/SLAMF6, CD48/SLAMF2, SLAM/CD150, CD58/LFA-3, CD7, DPPIV/CD26, CD96, EphB6, CD160, Integrin alpha 4 beta 1, CD200, Integrin alpha 4 beta 7/LPAM-1, CD300a/LMIR1, LAG-3, CRTAM, TIM-1/KIM-1/HAVCR, DAP12, TIM-4, Dectin-1/CLEC7A, TSLP R, ICOSL, and/or biological equivalents thereof.

The co-stimulatory molecule can be coupled to the nanoparticle in the same manner as the pMHC complex. In one embodiment of the present disclosure, the co-stimulatory molecule and the antigen/MHC complex are separately attached to the nanoparticle. In another embodiment of the disclosure, the co-stimulatory molecule and the pMHC complex are first complexed together and are then subsequently complexed to the nanoparticle. Multiple co-stimulatory molecules may be coupled to the nanoparticle; these may be multiple of the same co-stimulatory molecule or multiple different co-stimulatory molecules. Typically, polypeptide complexes are added to the nanoparticles to yield nanoparticles with adsorbed or coupled polypeptide complexes having a ratio of number of co-stimulatory molecules:number of nanoparticles from about 1 to 6000 molecules per nanoparticle, or alternatively at least about or at most about 0.1, 0.5, 1, 10, 100, 500, 1000, 2000, 3000, 4000, 5000, 6000 or more to :1, and ranges in between, typically between about 0.1:1 to about 50:1. In another aspect, the ratio of the co-stimulatory molecule to the pMHC complex can be from about 0.1, 0.5, 1, 2, 5, 10, 50 or more to 1, preferably a ratio of 1:1, 1:2, 1:9, 1:10, 1:100, 2:1, 9:1, 10:1, or 100:1 of co-stimulatory molecule:pMHC complex is obtained. Similarly, density of the co-stimulatory molecules relative to nanoparticle surface area may be calculated according to the same relative formula as the pMHC complexes. In certain embodiments, the density of the co-stimulatory molecule per unit surface area of the nanoparticle is between about 0.0022 co-stimulatory molecules/100nm² to about 13.26 co-stimulatory molecules/100nm². In some embodiments, the density range of the co-stimulatory molecules may be the same or different from the density range for the pMHC complexes.

In some embodiments, wherein the nanoparticle comprises a one or more co-stimulatory molecules and does not comprise a pMHC complex, the nanoparticle has a co-stimulatory density of about 0.2 co-stimulatory molecule/100 nm² to about 6.5 co-stimulatory molecule/100 nm², from about 0.2 co-stimulatory molecule/100 nm² to about 6 co-stimulatory molecule/100 nm², from about 0.2 co-stimulatory molecule/100 nm² to about 5.8 co-stimulatory molecule/100 nm², from about 0.2 co-stimulatory molecule/100 nm² to about 5.75 co-stimulatory molecule/100 nm², from about 0.2 co-stimulatory molecule/100 nm² to about 5.5 co-stimulatory molecule/100 nm², from about 0.2 co-stimulatory molecule/100 nm² to about 5 co-stimulatory molecule/100 nm², from about 0.2 co-stimulatory molecule/100 nm² to about 4.5 co-stimulatory molecule/100 nm², from about 0.2 co-stimulatory molecule/100 nm² to about 4 co-stimulatory molecule/100 nm², f from about 0.2 co-stimulatory molecule/100 nm² to about 3.5 co-stimulatory molecule/100 nm², from about 0.2 co-stimulatory molecule/100 nm² to about 3 co-stimulatory molecule/100 nm², from about 0.2 co-stimulatory molecule/100 nm² to about 2.5 co-stimulatory molecule/100 nm², from about 0.2 co-stimulatory molecule/100 nm² to about 2 co-stimulatory molecule/100 nm², from about 0.2 co-stimulatory molecule/100 nm² to about 1.5 co-stimulatory molecule/100 nm², from about 0.2 co-stimulatory molecule/100 nm² to about 1.25 co-stimulatory molecule/100 nm², from about 0.2 co-stimulatory molecule/100 nm² to about 1 co-stimulatory molecule/100 nm², or from about 0.2 co-stimulatory molecule/100 nm² to about 0.75 co-stimulatory molecule/100 nm².

In another aspect, the nanoparticle may have a co-stimulatory molecule density of from about 0.11 co-stimulatory molecule/100 nm² to about 5 co-stimulatory molecule/100 nm², from about 0.11 co-stimulatory molecule/100 nm² to about 4.5 co-stimulatory molecule/100 nm², from about 0.11 co-stimulatory molecule/100 nm² to about 4 co-stimulatory molecule/100 nm², from about 0.11 co-stimulatory molecule/100 nm² to about 3.5 co-stimulatory molecule/100 nm², from about 0.11 co-stimulatory molecule/100 nm² to about 3 co-stimulatory molecule/100 nm², from about 0.11 co-stimulatory molecule/100 nm² to about 2.5 co-stimulatory molecule/100 nm², from about 0.11 co-stimulatory molecule/100 nm² to about 2 co-stimulatory molecule/100 nm², from about 0.11 co-stimulatory molecule/100 nm² to about 1.5 co-stimulatory molecule/100 nm², from about 0.11 co-stimulatory molecule/100 nm² to about 1 pMHC/100 nm², or from about 0.11 co-stimulatory molecule/100 nm² to about 0.75 co-stimulatory molecule/100 nm². In some aspects, the nanoparticle core has a co-stimulatory molecule density of from about 0.11 co-stimulatory molecule/100 nm² to about 5 co-stimulatory molecule/100 nm², 0.12 co-stimulatory molecule/100 nm² to about 4.5 co-stimulatory molecule/100 nm², from about 0.13 co-stimulatory molecule/100 nm² to about 4 co-stimulatory molecule/100 nm², from about 0.14 co-stimulatory molecule/100 nm² to about 3.5 co-stimulatory molecule/100 nm², from about 0.12 co-stimulatory molecule/100 nm² to about 2 co-stimulatory molecule/100 nm², from about 0.25 co-stimulatory molecule/100 nm² to about 1.5 co-stimulatory molecule/100 nm², or from about 0.3 co-stimulatory molecule/100 nm² to about 0.75 co-stimulatory molecule/100 nm². In a further aspect, the nanoparticle core has a co-stimulatory molecule density of from about 0.2 co-stimulatory molecule/100 nm² to about 0.65 co-stimulatory molecule/100 nm², alternatively from about 0.25 co-stimulatory molecule/100 nm² to about 0.45 co-stimulatory molecule/100 nm², or alternatively from about 0.3 co-stimulatory molecule/100 nm² to about 0.4 co-stimulatory molecule/100 nm².

In some embodiments, wherein the nanoparticle comprises a pMHC complex and one or more co-stimulatory molecules, the nanoparticle has a co-stimulatory density of about 0.4 co-stimulatory molecule/100 nm² to about 13 co-stimulatory molecule/100 nm², from about 0.4 co-stimulatory molecule/100 nm² to about 12 co-stimulatory molecule/100 nm², from about 0.4 co-stimulatory molecule/100 nm² to about 11.6 co-stimulatory molecule/100 nm², from about 0.4 co-stimulatory molecule/100 nm² to about 11.5 co-stimulatory molecule/100 nm², from about 0.4 co-stimulatory molecule/100 nm² to about 11 co-stimulatory molecule/100 nm², from about 0.4 co-stimulatory molecule/100 nm² to about 10 co-stimulatory molecule/100 nm², from about 0.4 co-stimulatory molecule/100 nm² to about 9 co-stimulatory molecule/100 nm², from about 0.4 co-stimulatory molecule/100 nm² to about 8 co-stimulatory molecule/100 nm², from about 0.4 co-stimulatory molecule/100 nm² to about 7 co-stimulatory molecule/100 nm², from about 0.4 co-stimulatory molecule/100 nm² to about 6 co-stimulatory molecule/100 nm², from about 0.4 co-stimulatory molecule/100 nm² to about 5 co-stimulatory molecule/100 nm², from about 0.4 co-stimulatory molecule/100 nm² to about 4 co-stimulatory molecule/100 nm², from about 0.4 co-stimulatory molecule/100 nm² to about 3 co-stimulatory molecule/100 nm², from about 0.4 co-stimulatory molecule/100 nm² to about 2.5 co-stimulatory molecule/100 nm², from about 0.4 co-stimulatory molecule/100 nm² to about 2 co-stimulatory molecule/100 nm², or from about 0.4 co-stimulatory molecule/100 nm² to about 1.5 co-stimulatory molecule/100 nm².

In another aspect, the nanoparticle may have a co-stimulatory molecule density of from about 0.22 co-stimulatory molecule/100 nm² to about 10 co-stimulatory molecule/100 nm², from about 0.22 co-stimulatory molecule/100 nm² to about 9 co-stimulatory molecule/100 nm², from about 0.22 co-stimulatory molecule/100 nm² to about 8 co-stimulatory molecule/100 nm², from about 0.22 co-stimulatory molecule/100 nm² to about 7 co-stimulatory molecule/100 nm², from about 0.22 co-stimulatory molecule/100 nm² to about 6 co-stimulatory molecule/100 nm², from about 0.22 co-stimulatory molecule/100 nm² to about 5 co-stimulatory molecule/100 nm², from about 0.22 co-stimulatory molecule/100 nm² to about 4 co-stimulatory molecule/100 nm², from about 0.22 co-stimulatory molecule/100 nm² to about 3 co-stimulatory molecule/100 nm², from about 0.22 co-stimulatory molecule/100 nm² to about 2 co-stimulatory molecule/100 nm², or from about 0.22 co-stimulatory molecule/100 nm² to about 1.5 co-stimulatory molecule/100 nm². In some aspects, the nanoparticle core has a co-stimulatory molecule density of from about 0.22 co-stimulatory molecule/100 nm² to about 10 co-stimulatory molecule/100 nm², 0.24 co-stimulatory molecule/100 nm² to about 9 co-stimulatory molecule/100 nm², from about 0.26 co-stimulatory molecule/100 nm² to about 8 co-stimulatory molecule/100 nm², from about 0.28 co-stimulatory molecule/100 nm² to about 7 co-stimulatory molecule/100 nm², from about 0.24 co-stimulatory molecule/100 nm² to about 4 co-stimulatory molecule/100 nm², from about 0.5 co-stimulatory molecule/100 nm² to about 3 co-stimulatory molecule/100 nm², or from about 0.6 co-stimulatory molecule/100 nm² to about 1.5 co-stimulatory molecule/100 nm². In a further aspect, the nanoparticle has a co-stimulatory molecule density of from about 0.4 co-stimulatory molecule/100 nm² to about 1.3 co-stimulatory molecule/100 nm², alternatively from about 0.5 co-stimulatory molecule/100 nm² to about 0.9 co-stimulatory molecule/100 nm², or alternatively from about 0.6 co-stimulatory molecule/100 nm² to about 0.8 co-stimulatory molecule/100 nm².

In one aspect of the disclosure, the nanoparticle comprises a co-stimulatory molecule and does not comprise any peptide-MHC component. In certain embodiments, this co-stimulatory molecule nanoparticle complex is mixed with a nanoparticle peptide-MHC -complex to form a composition that comprises two different populations of nanoparticles, one comprising only co-stimulatory molecules, one comprising only-peptide MHC. These two populations can be mixed at any suitable ratio including 1:1, 1:2, 1:3, 1:4, 1:5, 1:6, 1:7, 1:8, or 1:9 costimulatory molecule comprising nanoparticle to peptide MHC comprising nanoparticle; or 1:1, 1:2, 1:3, 1:4, 1:5, 1:6, 1:7, 1:8, 1:9 peptide-MHC comprising nanoparticle to co-stimulatory molecule comprising nanoparticle.

### Cytokines

In certain aspects of the disclosure, the NPs further comprise, or alternatively consist essentially of, or yet further consist of at least one cytokine molecule. As used herein, the term "cytokine" encompasses low molecular weight proteins secreted by various cells in the immune system that act as signaling molecules for regulating a broad range of biological processes within the body at the molecular and cellular levels. "Cytokines" include individual immunomodulating proteins that fall within the class of lymphokines, interleukins, or chemokines.

Non limiting examples are disclosed herein: for instance, IL-1A and IL-1B are two distinct members of the human interleukin-1 (IL-1) family. Mature IL-1A is a 18 kDa protein, also known as fibroblast-activating factor (FAF), lymphocyte-activating factor (LAF), B-cell-activating factor (BAF), leukocyte endogenous mediator (LEM), etc. IL-4 is a cytokine that induces T helper-2 (Th2) cell differentiation, and is closely related to and has similar functions to IL-13. IL-5 is produced by Th2 cells and mast cells. It acts to stimulate B cell growth and increase immunoglobulin secretion. It is also involved in eosinophil activation. IL-6 is an interleukin that can act as either a pro-inflammatory or anti-inflammatory cytokine. It is secreted by T cells and macrophages to stimulate immune response to trauma or other tissue damage leading to inflammation. IL-6 is also produced from muscle in response to muscle contraction. IL-8 is a chemokine produced by macrophages and other cell types such as epithelial cells and endothelial cells, and acts as an important mediator of the immune reaction in the innate immune system response. IL-12 is involved in the differentiation of naive T cells to T helper (Th1 or Th2) cells. As a heterodimeric cytokine, IL-12 is formed after two subunits encoded by two separate genes, IL-12A (p35) and IL-12B (p40), dimerize following protein synthesis. IL-12p70 indicates this heterodimeric composition. IL-13, a cytokine secreted by many cell types, especially Th2 cells, is an important mediator of allergic inflammation and disease. IL-17 is a cytokine produced by T helper cells and is induced by IL-23, resulting in destructive tissue damage in delayed-type reactions. IL-17 functions as a pro-inflammatory cytokine that responds to the invasion of the immune system by extracellular pathogens and induces destruction of the pathogen's cellular matrix. IP-10, or Interferon gamma-induced protein 10, is also known as C-X-C motif chemokine 10 (CXCL10) or small-inducible cytokine B10. As a small cytokine belonging to the CXC chemokine family, IP-10 is secreted by several cell types (including monocytes, endothelial cells, and fibroblasts) in response to IFN-γ. Macrophage Inflammatory Proteins (MIP) belong to the family of chemokines. There are two major forms of human MIP, MIP-1α and MIP-1β, which are also known as chemokine (C-C motif) ligand 3 (CCL3) and CCL4, respectively. Both are produced by macrophages following stimulation with bacterial endotoxins. Granulocyte colony-stimulating factor (G-CSF or GCSF), also known as colony-stimulating factor 3 (CSF 3), is a colony-stimulating factor hormone. G-CSF is a glycoprotein, growth factor, and cytokine produced by a number of different tissues to stimulate the bone marrow to produce granulocytes and stem cells. G-CSF also stimulates the survival, proliferation, differentiation, and function of neutrophil precursors and mature neutrophils. Epidermal growth factor or EGF is a growth factor that plays an important role in the regulation of cell growth, proliferation, and differentiation by binding with high affinity to its receptor EGFR. Vascular endothelial growth factor (VEGF) is a family of growth factors that are important signaling proteins involved in both vasculogenesis (the de novo formation of the embryonic circulatory system) and angiogenesis (the growth of blood vessels from pre-existing vasculature).

The cytokine or cytokines can be coupled to the nanoparticle in the same manner as the pMHC complex. In one embodiment of the present disclosure, the cytokine or cytokines and the pMHC complex are separately attached to the nanoparticle. In another embodiment of the disclosure, the cytokine or cytokines molecule and the pMHC complex are first complexed together and are then subsequently complexed to the nanoparticle. Multiple cytokines may be coupled to the nanoparticle; these may be multiple of the same cytokine or different cytokines.

In some embodiments, the cytokine is complexed to an anti-cytokine antibody to form a cytokine/anti-cytokine antibody complex, which complex is subsequently complexed to the nanoparticle. In some embodiments, the cytokine/anti-cytokine antibody complex includes but is not limited to IL-2/anti-IL-2 complexes. The IL-2/anti-IL-2 complexes can have agonistic properties or antagonistic properties.

In some embodiments, the cytokine is complexed to a cytokine receptor to form a cytokine/cytokine receptor complex, which complex is subsequently complexed to the nanoparticle. In some embodiments, the cytokine/cytokine receptor complex includes but is not limited to IL15/IL-15Ra and/or IL-1/IL-2Ra. In some embodiments, the IL15/IL-15Ra complex can function as a T-cell co-stimulator.

Typically, polypeptide complexes are added to the nanoparticles to yield nanoparticles with adsorbed or coupled polypeptide complexes having a ratio of number of cytokines:number of nanoparticles from about 1 to 5999 molecules per nanoparticle, or alternatively at least about or at most about 0.1, 0.5, 1, 10, 100, 500, 1000, 2000, 3000, 4000, 5000, 6000 or more to 1, and ranges in between, for example between about 0.1:1 to about 50:1. In other aspects, the ratio of the cytokine to the antigen/MHC complex can be from about 0.1, 0.5, 1, 2, 5, 10, 50 or more to 1, preferably a ratio of 1:1, 1:2, 1:9, 1:10, 1:100, 2:1, 9:1, 10:1, or 100:1 of cytokine:antigen/MHC complex is obtained. Similarly, density of the cytokines relative to nanoparticle surface area may be calculated according to the same relative formula as the antigen/MHC complexes. In certain embodiments, the density of the cytokines per unit surface area of the nanoparticle is between about 0.0022 cytokines/100nm² to about 13.26 cytokines/100nm². In some embodiments, the density range of the cytokines may be the same or different from the density range for the antigen/MHC complexes.

In one aspect of the disclosure, the nanoparticle comprises a cytokine molecule and does not comprise any peptide-MHC component. In certain embodiments, this cytokine molecule nanoparticle complex is mixed with a nanoparticle peptide-MHC complex to form a composition that comprises two different populations of nanoparticles one comprising only cytokine molecules one comprising only-peptide MHC. These two populations can be mixed at any suitable ratio including 1:1, 1:2, 1:3, 1:4, 1:5, 1:6, 1:7, 1:8, 1:9 cytokine molecule comprising nanoparticle to peptide MHC comprising nanoparticle; or :1, 1:2, 1:3, 1:4, 1:5, 1:6, 1:7, 1:8, 1:9 peptide-MHC comprising nanoparticle to cytokine molecule comprising nanoparticle.

### NP synthesis

Nanoparticles may be formed by contacting an aqueous phase containing the co-stimulatory molecule(s), the pMHC complex, and/or cytokine, and a polymer and a nonaqueous phase followed by evaporation of the nonaqueous phase to cause the coalescence of particles from the aqueous phase as taught in U.S. Patent No. 4,589,330 or 4,818,542. Certain polymers for such preparations are natural or synthetic copolymers or polymers which include gelatin, agar, starch, arabinogalactan, albumin, collagen, polyglycolic acid, polylactic acid, glycolide-L(-) lactide poly(epsilon-caprolactone, poly(epsilon-caprolactone-CO-lactic acid), poly(epsilon-caprolactone-CO-glycolic acid), poly(β-hydroxy butyric acid), poly(ethylene oxide), polyethylene, poly(alkyl-2-cyanoacrylate), poly(hydroxyethyl methacrylate), polyamides, poly(amino acids), poly(2-hydroxyethyl DL-aspartamide), poly(ester urea), poly(L-phenylalanine/ethylene glycol/1,6-diisocyanatohexane), and poly(methyl methacrylate). Particularly, certain polymers are polyesters, such as polyglycolic acid, polylactic acid, glycolide-L(-) lactide poly(epsilon-caprolactone), poly(epsilon-caprolactone-CO-lactic acid), and poly(epsilon-caprolactone-CO-glycolic acid). Solvents useful for dissolving the polymer include: water, hexafluoroisopropanol, methylenechloride, tetrahydrofuran, hexane, benzene, or hexafluoroacetone sesquihydrate.

Gold nanoparticles (GNPs) are synthesized using chemical reduction of gold chloride with sodium citrate as described (Perrault, S.D. et al. (2009) Nano Lett 9:1909-1915). Briefly, 2 mL of 1% of HAuCl₄ (Sigma Aldrich) is added to 100 mL H₂O under vigorous stirring and the solution is heated in an oil bath. Six (for 14 nm GNPs) or two mL (for 40 nm GNPs) of 1% Na Citrate is added to the boiling HAuCl₄ solution, which is stirred for an additional 10 min and then is cooled down to room temperature. GNPs are stabilized by the addition of 1 µMol of thiol-PEG linkers (Nanocs, MA) functionalized with -COOH or -NH₂ groups as acceptors of MHC. Pegylated GNPs are washed with water to remove free thiol-PEG, concentrated, and stored in water for further analysis. NP density is determined via spectrophotometry and calculated according to Beer's law.

The SFP series iron oxide NPs (SFP IONPs) can also be produced by thermal decomposition of iron acetate in organic solvents in the presence of surfactants, then rendered solvent in aqueous buffers by pegylation (Xie, J. et al. (2007) Adv Mater 19:3163; Xie, J. et al. (2006) Pure Appl. Chem. 78:1003-1014; Xu, C. et al. (2007) Polymer International 56:821-826). Briefly, 2 mMol Fe(acac)₃ (Sigma Aldrich, Oakville, ON) are dissolved in a mixture of 10 mL benzyl ether and oleylamine and heated to 100°C for 1 hr followed by 300°C for 2 hr with reflux under the protection of a nitrogen blanket. Synthesized NPs are precipitated by addition of ethanol and resuspended in hexane. For pegylation of the IONPs, 100 mg of different 3.5 kDa DPA-PEG linkers (JenKem Tech USA) are dissolved in a mixture of CHCl₃ and HCON(CH₃)₂ (dimethylformamide (DMF)). The NP solution (20 mg Fe) is then added to the DPA-PEG solution and stirred for 4 hr at room temperature. Pegylated SFP NPs are precipitated overnight by addition of hexane and then resuspended in water. Trace amounts of aggregates are removed by high-speed centrifugation (20,000 xg, 30 min), and the monodisperse SFP NPs are stored in water for further characterization and pMHC conjugation. The concentration of iron in IONP products is determined by spectrophotometry at A410 in 2N HCL. Based on the molecular structure and diameter of SFP NPs (Fe₃O₄; 8±1 nm diameter) (Xie, J. et al. (2007) Adv Mater 19:3163; Xie, J. et al. (2006) Pure Appl. Chem. 78:1003-1014), Applicant estimates that SFP solutions containing 1 mg of iron contain 5×10¹⁴ NPs.

The nanoparticles can also be made by thermally decomposing or heating a nanoparticle precursor. In one embodiment, the nanoparticle is a metal or a metal oxide nanoparticle. In one embodiment, the nanoparticle is an iron oxide nanoparticle. In one embodiment, the nanoparticle is a gold nanoparticle. In one embodiment, provided herein are the nanoparticles prepared in accordance with the present technology. In one embodiment, provided herein is a method of making iron oxide nanoparticles comprising a thermal decomposition reaction of iron acetyl acetonate. In one embodiment, the iron oxide nanoparticle obtained is water-soluble. In one aspect, the iron oxide nanoparticle is suitable for protein conjugation. In one embodiment, the method comprises a single-step thermal decomposition reaction.

In one aspect, the thermal decomposition occurs in the presence of functionalized PEG molecules.

In one aspect, the thermal decomposition comprises heating iron acetyl acetonate. In one embodiment, the thermal decomposition comprises heating iron acetyl acetonate in the presence of functionalized PEG molecules. In one embodiment, the thermal decomposition comprises heating iron acetylacetonate in the presence of benzyl ether and functionalized PEG molecules.

Without being bound by theory, in one embodiment, functionalized PEG molecules are used as reducing reagents and as surfactants. The method of making nanoparticles provided herein simplifies and improves conventional methods, which use surfactants that are difficult to be displaced, or are not displaced to completion, by PEG molecules to render the particles water-soluble. Conventionally, surfactants can be expensive (e.g., phospholipids) or toxic (e.g., Oleic acid or oleilamine). In another aspect, without being bound by theory, the method of making nanoparticles obviates the need to use conventional surfactants, thereby achieving a high degree of molecular purity and water solubility.

In one embodiment, the thermal decomposition involves iron acetyl acetonate and benzyl ether and in the absence of conventional surfactants other than those employed herein.

In one embodiment, the temperature for the thermal decomposition is about 80°C to about 300°C, or about 80°C to about 200°C, or about 80°C to about 150°C, or about 100°C to about 250°C, or about 100°C to about 200°C, or about 150°C to about 250°C, or about 150°C to about 250°C. In one embodiment, the thermal decomposition occurs at about 1 to about 2 hours of time.

In one embodiment, the method of making the iron oxide nanoparticles comprises a purification step, such as by using Miltenyi Biotec LS magnet column.

In one embodiment, the nanoparticles are stable at about 4°C in phosphate buffered saline (PBS) without any detectable degradation or aggregation. In one embodiment, the nanoparticles are stable for at least 6 months.

In one aspect, provided herein is a method of making nanoparticle complexes comprising contacting pMHC with iron oxide nanoparticles provided herein. Without being bound by theory, pMHC encodes a Cysteine at its carboxyterminal end, which can react with the maleimide group in functionalized PEG at about pH 6.2 to about pH 6.5 for about 12 to about 14 hours.

In one aspect, the method of making nanoparticle complexes comprises a purification step, such as by using Miltenyi Biotec LS magnet column.

### Disease-relevant Antigens

The pMHC complex of the pMHC-NP is selected for use based on the disease to be treated. For example, a diabetes-relevant antigen is an antigen or fragment thereof that is expressed in the cell, tissue or organ targeted in that autoimmune disease and that is exposed to the immune system upon cell, tissue or organ damage caused by the autoimmune response, even if the antigen is not the trigger of the disease process or a key player in its pathogenesis, and when presented, produces an immune response that serves to treat diabetes; thus, a diabetes-relevant antigen meeting this definition is selected to treat diabetes. An MS-relevant antigen is selected to treat MS. A diabetes-relevant antigen would not be selected to treat MS. A cancer-relevant or tumor-relevant antigen would treat cancers and/or tumors. Non-limiting, exemplary disease-relevant antigens are disclosed herein and further, such antigens may be determined for a particular disease based on techniques, mechanisms, and methods well documented in the literature.

In certain aspects, the disease-relevant antigen comprised in the antigen-MHC complex is selected from an autoimmune disease-relevant antigen, an inflammation-relevant antigen, or an allergic disease-relevant antigen. In further aspects, the immune inflammation-relevant antigen is one or more selected from the group of an asthma-relevant antigen, a diabetes-relevant antigen, a pre-diabetes-relevant antigen, a multiple sclerosis-relevant antigen, an allergic asthma-relevant antigen, a primary biliary cirrhosis-relevant antigen, a cirrhosis-relevant antigen, a Neuromyelitis optica spectrum disorder (Devic's disease, NMO)-relevant antigen, an autoimmune encephalitis-relevant antigen, an antigen relevant to autoantibody-mediated neurological syndromes, a Stiff Man syndrome-relevant antigen, a paraneoplastic disease-relevant antigen, antigens relevant to other diseases of the central and peripheral nervous systems, a Pemphigus vulgaris-relevant antigen, inflammatory bowel disease (IBD)-relevant antigen, Crohn's disease-relevant antigen, Ulcerative Colitis-relevant antigen, an arthritis-relevant antigen, a Rheumatoid Arthritis-relevant antigen, a systemic lupus erythematosus (SLE)-relevant antigen, a Celiac Disease-relevant antigen, a psoriasis-relevant antigen, an Alopecia Areata-relevant antigen, an Acquired Thrombocytopenic Purpura-relevant antigen, an autoimmune cardiomyopathy-relevant antigen, an idiopathic dilated cardiomyopathy (IDCM)-relevant antigen, a Myasthyenia Gravis-relevant antigen, an Uveitis-relevant antigen, an Ankylosing Spondylitis-relevant antigen, a Grave's Disease-relevant antigen, a Hashimoto's thyroiditis-relevant antigen, an Immune Mediated Myopathies-relevant antigen, an anti-phospholipid syndrome (ANCA+)-relevant antigen, an atherosclerosis-relevant antigen, a scleroderma-relevant antigen, an autoimmune hepatitis-relevant antigen, a dermatomyositis-relevant antigen, a chronic obstructive pulmonary disease-relevant antigen, a spinal cord injury-relevant antigen, a traumatic injury-relevant antigen, a tobacco-induced lung destruction-relevant antigen, a Chronic Obstructive Pulmonary Disease (COPD)-relevant antigen, a lung emphysema-relevant antigen, a sclerosing cholangitis-relevant antigen, a peripheral neuropathy-relevant antigen, a narcolepsy-relevant antigen, a Goodpasture Syndrome-relevant antigen, a Kawasaki's Disease-relevant antigen, an autoimmune uveitis-relevant antigen, a colitis-relevant antigen, an emphysema-relevant antigen, a pemphigus-relevant antigen, a pemphigus folliaceus-relevant antigen, an arthritis-relevant antigen, a Sjögren's Syndrome-relevant antigen, an ANCA-associated vasculitis-relevant antigen, a primary sclerosing cholangitis-relevant antigen, an adipose tissue inflammation/diabetes type II-relevant antigen, or an obesity associated adipose tissue inflammation/insulin resistance-relevant antigen.

In certain aspects, the disease-relevant antigen is derived from one or more of the group: PPI, IGRP, GAD, peripherin, aGlia, PDC-E2, Insulin, DG1EC2, DG3, AQP4, PLP, MOG, MBP, CII, DERP1, DERP2, OVA, BacInt, CBir, Fla-X, Fla-2, YIDX, AChR, Thyroid peroxidase, Thyroid receptor, Phospholipid antigen, H4, H2B, H1, DNA, ApoB, ApoE, NMDAR, Voltage-gated potassium channel, Elastin, Arrestin, PERM_HUMAN Myeloperoxidase, PRTN3_HUMAN Myeloblastin, CP2D6_HUMAN Cytochrome P450 2D6, SPCS_HUMAN O-phosphoseryl-tRNA(Sec) selenium transferase, CAMP_HUMAN Cathelicidin antimicrobial peptide, DNA topoisomerase I, CENP-C, APOH_HUMAN Beta-2-glycoprotein 1, RO60_HUMAN 60 kDa SS-A/Ro ribonucleoprotein, LA_HUMAN Lupus La protein, IRBP, myosin, CD1d-binding lipid antigens, Cap18, CP2D6, SPCS, RO60, RO52, LA, APOH, MPO, PRTN3, or HSP.

In some embodiments, the disease-relevant antigen is:
a) a diabetes-relevant antigen and is derived from an antigen selected from one or more of the group: preproinsulin (PPI), islet-specific glucose-6-phosphatase (IGRP), glutamate decarboxylase (GAD), islet cell autoantigen-2 (ICA2), insulin, proinsulin, or a fragment or an equivalent of each thereof;
b) a multiple sclerosis-relevant antigen and is derived from an antigen selected from one or more of the group: myelin basic protein, myelin associated glycoprotein, myelin oligodendrocyte protein, proteolipid protein, oligodendrocyte myelin oligoprotein, myelin associated oligodendrocyte basic protein, oligodendrocyte specific protein, heat shock proteins, oligodendrocyte specific proteins, NOGO A, glycoprotein Po, peripheral myelin protein 22, 2'3'-cyclic nucleotide 3'-phosphodiesterase, or a fragment or an equivalent of each thereof;
c) a Celiac Disease-relevant antigen and is derived from gliadin or a fragment or an equivalent thereof;
d) a primary biliary cirrhosis-relevant antigen and is derived from PDC-E2 or a fragment or an equivalent thereof;
e) a pemphigus folliaceus-relevant antigen and/or pemphigus vulgaris-relevant antigen and is derived from an antigen selected from one or more of the group: DG1, DG3, or a fragment or an equivalent of each thereof;
f) a neuromyelitis optica spectrum disorder-relevant antigen and is derived from AQP4 or a fragment or an equivalent thereof;
g) an arthritis-relevant antigen and is derived from an antigen selected from one or more of the group: heat shock proteins, immunoglobulin binding protein, heterogeneous nuclear RNPs, annexin V, calpastatin, type II collagen, glucose-6-phosphate isomerase, elongation factor human cartilage gp39, mannose binding lectin, citrullinated vimentin, type II collagen, fibrinogen, alpha enolase, anti-carbamylated protein (anti-CarP), peptidyl arginine deiminase type 4 (PAD4), BRAF, fibrinogen gamma chain, inter-alpha-trypsin inhibitor heavy chain H1, alpha-1-antitrypsin, plasma protease C1 inhibitor, gelsolin, alpha 1-B glycoprotein, ceruloplasmin, inter-alpha-trypsin inhibitor heavy chain H4, complement factor H, alpha 2 macroglobulin, serum amyloid, C-reactive protein, serum albumin, fibrogen beta chain, serotransferin, alpha 2 HS glycoprotein, vimentin, Complement C3, or a fragment or an equivalent of each thereof;
h) an allergic asthma-relevant antigen and is derived from an antigen selected from one or more of the group: DERP1, DERP2, or a fragment or an equivalent of each thereof;
i) an inflammatory bowel disease-relevant antigen and is derived from an antigen selected from one or more of the group: Flagelin, Fla-2, Fla-X, YIDX, bacteroides integrase, or a fragment or an equivalent of each thereof;
j) a systemic lupus erythematosus-relevant antigen and is derived from an antigen selected from one or more of the group: double-stranded (ds)DNA, ribonucleoprotein (RNP), Smith (Sm), Sjögren's-syndrome-related antigen A (SS-A)/Ro, Sjögren's-syndrome-related antigen B (SS-B)/La, RO60, RO52, histones, or a fragment or an equivalent of each thereof;
k) an atherosclerosis-relevant antigen and is derived from an antigen selected from one or more of the group: ApoB, ApoE or a fragment or an equivalent of each thereof;
l) a COPD-relevant antigen and/or emphysema-relevant antigen and is derived from elastin or a fragment or an equivalent thereof;
m) a psoriasis-relevant antigen and is derived from an antigen selected from one or more of the group: Cap18, ADMTSL5, ATL5, or a fragment or an equivalent of each thereof;
n) an autoimmune hepatitis-relevant antigen and is derived from an antigen selected from one or more of the group: CYP2D6, SLA, or a fragment or an equivalent of each thereof;
o) an uveitis-relevant antigen and is derived from arrestin or a fragment or an equivalent thereof;
p) a Sjögren's Syndrome-relevant antigen and is derived from an antigen selected from one or more of the group: (SS-A)/Ro, (SS-B)/La, MR3, RO60, RO52, or a fragment or an equivalent of each thereof;
q) a scleroderma-relevant antigen and is derived from an antigen selected from one or more of the group: CENP-C, TOP 1, RNA polymerase III, or a fragment or an equivalent of each thereof;
r) an anti-phospholipid syndrome-relevant antigen and is derived from APOH or a fragment or an equivalent thereof;
s) an ANCA-associated vasculitis-relevant antigen and is derived from an antigen selected from one or more of the group: MPO, PRTN3, or a fragment or an equivalent of each thereof; or
t) a Stiff Man Syndrome-relevant antigen and is derived from GAD or a fragment or an equivalent thereof.

### Diabetes-relevant antigens

Diabetes-relevant antigens include but are not limited to those derived from PPI, IGRP, GAD, islet cell autoantigen-2 (ICA2), and/or insulin. Autoreactive, diabetes-relevant antigenic peptides include, but are not limited to, include those listed in the following **Table 1,** in addition to the peptides and proteins disclosed in U.S. Publication 2005/0202032, as well as equivalents and/or combinations of each thereof,

| **Table 1.** | | | |
|---|---|---|---|
| **Peptide** | | **Peptide** | |
| hInsB₁₀₋₁₈ | HLVEALYLV | Pro-insulin_{L2-10} | ALWMRLLPL |
| hIGRP₂₂₈₋₂₃₆ | LNIDLLWSV | Pro-insulin_{L3-11} | LWMRLLPLL |
| hIGRP₂₆₅₋₂₇₃ | VLFGLGFAI | Pro-insulin_{L6-14} | RLLPLLALL |
| IGRP₂₀₆₋₂₁₄ | VYLKTNVFL | Pro-insulin_{B5-14} | HLCGSHLVEA |
| hIGRP₂₀₆₋₂₁₄ | VYLKTNLFL | Pro-insulin_{B10-18} | HLVEALYLV |
| NRP-A7 | KYNKANAFL | Pro-insulin_{B14-22} | ALYLVCGER |
| NRP-14 | KYNIANVFL | Pro-insulin_{B15-24} | LYLVCGERGF |
| NRP-V7 | KYNKANVFL | Pro-insulin_{B17-25} | LVCGERGFF |
| YAI/D^{b} | FQDENYLYL | Pro-insulin_{B18-27} | VCGERGFFYT |
| INS B₁₅₋₂₃ | LYLVCGERG | Pro-insulin_{B20-27} | GERGFFYT |
| PPI_{76-90 (K88S)} | SLQPLALEGSLQSRG | Pro-insulin_{B21-29} | ERGFFYTPK |
| IGRP₁₃₋₂₅ | QHLQKDYRAYYTF | Pro-insulin_{B25-C1} | FYTPKTRRE |
| GAD₅₅₅₋₅₆₇ | NFFRMVISNPAAT | Pro-insulin_{B27-C5} | TPKTRREAEDL |
| GAD₅₅₅₋₅₆₇₍₅₅₇₁₎ | NFIRMVISNPAAT | Pro-insulin_{C20-28} | SLQPLALEG |
| IGRP₂₃₋₃₅ | YTFLNFMSNVGDP | Pro-insulin_{C25-33} | ALEGSLQKR |
| B₂₄-C₃₆ | FFYTPKTRREAED | Pro-insulin_{C29-A5} | SLQKRGIVEQ |
| PPI₇₆₋₉₀ | SLQPLALEGSLQKRG | Pro-insulin_{A1-10} | GIVEQCCTSI |
| INS-I9 | LYLVCGERI | Pro-insulin_{A2-10} | IVEQCCTSI |
| TUM | KYQAVTTTL | Pro-insulin_{A12-20} | SLYQLENYC |
| G6Pase | KYCLITIFL | | |

### MS-relevant antigens

Antigens of the disclosure include antigens related to multiple sclerosis. Such antigens include, for example, those disclosed in U.S. Patent Application Publication No. 2012/0077686, and antigens derived from myelin basic protein, myelin associated glycoprotein, myelin oligodendrocyte protein, proteolipid protein, oligodendrocyte myelin oligoprotein, myelin associated oligodendrocyte basic protein, oligodendrocyte specific protein, heat shock proteins, oligodendrocyte specific proteins NOGO A, glycoprotein Po, peripheral myelin protein 22, or 2'3'-cyclic nucleotide 3'-phosphodiesterase. In certain embodiments, the antigen is derived from Myelin Oligodendrocyte Glycoprotein (MOG).

In still further aspects, peptide antigens for the treatment of MS and MS-related disorders include without limitation those listed in **Table 2** as well as equivalents and/or combinations of each thereof:

| **Table 2.** | | | |
|---|---|---|---|
| **Peptide** | | **Peptide** | |
| MOG₃₅₋₅₅ | MEVGWYRSPFSRVVHLYRN GK | MOG₉₇₋₁₀₉ | TCFFRDHSYQEEA |
| MOG₃₆₋₅₅ | EVGWYRSPFSRVVHLYRNG K | MOG_{97-109(E107S)} | TCFFRDHSYQEEA |
| MAG₂₈₇₋₂₉₅ | SLLLELEEV | MOG_{97-109(E107S)} | TCFFRDHSYQSEA |
| MAG₅₀₉₋₅₁₇ | LMWAKIGPV | MBP₈₉₋₁₀₁ | VHFFKNIVTPRTP |
| MAG₅₅₆₋₅₆₄ | VLFSSDFRI | PLP₁₇₅₋₁₉₂ | YIYFNTWTTCQSIAFPSK |
| MBP₁₁₀₋₁₁₈ | SLSRFSWGA | PLP₉₄₋₁₀₈ | GAVRQIFGDYKTTIC |
| MOG₁₁₄₋₁₂₂ | KVEDPFYWV | MBP₈₆₋₉₈ | PVVHFFKNIVTPR |
| MOG₁₆₆₋₁₇₅ | RTFDPHFLRV | PLP₅₄₋₆₈ | NYQDYEYLINVIHAF |
| MOG₁₇₂₋₁₈₀ | FLRVPCWKI | PLP₂₄₉₋₂₆₃ | ATLVSLLTFMIAATY |
| MOG₁₇₉₋₁₈₈ | KITLFVIVPV | MOG₁₅₆₋₁₇₀ | LVLLAVLPVLLLQIT |
| MOG₁₈₈₋₁₉₆ | VLGPLVALI | MOG₂₀₁₋₂₁₅ | FLRVPCWKITLFVIV |
| MOG₁₈₁₋₁₈₉ | TLFVIVPVL | MOG₃₈₋₅₂ | RHPIRALVGDEVELP |
| MOG₂₀₅₋₂₁₄ | RLAGQFLEEL | MOG₂₀₃₋₂₁₇ | RVPCWKITLFVIVPV |
| PLP₈₀₋₈₈ | FLYGALLLA | PLP₂₅₀₋₂₆₄ | TLVSLLTFMIAATYN |
| MAG₂₈₇₋₂₉₅ | SLLLELEEV | MPB₁₃₋₃₂ | KYLATASTMDHARHGFLPRH |
| MAG₅₀₉₋₅₁₇ | LMWAKIGPV | MPB₈₃₋₉₉ | ENPVVHFFKNIVTPRTP |
| MAG₅₅₆₋₅₆₄ | VLFSSDFRI | MPB₁₁₁₋₁₂₉ | LSRFSWGAEGQRPGFGYGG |

### Celiac Disease (CD)-relevant antigens

Antigens relevant to celiac disease include, but are not limited to, those derived from gliadin. In some embodiments, non-limiting types of gliadin include alpha/beta gliadin, γ-gliadin, or ω-gliadin. Other non-limiting exemplary celiac disease-relevant antigens include those listed in Table **3** as well as equivalents and/or combinations of each thereof.

| **Table 3.** | |
|---|---|
| **Peptide** | |
| aGlia₅₇₋₆₈ | QLQPFPQPELPY |
| aGlia₆₂₋₇₂ | PQPELPYPQPE |
| aGlia₂₁₇₋₂₂₉ | SGEGSFQPSQQNP |

### Primary Biliary Cirrhosis (PBC)-relevant antigens

Antigens relevant to primary biliary cirrhosis include, but are not limited to, those derived from PDC-E2. Non-limiting examples of exemplary antigens include those listed in **Table 4** as well as equivalents and/or combinations of each thereof.

| **Table 4.** | | | |
|---|---|---|---|
| **Peptide** | | **Peptide** | |
| PDC-E2₁₂₂₋₁₃₅ | GDLIAEVETDKATV | PDC-E2₄₂₂₋₄₃₆ | DIPISNIRRVIAQRL |
| PDC-E2₂₄₉₋₂₆₂ | GDLLAEIETDKATI | PDC-E2₆₂₉₋₆₄₃ | AQWLAEFRKYLEKPI |
| PDC-E2₂₄₉₋₂₆₃ | GDLLAEIETDKATIG | PDC-E2₈₀₋₉₄ | SPGRRYYSLPPHQKV |
| PDC-E2₆₂₉₋₆₄₃ | AQWLAEFRKYLEKPI | PDC-E2₃₅₃₋₃₆₇ | GRVFVSPLAKKLAVE |
| PDC-E2₇₂₋₈₆ | RLLLQLLGSPGRRYY | PDC-E2₅₃₅₋₅₄₉ | ETIANDVVSLATKAR |
| PDC-E2₃₅₃₋₃₆₇ | GRVFVSPLAKKLAVE | | |

### Pemphigus Folliaceus (PF)- and Pemphigus Vulgaris (PV)-relevant antigens

Antigens relevant to PF and PV include, but are not limited to, those derived from desmoglein 3 (DG3) and/or desmoglein 1 (DG1). Non-limiting examples include those listed in **Table 5** as well as equivalents and/or combinations of each thereof.

| **Table 5.** | | | |
|---|---|---|---|
| **Peptide** | | **Peptide** | |
| DG1₂₁₆₋₂₂₉ | GEIRTMNNFLDREI | DG3₄₃₈₋₄₅₂ | DSKTAEIKFVKNMNR |
| DG3₉₇₋₁₁₁ | FGIFVVDKNTGDINI | DG1₄₈₋₆₂ | KREWIKFAAACREGE |
| DG3₂₅₁₋₂₆₅ | CECNIKVKDVNDNFP | DG1₂₀₆₋₂₂₂ | MFIINRNTGEIRTMN |
| DG3₃₅₁₋₃₆₅ | NKAEFHQSVISRYRV | DG1₃₆₃₋₃₇₇ | SQYKLKASAISVTVL |
| DG3₄₅₃₋₄₆₇ | DSTFIVNKTITAEVL | DG1₃₋₁₇ | WSFFRVVAMLFIFLV |
| DG3₅₄₀₋₅₅₄ | SITTLNATSALLRAQ | DG1₁₉₂₋₂₀₆ | SKIAFKIIRQEPSDS |
| DG3₂₈₀₋₂₉₄ | ILSSELLRFQVTDLD | DG1₃₂₆₋₃₄₀ | TNVGILKVVKPLDYE |
| DG3₃₂₆₋₃₄₀ | EGILKVVKALDYEQL | DG1₁₋₁₅ | MDWSFFRVVAMLFIF |
| DG3₃₆₇₋₃₈₁ | STPVTIQVINVREGI | DG1₃₅₋₄₉ | KNGTIKWHSIRRQKR |
| DG3₁₃₋₂₇ | AIFVVVILVHGELRI | DG1₃₂₅₋₃₃₉ | RTNVGILKVVKPLDY |
| DG3₃₂₃₋₃₃₇ | RTNEGILKVVKALDY | | |

### Neuromyelitis optica spectrum disorder (NMO)-relevant antigens

Antigens relevant to NMO include, but are not limited to, those derived from AQP4 or aquaporin 4. Non-limiting examples include those listed in **Table 6** as well as equivalents and/or combinations of each thereof.

| **Table 6.** | | | |
|---|---|---|---|
| **Peptide** | | **Peptide** | |
| AQP4₁₂₉₋₁₄₃ | GAGILYLVTPPSVVG | AQP4₁₂₉₋₁₄₃ | GAGILYLVTPPSVVG |
| AQP4₂₈₄₋₂₉₈ | RSQVETDDLILKPGV | AQP4₃₉₋₅₃ | TAEFLAMLIFVLLSL |
| AQP4₆₃₋₇₆ | EKPLPVDMVLISLC | | |

### Arthritis-relevant antigens

Antigens relevant to arthritis include, but are not limited to, those derived from heat shock proteins, immunoglobulin binding protein, heterogeneous nuclear RNPs, annexin V, calpastatin, type II collagen, glucose-6-phosphate isomerase, elongation factor human cartilage gp39, mannose binding lectin, citrullinated vimentin, type II collagen, fibrinogen, alpha enolase, anti-carbamylated protein (anti-CarP), peptidyl arginine deiminase type 4 (PAD4), BRAF, fibrinogen gamma chain, inter-alpha-trypsin inhibitor heavy chain H1, alpha-1-antitrypsin, plasma protease C1 inhibitor, gelsolin, alpha 1-B glycoprotein, ceruloplasmin, inter-alpha-trypsin inhibitor heavy chain H4, complement factor H, alpha 2 macroglobulin, serum amyloid, C-reactive protein, serum albumin, fibrogen beta chain, serotransferin, alpha 2 HS glycoprotein, vimentin, Complement C3, or a fragment or an equivalent of each thereof.

### Allergic asthma-relevant antigens

Antigens relevant to allergic asthma include, but are not limited to, those derived from DERP1 and DERP2. Non-limiting examples include those listed in **Table 7** as well as equivalents and/or combinations of each thereof.

| **Table 7.** | | | |
|---|---|---|---|
| **Peptide** | | **Peptide** | |
| DERP-1₁₆₋₃₀ | LRQMRTVTPIRMQGG | DERP-2₂₆₋₄₀ | PCIIHRGKPFQLEAV |
| DERP-1₁₇₁₋₁₈₅ | AVNIVGYSNAQGVDY | DERP-2₁₀₇₋₁₂₁ | TVKVMGDDGVLACAI |
| DERP-1₁₁₀₋₁₂₄ | RFGISNYCQIYPPNV | | |

### Inflammatory Bowel Disease-relevant antigens

Antigens relevant to inflammatory bowel disease include but are not limited to Crohn's Disease-relevant antigens and ulcerative colitis-relevant antigens. In some embodiments, inflammatory bowel disease-relevant antigens include, but are not limited to, those derived from bacteroides integrase, flagelin, flagellin 2 (Fla-2/Fla-X), or uncharacterized *E. coli* protein (YIDX). Non-limiting examples include those listed in **Table 8** as well as equivalents and/or combinations of each thereof.

| **Table 8.** | | | |
|---|---|---|---|
| **Peptide** | | **Peptide** | |
| bacteroides integrase antigen₁₈₃₋₁₉₇ | EAINQGYMHADAYPF | Fla-2/Fla-X₁₋₁₅ | MVVQHNLRAMNSNRM |
| bacteroides integrase antigen₁₄₆₋₁₆₀ | KDLTYTFLRDFEQYL | Fla-2/Fla-X₅₁₋₆₅ | KMRKQIRGLSQASLN |
| bacteroides integrase antigen₁₇₅₋₁₈₉ | RQLRTLVNEAINQGY | Fla-2/Fla-X₂₆₉₋₂₈₃ | GAYKLIQKELGLASS |
| bacteroides integrase antigen₁₋₁₅ | MDKIRYRLVYNRQNT | Fla-2/Fla-X₄₋₁₈ | QHNLRAMNSNRMLGI |
| bacteroides integrase antigen₁₈₃₋₁₉₇ | EAINQGYMHADAYPF | Fla-2/Fla-X₂₇₁₋₂₈₅ | YKLIQKELGLASSIG |
| bacteroides integrase antigen₃₀₋₄₄ | LNQRKIYLKTNVYLK | YIDX₇₈₋₉₂ | ADDIVKMLNDPALNR |
| bacteroides integrase antigen₇₀₋₈₄ | EYILYLQGIELGYWK | YIDX₉₃₋₁₀₇ | HNIQVADDARFVLNA |
| bacteroides integrase antigen₃₃₇₋₃₅₁ | TCATLLIHQGVAITT | YIDX₉₈₋₁₁₂ | ADDARFVLNAGKKKF |
| bacteroides integrase antigen₁₇₁₋₁₈₅ | AKHMRQLRTLVNEAI | YIDX₂₃₋₃₇ | GCISYALVSHTAKGS |
| bacteroides integrase antigen₄₋₁₈ | IRYRL VYNRQNTLNR | YIDX₇₈₋₉₂ | ADDIVKMLNDPALNR |
| bacteroides integrase antigen₂₅₆₋₂₇₀ | ENFIRINGKRWLYFK | YIDX₁₉₅₋₂₀₉ | LPVTVTLDIITAPLQ |
| Fla-2/Fla-X₃₆₆₋₃₈₀ | TGAAATYAIDSIADA | YIDX₂₂₋₃₆ | SGCISYALVSHTAKG |
| Fla-2/Fla-X₁₆₄₋₁₇₈ | NATFSMDQLKFGDTI | YIDX₈₀₋₉₄ | DIVKMLNDPALNRHN |
| Fla-2/Fla-X₂₆₁₋₂₇₅ | DRTVVSSIGAYKLIQ | YIDX₁₀₁₋₁₁₅ | ARFVLNAGKKKFTGT |

### Systemic Lupus Erythematosus (SLE)-relevant antigens

Antigens relevant to SLE include, but are not limited to, those derived from H4, H2B, H1', dsDNA, RNP, Smith (Sm), Sjogren's Syndrome-related Antigen A (SS-A)/Ro, Sjogren's Syndrome-related Antigen B (SS-B)/La, and/or histones. In some embodiments, SS-A includes, but is not limited to, RO60 and RO52. In some embodiments, histones include but are not limited to H4, H2B, H1'. Non-limiting examples include those listed in Table 9 as well as equivalents and/or combinations of each thereof.

| **Table 9.** | | | |
|---|---|---|---|
| **Peptide** | | **Peptide** | |
| H4₇₁₋₉₄ | TYTEHAKRKTVTAMDVVYALKRQ G | H4₈₀₋₉₄ | TVTAMDVVYALKRQ |
| H4₇₄₋₈₈ | EHAKRKTVTAMDVVY | H2B₁₀₋₂₄ | PKKGSKKAVTKAQKK |
| H4₇₆₋₉₀ | AKRKTVTAMDVVYAL | H2B₁₆₋₃₀ | KAVTKAQKKDGKKRK |
| H4₇₅₋₈₉ | HAKRKTVTAMDVVYA | H1'₂₂₋₄₂ | STDHPKYSDMIVAAIQAEKN R |
| H4₇₈₋₉₂ | RKTVTAMDVVYALKR | H1'₂₇₋₄₁ | KYSDMIVAAIQAEKN |

### Atherosclerosis-relevant antigens

Antigens relevant to atherosclerosis include, but are not limited to, those derived from Apolipoprotein B (ApoB) or Apolipoprotein E (ApoE). Non-limiting examples include those listed in **Table 10** as well as equivalents and/or combinations of each thereof.

| **Table 10.** | | | |
|---|---|---|---|
| **Peptide** | | **Peptide** | |
| ApoB₃₅₀₁₋₃₅₁₆ | SQEYSGSVANEANVY | ApoB_{210A} | KTTKQSFDLSVKAQYKKNKH |
| ApoB₁₉₅₂₋₁₉₆₆ | SHSLPYESSISTALE | ApoB_{210B} | KTTKQSFDLSVKAQY |
| ApoB₉₇₈₋₉₉₃ | TGAYSNASSTESASY | ApoB_{210C} | TTKQSFDLSVKAQYK |
| ApoB₃₄₉₈₋₃₅₁₃ | SFLSQEYSGSVANEA | | |

### Chronic Obstructive Pulmonary Disease (COPD)- and/or Emphysema-relevant antigens

Antigens relevant to COPD and/or emphysema include, but are not limited to, those derived from elastin. Non-limiting examples include those listed in **Table 11** as well as equivalents and/or combinations of each thereof.

| **Table 11.** | | | |
|---|---|---|---|
| **Peptide** | | **Peptide** | |
| elastin₈₉₋₁₀₃ | GALVPGGVADAAAAY | elastin₅₆₃₋₅₇₇ | VAAKAQLRAAAGLGA |
| elastin₆₉₈₋₇₁₂ | AAQFGLVGAAGLGGL | elastin₅₅₈₋₅₇₂ | KSAAKVAAKAQLRAA |
| elastin₈₋₂₂ | APRPGVLLLLLSILH | elastin₆₉₈₋₇₁₂ | AAQFGLVGAAGLGGL |
| elastin₉₄₋₁₀₈ | GGVADAAAAYKAAKA | elastin₅₆₆₋₅₈₀ | KAQLRAAAGLGAGIP |
| elastin₁₃₋₂₇ | VLLLLLSILHPSRPG | elastin₆₄₅₋₆₅₉ | VPGALAAAKAAKYGA |
| elastin₆₉₅₋₇₀₉ | AAKAAQFGLVGAAGL | | |

### Psoriasis-Relevant Antigens

Antigens relevant to psoriasis include, but are not limited to, those listed in the following **Table 12**, as well as equivalents and/or combinations thereof. Other non-limiting exemplary psoriasis-relevant antigens can be derived from human adamis-like protein 5 (ATL5), cathelicidin antimicrobial peptide (CAP18), and/or ADAMTS-like protein 5 (ADMTSL5).

| **Table 12.** | | | |
|---|---|---|---|
| **Peptide** | | **Peptide** | |
| Cap18₆₄₋₇₈ | RPTMDGDPDTPKPVS | ADMTSL5₃₇₂₋₃₈₆ | RLLHYCGSDFVFQAR |
| Cap18₃₄₋₄₈ | SYKEAVLRAIDGINQ | ADMTSL5₂₈₉₋₃₀₃ | HDLLLQVLLQEPNPG |
| Cap18₄₇₋₆₁ | NQRSSDANLYRLLDL | ADMTSL5₃₉₆₋₄₁₀ | ETRYEVRIQLVYKNR |
| Cap18₁₅₁₋₁₆₅ | KRIVQRIKDFLRNLV | ADMTSL5₄₃₃₋₄₄₇ | HRDYLMAVQRLVSPD |
| Cap18₁₄₉₋₁₆₃ | EFKRIVQRIKDFLRN | ADMTSL5₁₄₂₋₁₅₆ | EGHAFYHSFGRVLDG |
| Cap18₁₅₂₋₁₆₆ | RIVQRIKDFLRNLVP | ADMTSL5₂₃₆₋₂₅₀ | RNHLALMGGDGRYVL |
| Cap18₁₃₁₋₁₄₅ | RFALLGDFFRKSKEK | ADMTSL5₃₀₁₋₃₁₅ | NPGIEFEFWLPRERY |
| Cap18₂₄₋₃₈ | QRIKDFLRNLVPRTE | ADMTSL5₂₀₃₋₂₁₇ | VQRVFRDAGAFAGYW |
| ADMTSL5₂₄₅₋₂₅₉ | DGRYVLNGHWVVSPP | ADMTSL5₄₀₄₋₄₁₈ | QLVYKNRSPLRAREY |
| ADMTSL5₂₆₇₋₂₈₁ | THVVYTRDTGPQETL | | |

### Autoimmune Hepatitis-Relevant Antigens

Autoimmune hepatitis-relevant antigens include, but are not limited to, those disclosed in the following **Table 13,** as well as equivalents and/or combinations thereof. Other non-limiting exemplary autoimmune hepatitis-relevant antigens can be derived from microsomal cytochrome P450IID6 (CYP2D6) and/or soluble liver antigen (SLA).

| **Table 13.** | | | |
|---|---|---|---|
| **Peptide** | | **Peptide** | |
| CYP2D6₁₉₃₋₂₀₇ | RRFEYDDPRFLRLLD | SLA₃₃₄₋₃₄₈ | YKKLLKERKEMFSYL |
| CYP2D6₇₆₋₉₀ | TPVVVLNGLAAVREA | SLA ₁₉₆₋₂₁₀ | DELRTDLKAVEAKVQ |
| CYP2D6₂₉₃₋₃₀₇ | ENLRIVVADLFSAGM | SLA₁₁₅₋₁₂₉ | NKITNSLVLDIIKLA |
| CYP2D6₃₁₃₋₃₃₂ | TLAWGLLLMILHPDVQRRVQ | SLA ₃₇₃₋₃₈₆ | NRLDRCLKAVRKER |
| CYP2D6₃₉₃₋₄₁₂ | TTLITNLSSVLKDEAVWEKP | SLA ₁₈₆₋₁₉₇ | LIQQGARVGRID |
| CYP2D6₁₉₉₋₂₁₃ | DPRFLRLLDLAQEGL | SLA₃₁₇₋₃₃₁ | SPSLDVLITLLSLGS |
| CYP2D6₄₅₀₋₄₆₄ | RMELFLFFTSLLQHF | SLA ₁₇₁₋₁₈₅ | DQKSCFKSMITAGFE |
| CYP2D6₃₀₁₋₃₁₅ | DLFSAGMVTTSTTLA | SLA ₄₁₇₋₄₃₁ | YTFRGFMSHTNNYPC |
| CYP2D6₄₅₂₋₄₆₆ | ELFLFFTSLLQHFSF | SLA ₃₅₉₋₃₇₃ | YNERLLHTPHNPISL |
| CYP2D6₅₉₋₇₃ | DQLRRRFGDVFSLQL | SLA ₂₁₅₋₂₂₉ | DCILCIHSTTSCFAP |
| CYP2D6₁₃₀₋₁₄₄ | EQRRF SVSTLRNLGL | SLA₁₁₁₋₁₂₅ | SSLLNKITNSLVLDI |
| CYP2D6₁₉₃₋₂₁₂ | RRFEYDDPRFLRLLDLAQEG | SLA₁₁₀₋₁₂₄ | GSSLLNKITNSLVLD |
| CYP2D6₃₀₅₋₃₂₄ | AGMVTTSTTLAWGLLLMILH | SLA₂₉₉₋₃₁₃ | NDSFIQEISKMYPGR |
| CYP2D6₁₃₁₋₁₄₅ | QRRFSVSTLRNLGLG | SLA₃₄₂₋₃₅₆ | KEMFSYLSNQIKKLS |
| CYP2D6₂₁₆₋₂₃₀ | ESGFLREVLNAVPVL | SLA₄₉₋₆₃ | STLELFLHELAIMDS |
| CYP2D6₂₃₈₋₂₅₂ | GKVLRFQKAFLTQLD | SLA₁₁₉₋₁₃₃ | NSLVLDIIKLAGVHT |
| CYP2D6₁₉₉₋₂₁₃ | DPRFLRLLDLAQEGL | SLA₂₆₀₋₂₇₄ | SKCMHLIQQGARVGR |
| CYP2D6₂₃₅₋₂₅₂ | GKVLRFQKAFLTQLD | SLA₂₆₋₄₀ | RSHEHLIRLLLEKGK |
| CYP2D6₂₉₃₋₃₀₇ | ENLRIVVADLFSAGM | SLA₈₆₋₁₀₀ | RRHYRFIHGIGRSGD |
| CYP2D6₃₈₁₋₃₉₅ | DIEVQGFRIPKGTTL | SLA₃₃₁₋₃₄₅ | SNGYKKLLKERKEMF |
| CYP2D6₄₂₉₋₄₄₃ | KPEAFLPFSAGRRAC | | |

### Uveitis-Relevant Antigens

Uveitis-relevant antigens include, but are not limited to, those disclosed in the following **Table 14,** as well as equivalents and/or combinations thereof. Other non-limiting exemplary uveitis-relevant antigens can be derived from arrestin, human retinal S-antigen, and/or interphotoreceptor retinoid-binding protein (IRBP).

| **Table 14.** | | | |
|---|---|---|---|
| **Peptide** | | **Peptide** | |
| arrestin₁₉₉₋₂₁₃ | QFFMSDKPLHLAVSLN | arrestin₁₀₂₋₁₁₆ | STPTKLQESLLKKLG |
| arrestin₇₇₋₉₁ | DVIGLTFRRDLYFSR | arrestin₅₉₋₇₃ | KKVYVTLTCAFRYGQ |
| arrestin₂₅₀₋₂₆₄ | NVVLYSSDYYVKPVA | arrestin₂₈₀₋₂₉₄ | KTLTLLPLLANNRER |
| arrestin₁₇₂₋₁₈₆ | SSVRLLIRKVQHAPL | arrestin₂₉₁₋₃₀₆ | NRERRGIALDGKIKHE |
| arrestin₃₅₄₋₃₆₈ | EVPFRLMHPQPEDPA | arrestin₁₉₅₋₂₀₉ | EAAWQFFMSDKPLHL |
| arrestin₂₃₉₋₂₅₃ | KKIKAFVEQVANVVI, | arrestin₂₀₀₋₂₁₄ | QFFMSDKPLHLAVSL |

### Sjogren's Syndrome-Relevant Antigens

Sjogren's Syndrome-relevant antigens include, but are not limited to, those disclosed in the following **Table 15,** as well as equivalents and/or combinations thereof. Other non-limiting exemplary Sjogren's Syndrome-relevant antigens can be derived from (SS-A)/Ro, (SS-B)/La, RO60, RO52, and/or muscarinic receptor 3 (MR3).

| **Table 15.** | | | |
|---|---|---|---|
| **Peptide** | | **Peptide** | |
| RO60₁₂₇₋₁₄₁ | TFIQFKKDLKESMKC | LA₁₀₁₋₁₁₅ | TDEYKNDVKNRSVYI |
| RO60₅₂₃₋₅₃₇ | DTGALDVIRNFTLDM | LA₁₅₃₋₁₆₇ | SIFVVFDSIESAKKF |
| RO60₂₄₃₋₂₅₇ | EVIHLIEEHRLVREH | LA₁₇₈₋₁₉₂ | TDLLILFKDDYFAKK |
| RO60₄₈₄₋₄₉₈ | REYRKKMDIPAKLIV | LA₁₉₋₃₃ | HQIEYYFGDFNLPRD |
| RO60₃₄₇₋₃₆₁ | EEILKALDAAFYKTF | LA₃₇₋₅₁ | KEQIKLDEGWVPLEI |
| RO60₃₆₉₋₃₈₃ | KRFLLAVDVSASMNQ | LA₁₃₃₋₁₄₇ | DKGQVLNIQMRRTLH |
| RO60₄₂₆₋₄₄₀ | TDMTLQQVLMAMSQI | LA₅₀₋₆₄ | EIMIKFNRLNRLTTD |
| RO60₂₆₇₋₂₈₁ | EVWKALLQEMPLTAL | LA₃₂₋₄₆ | RDKFLKEQIKLDEGW |
| RO60₁₇₈₋₁₉₂ | SHKDLLRLSHLKPSS | LA₁₅₃₋₁₆₇ | SIFVVFDSIESAKKF |
| RO60₃₅₈₋₃₇₂ | YKTFKTVEPTGKRFL | LA₈₃₋₉₇ | SEDKTKIRRSPSKPL |
| RO60₂₂₁₋₂₃₅ | ETEKLLKYLEAVEKV | LA₁₃₆₋₁₅₀ | QVLNIQMRRTLHKAF |
| RO60₃₁₈₋₃₃₂ | RIHPFHILIALETYK | LA₂₉₇₋₃₁₁ | RNKEVTWEVLEGEVE |
| RO60₄₀₇₋₄₂₁ | EKDSYVVAFSDEMVP | LA₅₉₋₇₃ | NRLTTDFNVIVEALS |
| RO60₄₅₉₋₄₇₃ | TPADVFIVFTDNETF | LA₁₅₁₋₁₆₅ | KGSIFVVFDSIESAK |
| RO60₅₁₋₆₅ | QKLGLENAEALIRLI | LA₈₆₋₁₀₀ | KTKIRRSPSKPLPEV |
| RO60₃₁₂₋₃₂₆ | KLLKKARIHPFHILI | LA₁₅₄₋₁₆₈ | IFVVFDSIESAKKFV |
| LA₂₄₁₋₂₅₅ | DDQTCREDLHILFSN | | |

### Scleroderma-Relevant Antigens

Scleroderma-relevant antigens include, but are not limited to, those disclosed in the following **Table 16,** as well as equivalents and/or combinations thereof. Non-limiting exemplary Scleroderma-relevant antigens can be derived from centromere autoantigen centromere protein C (CENP-C), DNA topoisomerase I (TOP1), and/or RNA polymerase III.

| **Table 16.** | | | |
|---|---|---|---|
| **Peptide** | | **Peptide** | |
| TOP1₃₄₆₋₃₆₀ | KERIANFKIEPPGLF | CENP-C₈₅₇₋₈₇₁ | KVYKTLDTPFFSTGK |
| TOP1₄₂₀₋₄₃₄ | QGSIKYIMLNPSSRI | CENP-C₈₈₇₋₉₀₁ | QDILVFYVNFGDLLC |
| TOP1₇₅₀₋₇₆₄ | QREKFAWAIDMADED | CENP-C₂₁₂₋₂₂₆ | KVMLKKIEIDNKVSD |
| TOP1₄₁₉₋₄₃₃ | IQGSIKYIMLNPSSR | CENP-C₆₄₃₋₆₅₇ | EDNIMTAQNVPLKPQ |
| TOP1₅₉₁₋₆₀₅ | YNASITLQQQLKELT | CENP-C₈₃₂₋₈₄₆ | TREIILMDLVRPQDT |
| TOP1₆₉₅₋₇₀₉ | EQLMKLEVQATDREE | CENP-C₁₆₇₋₁₈₁ | TSVSQNVIPSSAQKR |
| TOP1₃₀₅₋₃₁₉ | SQYFKAQTEARKQMS | CENP-C₂₄₆₋₂₆₀ | RIRDSEYEIQRQAKK |
| TOP1₃₄₆₋₃₆₀ | KERIANFKIEPPGLF | CENP-C₈₄₆₋₈₆₀ | TYQFFVKHGELKVYK |
| TOP1₄₁₉₋₄₃₃ | IQGSIKYIMLNPSSR | CENP-C₁₄₉₋₁₆₃ | DEEFYLSVGSPSVLL |
| TOP1₄₂₅₋₄₃₉ | YIMLNPSSRIKGEKD | CENP-C₈₃₃₋₈₄₇ | REIILMDLVRPQDTY |
| TOP1₆₁₄₋₆₂₈ | KILSYNRANRAVAIL | CENP-C₈₄₇₋₈₆₁ | YQFFVKHGELKVYKT |
| CENP-C₂₉₇₋₃₁₁ | KLIEDEFIIDESDQS | | |

### Anti-Phospholipid Syndrome-Relevant Antigens

Anti-phospholipid syndrome-relevant antigens include, but are not limited to, those disclosed in the following **Table 17,** as well as equivalents and/or combinations thereof. Non-limiting exemplary anti-phospholipid syndrome-relevant antigens can be derived from beta-2-glycoprotein 1 (BG2P1 or APOH).

| **Table 17.** | | | |
|---|---|---|---|
| **Peptide** | | **Peptide** | |
| APOH₂₃₅₋₂₄₉ | HDGYSLDGPEEIECT | APOH₂₉₅₋₃₀₉ | KVSFFCKNKEKKCSY |
| APOH₃₀₆₋₃₂₀ | KCSYTEDAQCIDGTI | APOH₄₉₋₆₃ | YSCKPGYVSRGGMRK |
| APOH₂₃₇₋₂₅₁ | GYSLDGPEEIECTKL | APOH₂₆₉₋₂₈₃ | KKATVVYQGERVKIQ |
| APOH₂₉₅₋₃₀₉ | KVSFFCKNKEKKCSY | APOH₂₉₅₋₃₀₉ | KVSFFCKNKEKKCSY |
| APOH₂₈₋₄₂ | DLPFSTVVPLKTFYE | APOH₃₂₁₋₃₅₅ | EVPKCFKEHSSLAFW |
| APOH₁₇₃₋₁₈₇ | ECLPQHAMFGNDTIT | APOH₃₂₂₋₃₃₆ | VPKCFKEHSSLAFWK |
| APOH₂₆₄₋₂₇₈ | CKVPVKKATVVYQGE | APOH₃₂₄₋₃₃₈ | KCFKEHSSLAFWKTD |

### ANCA-Associated Vasculitis-Relevant Antigens

ANCA-associated vasculitis-relevant antigens include, but are not limited to, those disclosed in the following **Table 18,** as well as equivalents and/or combinations thereof. Non-limiting exemplary ANCA-associated vasculitis-relevant antigens can be derived from myeloperoxidase (MPO), proteinase (PRTN3), or bacterial permeability increasing factor (BPI).

| **Table 18.** | | | |
|---|---|---|---|
| **Peptide** | | **Peptide** | |
| MPO₅₀₆₋₅₂₀ | QPFMFRLDNRYQPME | PRTN3₄₄₋₅₈ | SLQMRGNPGSHFCGG |
| MPO₃₀₂₋₃₁₆ | RIKNQADCIPFFRSC | PRTN3₂₃₄₋₂₄₈ | TRVALYVDWIRSTLR |
| MPO₇₋₂₁ | SSLRCMVDLGPCWAG | PRTN3₅₉₋₇₃ | TLIHPSFVLTAAHCL |
| MPO₆₈₉₋₇₀₃ | QQRQALAQISLPRII | PRTN3₁₁₇₋₁₃₁ | NDVLLIQLSSPANLS |
| MPO₂₄₈₋₂₆₂ | RSLMFMQWGQLLDHD | PRTN3₁₆₄₋₁₇₈ | DPPAQVLQELNVTVV |
| MPO₄₄₄₋₄₅₈ | QEARKIVGAMVQIIT | PRTN3₇₁₋₈₅ | HCLRDIPQRLVNVVL |
| MPO₅₁₃₋₅₂₇ | DNRYQPMEPNPRVPL | PRTN3₂₄₁₋₂₅₅ | DWIRSTLRRVEAKGR |
| MPO₉₇₋₁₁₁ | ELLSYFKQPVAATRT | PRTN3₅₉₋₇₃ | TLIHPSFVLTAAHCL |
| MPO₆₁₆₋₆₃₀ | QLGTVLRNLKLARKL | PRTN3₁₈₃₋₁₉₇ | RPHNICTFVPRRKAG |
| MPO₄₆₂₋₄₇₆ | YLPLVLGPTAMRKYL | PRTN3₆₂₋₇₆ | HPSFVLTAAHCLRDI |
| MPO₆₁₇₋₆₃₁ | LGTVLRNLKLARKLM | PRTN3₁₁₈₋₁₃₂ | DVLLIQLSSPANLSA |
| MPO₇₁₄₋₇₂₈ | KNNIFMSNSYPRDFV | PRTN3₂₃₉₋₂₅₃ | YVDWIRSTLRRVEAK |

### Stiff Man Syndrome-Relevant Antigens

Stiff Man Syndrome-relevant antigens include, but are not limited to, those disclosed in the following **Table 14,** as well as equivalents and/or combinations thereof. Non-limiting exemplary Stiff Man Syndrome-relevant antigens can be derived from glutamate decarboxylase (GAD). In some embodiments, GAD includes, but is not limited to, GAD65.

| **Table 19.** | |
|---|---|
| **Peptide** | |
| GAD₂₁₂₋₂₂₆ | EYVTLKKMREIIGWP |
| GAD₅₅₅₋₅₆₉ | NFFRMVISNPAATHQ |
| GAD₂₉₇₋₃₁₁ | DSVILIKCDERGKMI |

It is contemplated that in compositions of the disclosure, there is between about 0.001 mg and about 10 mg of total protein per ml in the composition. Thus, the concentration of protein in a composition can be about, at least about or at most about 0.001, 0.010, 0.050, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 50, 100 µg/ml or mg/ml or more (or any range derivable therein). Of this, about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% may be peptide/MHC/nanoparticle complex.

The present disclosure contemplates the administration of a peptide/MHC/nanoparticle complex to effect a diagnosis, treatment or preventative therapy against the development of a disease or condition associated with autoimmune responses or cancer.

In addition, U.S. Patent No. 4,554,101 (Hopp), teaches the identification and preparation of epitopes from primary amino acid sequences on the basis of hydrophilicity. Through the methods disclosed in Hopp, one of skill in the art would be able to identify potential epitopes from within an amino acid sequence and confirm their immunogenicity. Numerous scientific publications have also been devoted to the prediction of secondary structure and to the identification of epitopes, from analyses of amino acid sequences (Chou & Fasman, 1974a,b; 1978a,b; 1979). Any of these may be used, if desired, to supplement the teachings of Hopp in U.S. Patent No. 4,554,101.

### Other Antigenic Components

Molecules other than peptides can be used as antigens or antigenic fragments in complex with MHC molecules. Such molecules include, but are not limited to, carbohydrates, lipids, small molecules, and the like. Carbohydrates are major components of the outer surface of a variety of cells. Certain carbohydrates are characteristic of different stages of differentiation and very often these carbohydrates are recognized by specific antibodies. Expression of distinct carbohydrates can be restricted to specific cell types. Autoantibody responses to endometrial and serum antigens have been shown to be a common feature of endometriosis. There has been described a serum autoantibody response in endometriosis to a number of previously identified antigens, including 2-Heremans Schmidt glycoprotein and carbonic anhydrase, which is specific for a carbohydrate epitope.

### Non-limiting, Exemplary Antigen-MHC Complexes

In certain embodiments, specific combinations of antigen and MHC may be optimized for the treatment of a specific disease. Non-limiting examples include, but are not limited to, the following examples:

For the treatment of type I diabetes, the antigen of the pMHC complex may be derived from an antigen of the group: PPI_{76-90(K88S)}, IGRP₁₃₋₂₅, GAD₅₅₅₋₅₆₇, GAD_{555-567(557I)}, IGRP₂₃₋₃₅, B₂₄-C₃₆, PPI₇₆₋₉₀, or a fragment or an equivalent of each thereof, and the MHC of the pMHC complex comprises all or part of a polypeptide of the group: HLA-DRB 1 *0401/DRA, HLA-DRB 1*0301/DRA, or a fragment or an equivalent of each thereof.

In some embodiments, the antigen of the pMHC complex comprises a:
a) a diabetes-relevant antigen and is derived from an antigen selected from one or more of the group: preproinsulin (PPI), islet-specific glucose-6-phosphatase (IGRP), glutamate decarboxylase (GAD), islet cell autoantigen-2 (ICA2), insulin, proinsulin, or a fragment or an equivalent of each thereof;
b) a multiple sclerosis-relevant antigen and is derived from an antigen selected from one or more of the group: myelin basic protein, myelin associated glycoprotein, myelin oligodendrocyte protein, proteolipid protein, oligodendrocyte myelin oligoprotein, myelin associated oligodendrocyte basic protein, oligodendrocyte specific protein, heat shock proteins, oligodendrocyte specific proteins, NOGO A, glycoprotein Po, peripheral myelin protein 22, 2'3'-cyclic nucleotide 3'-phosphodiesterase, or a fragment or an equivalent of each thereof;
c) a Celiac Disease-relevant antigen and is derived from gliadin or a fragment or an equivalent thereof;
d) a primary biliary cirrhosis-relevant antigen and is derived from PDC-E2 or a fragment or an equivalent thereof;
e) a pemphigus folliaceus-relevant antigen and/or pemphigus vulgaris-relevant antigen and is derived from an antigen selected from one or more of the group: DG1, DG3, or a fragment or an equivalent of each thereof;
f) a neuromyelitis optica spectrum disorder-relevant antigen and is derived from AQP4 or a fragment or an equivalent thereof;
g) an arthritis-relevant antigen and is derived from an antigen selected from one or more of the group: heat shock proteins, immunoglobulin binding protein, heterogeneous nuclear RNPs, annexin V, calpastatin, type II collagen, glucose-6-phosphate isomerase, elongation factor human cartilage gp39, mannose binding lectin, citrullinated vimentin, type II collagen, fibrinogen, alpha enolase, anti-carbamylated protein (anti-CarP), peptidyl arginine deiminase type 4 (PAD4), BRAF, fibrinogen gamma chain, inter-alpha-trypsin inhibitor heavy chain H1, alpha-1-antitrypsin, plasma protease C1 inhibitor, gelsolin, alpha 1-B glycoprotein, ceruloplasmin, inter-alpha-trypsin inhibitor heavy chain H4, complement factor H, alpha 2 macroglobulin, serum amyloid, C-reactive protein, serum albumin, fibrogen beta chain, serotransferin, alpha 2 HS glycoprotein, vimentin, Complement C3, or a fragment or an equivalent of each thereof;
h) an allergic asthma-relevant antigen and is derived from an antigen selected from one or more of the group: DERP1, DERP2, or a fragment or an equivalent of each thereof;
i) an inflammatory bowel disease-relevant antigen and is derived from an antigen selected from one or more of the group: Flagelin, Fla-2, Fla-X, YIDX, bacteroides integrase, or a fragment or an equivalent of each thereof;
j) a systemic lupus erythematosus-relevant antigen and is derived from an antigen selected from one or more of the group: double-stranded (ds)DNA, ribonucleoprotein (RNP), Smith (Sm), Sjögren's-syndrome-related antigen A (SS-A)/Ro, Sjögren's-syndrome-related antigen B (SS-B)/La, RO60, RO52, histones, or a fragment or an equivalent of each thereof;
k) an atherosclerosis-relevant antigen and is derived from an antigen selected from one or more of the group: ApoB, ApoE or a fragment or an equivalent of each thereof;
l) a COPD-relevant antigen and/or emphysema-relevant antigen and is derived from elastin or a fragment or an equivalent thereof;
m) a psoriasis-relevant antigen and is derived from an antigen selected from one or more of the group: Cap18, ADMTSL5, ATL5, or a fragment or an equivalent of each thereof;
n) an autoimmune hepatitis-relevant antigen and is derived from an antigen selected from one or more of the group: CYP2D6, SLA, or a fragment or an equivalent of each thereof;
o) an uveitis-relevant antigen and is derived from arrestin or a fragment or an equivalent thereof;
p) a Sjögren's Syndrome-relevant antigen and is derived from an antigen selected from one or more of the group: (SS-A)/Ro, (SS-B)/La, MR3, RO60, RO52, or a fragment or an equivalent of each thereof;
q) a scleroderma-relevant antigen and is derived from an antigen selected from one or more of the group: CENP-C, TOP 1, RNA polymerase III, or a fragment or an equivalent of each thereof;
r) an anti-phospholipid syndrome-relevant antigen and is derived from APOH or a fragment or an equivalent thereof;
s) an ANCA-associated vasculitis-relevant antigen and is derived from an antigen selected from one or more of the group: MPO, PRTN3, or a fragment or an equivalent of each thereof; or
t) a Stiff Man Syndrome-relevant antigen and is derived from GAD or a fragment or an equivalent thereof.

In some embodiments of the disclosure, the MHC protein of the pMHC complex comprises all or part of a classical MHC class I protein, non-classical MHC class I protein, classical MHC class II protein, non-classical MHC class II protein, MHC dimers (Fc fusions), MHC tetramers, or a polymeric form of a MHC protein, wherein the MHC protein optionally comprises a knob-in-hole based MHC-alpha-Fc/MHC-beta-Fc heterodimer or multimer.

In some embodiments, the MHC protein of the pMHC complex comprises all or part of a polypeptide of the group: HLA DR, HLA DQ, HLA DP, HLA-A, HLA-B, HLA-C, HLA-E, HLA-F, HLA-G, CD1d, or a fragment or an equivalent of each thereof.

In some embodiments, the MHC protein of the pMHC complex comprises all or part of a polypeptide of the group: HLA-DR, HLA-DQ, HLA-DP, or a fragment or an equivalent of each thereof.

In some embodiments, the MHC protein of the pMHC complex comprises all or part of a polypeptide of the group: HLA-DRB1/DRA, HLA-DRB3/DRA, HLA-DRB4/DRA, HLA-DRB5/DRA, HLA-DQA1/HLA-DQB1, HLA-DPB1/HLA-DPA1, or a fragment or an equivalent of each thereof.

In certain aspects, the pMHC complex comprises:
a) a diabetes-relevant antigen derived from an antigen selected from one or more of the group: hInsB₁₀₋₁₈, hIGRP₂₂₈₋₂₃₆, hIGRP₂₆₅₋₂₇₃, IGRP₂₀₆₋₂₁₄, hIGRP₂₀₆₋₂₁₄, NRP-A7, NRP-I4, NRP-V7, YAI/D^{b}, INS B₁₅₋₂₃, PPI_{76-90(K88S)}, IGRP₁₃₋₂₅, GAD₅₅₅₋₅₆₇, GAD_{555-567(557I)}, IGRP₂₃₋₃₅, B₂₄-C₃₆, PPI₇₆₋₉₀, INS-I9, TUM, G6Pase, Pro-insulin_{L2-10}, Pro-insulin_{L3-11}, Pro-insulin_{L6-14}, Proinsulin_{B5-14}, Pro-insulin_{B10-18}, Pro-insulin_{B14-22}, Pro-insulin_{B15-24}, Pro-insulin_{B17-25}, Pro-insulin_{B18-27}, Pro-insulin_{B20-27}, Pro-insulin_{B21-29}, Pro-insulin_{B25-C1}, Pro-insulin_{B27-C5}, Pro-insulin_{C20-28}, Proinsulin_{C25-33}, Pro-insulin_{C29-A5}, Pro-insulin_{A1-10}, Pro-insulin_{A2-10}, Pro-insulin_{A12-20}, or a fragment or an equivalent of each thereof;
b) a multiple sclerosis-relevant antigen derived from an antigen selected from one or more of the group: MOG₃₅₋₅₅, MOG₃₆₋₅₅, MAG₂₈₇₋₂₉₅, MAG₅₀₉₋₅₁₇, MAG₅₅₆₋₅₆₄, MBP₁₁₀₋₁₁₈, MOG₁₁₄₋₁₂₂, MOG₁₆₆₋₁₇₅, MOG₁₇₂₋₁₈₀, MOG₁₇₉₋₁₈₈, MOG₁₈₈₋₁₉₆, MOG₁₈₁₋₁₈₉, MOG₂₀₅₋₂₁₄, PLP₈₀₋₈₈, MAG₂₈₇₋₂₉₅, MAG₅₀₉₋₅₁₇, MAG₅₅₆₋₅₆₄, MOG₉₇₋₁₀₉ MOG_{97-109(E107S)}, MBP₈₉₋₁₀₁, PLP₁₇₅₋₁₉₂, PLP₉₄₋₁₀₈, MBP₈₆₋₉₈, PLP₅₄₋₆₈, PLP₂₄₉₋₂₆₃, MOG₁₅₆₋₁₇₀, MOG₂₀₁₋₂₁₅, MOG₃₈₋₅₂, MOG₂₀₃₋₂₁₇, PLP₂₅₀₋₂₆₄, MPB₁₃₋₃₂, MPB₈₃₋₉₉, MPB₁₁₁₋₁₂₉, MPB₁₄₆₋₁₇₀, MOG₂₂₃₋₂₃₇, MOG₆₋₂₀, PLP₈₈₋₁₀₂, PLP₁₃₉₋₁₅₄, or a fragment or an equivalent of each thereof;
c) a Celiac Disease-relevant antigen derived from an antigen selected from one or more of the group: aGlia₅₇₋₆₈, aGlia₆₂₋₇₂, aGlia₂₁₇₋₂₂₉, or a fragment or an equivalent of each thereof;
d) a primary biliary cirrhosis-relevant antigen derived from an antigen selected from one or more of the group: PDC-E2₁₂₂₋₁₃₅, PDC-E2₂₄₉₋₂₆₂, PDC-E2₂₄₉₋₂₆₃, PDC-E2₆₂₉₋₆₄₃, PDC-E2₇₂₋₈₆, PDC-E2₃₅₃₋₃₆₇, PDC-E2₄₂₂₋₄₃₆, PDC-E2₆₂₉₋₆₄₃, PDC-E2₈₀₋₉₄, PDC-E2₃₅₃₋₃₆₇, PDC-E2₅₃₅₋₅₄₉, or a fragment or an equivalent of each thereof;
e) a pemphigus folliaceus-relevant antigen and/or pemphigus vulgaris-relevant antigen, each of which is derived from an antigen selected from one or more of the group: DG1₂₁₆₋₂₂₉, DG3₉₇₋₁₁₁, DG3₂₅₁₋₂₆₅, DG3₄₄₁₋₄₅₅,DG3₃₅₁₋₃₆₅, DG3₄₅₃₋₄₆₇, DG3₅₄₀₋₅₅₄, DG3₂₈₀₋₂₉₄, DG3₃₂₆₋₃₄₀, DG3₃₆₇₋₃₈₁, DG3₁₃₋₂₇, DG3₃₂₃₋₃₃₇, DG3₄₃₈₋₄₅₂, DG1₄₈₋₆₂, DG1₂₀₆₋₂₂₂, DG1₃₆₃₋₃₇₇, DG1₃₋₁₇, DG1₁₉₂₋₂₀₆, DG1₃₂₆₋₃₄₀, DG1₁₋₁₅, DG1₃₅₋₄₉, DG1₃₂₅₋₃₃₉, or a fragment or an equivalent of each thereof;
f) a neuromyelitis optica spectrum disorder-relevant antigen derived from an antigen selected from one or more of the group: AQP4₁₂₉₋₁₄₃, AQP4₂₈₄₋₂₉₈, AQP4₆₃₋₇₆, AQP4₁₂₉₋₁₄₃, AQP4₃₉₋₅₃, or a fragment or an equivalent of each thereof;
g) an allergic asthma-relevant antigen derived from an antigen selected from one or more of the group: DERP1₁₆₋₃₀, DERP1₁₇₁₋₁₈₅, DERP 1₁₁₀₋₁₂₄, DERP-2₂₆₋₄₀, DERP-2 ₁₀₇₋₁₂₁, or a fragment or an equivalent of each thereof;
h) an inflammatory bowel disease-relevant antigen derived from an antigen selected from one or more of the group: bacteroides integrase antigen₁₈₃₋₁₉₇, bacteroides integrase antigen₁₄₆₋₁₆₀, bacteroides integrase antigen₁₇₅₋₁₈₉, bacteroides integrase antigen₁₋₁₅, bacteroides integrase antigen₁₈₃₋₁₉₇, bacteroides integrase antigen₃₀₋₄₄, bacteroides integrase antigen₇₀₋₈₄, bacteroides integrase antigen₃₃₇₋₃₅₁, bacteroides integrase antigen₁₇₁₋₁₈₅, bacteroides integrase antigen₄₋₁₈, bacteroides integrase antigen₂₅₆₋₂₇₀, Fla-2/Fla-X₃₆₆₋₃₈₀, Fla-2/Fla-X₁₆₄₋₁₇₈, Fla-2/Fla-X₂₆₁₋₂₇₅, Fla-2/Fla-X₁₋₁₅, Fla-2/Fla-X₅₁₋₆₅, Fla-2/Fla-X₂₆₉₋₂₈₃, Fla-2/Fla-X₄₋₁₈, Fla-2/Fla-X₂₇₁₋₂₈₅, YIDX₇₈₋₉₂, YIDX₉₃₋₁₀₇, YIDX₉₈₋₁₁₂, YIDX₂₃₋₃₇, YIDX₇₈₋₉₂, YIDX₁₉₅₋₂₀₉, YIDX₂₂₋₃₆, YIDX₈₀₋₉₄, YIDX₁₀₁₋₁₁₅, or a fragment or an equivalent of each thereof;
i) a systemic lupus erythematosus-relevant antigen derived from an antigen selected from one or more of the group: H4₇₁₋₉₄, H4₇₄₋₈₈, H4₇₆₋₉₀, H4₇₅₋₈₉, H4₇₈₋₉₂, H4₈₀₋₉₄, H2B₁₀₋₂₄, H2B₁₆₋₃₀, H1'₂₂₋₄₂, H1'₂₇₋₄₁, or a fragment or an equivalent of each thereof;
j) an atherosclerosis-relevant antigen derived from an antigen selected from one or more of the group: ApoB₃₅₀₁₋₃₅₁₆, ApoB₁₉₅₂₋₁₉₆₆, ApoB₉₇₈₋₉₉₃, ApoB₃₄₉₈₋₃₅₁₃, ApoB_{210A}, ApoB_{210B}, ApoB_{210C}, or a fragment or an equivalent of each thereof;
k) a COPD-relevant antigen and/or emphysema-relevant antigen, each of which is derived from an antigen selected from one or more of the group: elastin₈₉₋₁₀₃, elastin₆₉₈₋₇₁₂, elastin₈₋₂₂, elastin₉₄₋₁₀₈, elastin₁₃₋₂₇, elastin₆₉₅₋₇₀₉, elastin₅₆₃₋₅₇₇, elastin₅₅₈₋₅₇₂, elastin₆₉₈₋₇₁₂, elastin₅₆₆₋₅₈₀, elastin₆₄₅₋₆₅₉, or a fragment or an equivalent of each thereof;
l) a psoriasis-relevant antigen derived from an antigen selected from one or more of the group: Cap18₆₄₋₇₈, Cap18₃₄₋₄₈, Cap18₄₇₋₆₁, Cap18₁₅₁₋₁₆₅, Cap18₁₄₉₋₁₆₃, Cap18₁₅₂₋₁₆₆, Cap18₁₃₁₋₁₄₅, Cap₁₈₂₄₋₃₈, ADMTSL5245₋₂₅₉, ADMTSL5₂₆₇₋₂₈₁, ADMTSL5₃₇₂₋₃₈₆, ADMTSL5₂₈₉₋₃₀₃, ADMTSL5₃₉₆₋₄₁₀, ADMTSL5₄₃₃₋₄₄₇, ADMTSL5₁₄₂₋₁₅₆, ADMTSL5₂₃₆₋₂₅₀, ADMTSL5₃₀₁₋₃₁₅, ADMTSL5₂₀₃₋₂₁₇, ADMTSL5₄₀₄₋₄₁₈, or a fragment or an equivalent of each thereof;
m) an autoimmune hepatitis-relevant antigen derived from an antigen selected from one or more of the group: (CYP2D6)₁₉₃₋₂₀₇, CYP2D6₇₆₋₉₀, CYP2D6₂₉₃₋₃₀₇, CYP2D6₃₁₃₋₃₃₂, CYP2D6₃₉₃₋₄₁₂, CYP2D6₁₉₉₋₂₁₃, CYP2D6₄₅₀₋₄₆₄, CYP2D6₃₀₁₋₃₁₅, CYP2D6₄₅₂₋₄₆₆, CYP2D6₅₉₋₇₃, CYP2D6₁₃₀₋₁₄₄, CYP2D6₁₉₃₋₂₁₂, CYP2D6₃₀₅₋₃₂₄, CYP2D6₁₃₁₋₁₄₅, CYP2D6₂₁₆₋₂₃₀, CYP2D6₂₃₈₋₂₅₂, CYP2D6₁₉₉₋₂₁₃, CYP2D6₂₃₅₋₂₅₂, CYP2D6₂₉₃₋₃₀₇, CYP2D6₃₈₁₋₃₉₅, CYP2D6₄₂₉₋₄₄₃, SLA₃₃₄₋₃₄₈, SLA₁₉₆₋₂₁₀, SLA₁₁₅₋₁₂₉, SLA₃₇₃₋₃₈₆, SLA₁₈₆₋₁₉₇, SLA₃₁₇₋₃₃₁, SLA₁₇₁₋₁₈₅, SLA₄₁₇₋₄₃₁, SLA₃₅₉₋₃₇₃, SLA₂₁₅₋₂₂₉, SLA₁₁₁₋₁₂₅, SLA₁₁₀₋₁₂₄, SLA₂₉₉₋₃₁₃, SLA₃₄₂₋₃₅₆, SLA₄₉₋₆₃, SLA₁₁₉₋₁₃₃, SLA₂₆₀₋₂₇₄, SLA₂₆₋₄₀, SLA₈₆₋₁₀₀, SLA₃₃₁₋₃₄₅, or a fragment or an equivalent of each thereof;
n) an uveitis-relevant antigen derived from an antigen selected from one or more of the group: arrestin₁₉₉₋₂₁₃, arrestin₇₇₋₉₁, arrestin₂₅₀₋₂₆₄, arrestin₁₇₂₋₁₈₆, arrestin₃₅₄₋₃₆₈, arrestin₂₃₉₋₂₅₃, arrestin₁₀₂₋₁₁₆, arrestin₅₉₋₇₃, arrestin₂₈₀₋₂₉₄, arrestin₂₉₁₋₃₀₆, arrestin₁₉₅₋₂₀₉, arrestin₂₀₀₋₂₁₄, or a fragment or an equivalent of each thereof;
o) a Sjögren's Syndrome-relevant antigen derived from an antigen selected from one or more of the group: RO60₁₂₇₋₁₄₁, RO60₅₂₃₋₅₃₇, RO60₂₄₃₋₂₅₇, RO60₄₈₄₋₄₉₈, RO60₃₄₇₋₃₆₁, RO60₃₆₉₋₃₈₃, R060₄₂₆₋₄₄₀, RO60₂₆₇₋₂₈₁, RO60₁₇₈₋₁₉₂, R060₃₅₈₋₃₇₂, RO60₂₂₁₋₂₃₅, R060₃₁₈₋₃₃₂, RO60₄₀₇-₄₂₁, RO60₄₅₉₋₄₇₃, R060₅₁₋₆₅, RO60₃₁₂₋₃₂₆, LA₂₄₁₋₂₅₅, LA₁₀₁₋₁₁₅, LA₁₅₃₋₁₆₇, LA₁₇₈₋₁₉₂, LA₁₉₋₃₃, LA₃₇₋₅₁, LA₁₃₃₋₁₄₇, LA₅₀₋₆₄, LA₃₂₋₄₆, LA₁₅₃₋₁₆₇, LA₈₃₋₉₇, LA₁₃₆₋₁₅₀, LA₂₉₇₋₃₁₁, LA₅₉₋₇₃, LA₁₅₁₋₁₆₅, LA₈₆₋₁₀₀, LA₁₅₄₋₁₆₈, or a fragment or an equivalent of each thereof;
p) a scleroderma-relevant antigen derived from an antigen selected from one or more of the group: TOP1₃₄₆₋₃₆₀, TOP1₄₂₀₋₄₃₄, TOP1₇₅₀₋₇₆₄, TOP1₄₁₉₋₄₃₃, TOP1₅₉₁₋₆₀₅, TOP1₆₉₅₋₇₀₉, TOP1₃₀₅₋₃₁₉, TOP1₃₄₆₋₃₆₀, TOP1₄₁₉₋₄₃₃, TOP1₄₂₅₋₄₃₉, TOP1₆₁₄₋₆₂₈, CENP-C₂₉₇₋₃₁₁, CENP-C₈₅₇₋₈₇₁, CENP-C₈₈₇₋₉₀₁, CENP-C₂₁₂₋₂₂₆, CENP-C₆₄₃₋₆₅₇, CENP-C₈₃₂₋₈₄₆, CENP-C₁₆₇₋₁₈₁, CENP-C₂₄₆₋₂₆₀, CENP-C₈₄₆₋₈₆₀, CENP-C₁₄₉₋₁₆₃, CENP-C₈₃₃₋₈₄₇, CENP-C₈₄₇₋₈₆₁, or a fragment or an equivalent of each thereof;
q) an anti-phospholipid syndrome-relevant antigen derived from an antigen selected from one or more of the group: APOH₂₃₅₋₂₄₉, APOH₃₀₆₋₃₂₀, APOH₂₃₇₋₂₅₁, APOH₂₉₅₋₃₀₉, APOH₂₈₋₄₂, APOH₁₇₃₋₁₈₇, APOH₂₆₄₋₂₇₈, APOH₂₉₅₋₃₀₉, APOH₄₉₋₆₃, APOH₂₆₉₋₂₈₃, APOH₂₉₅₋₃₀₉, APOH₃₂₁₋₃₅₅, APOH₃₂₂₋₃₃₆, APOH₃₂₄₋₃₃₈, or a fragment or an equivalent of each thereof;
r) an ANCA-associated vasculitis-relevant antigen derived from an antigen selected from one or more of the group: MPO₅₀₆₋₅₂₀, MPO₃₀₂₋₃₁₆, MPO₇₋₂₁, MPO₆₈₉₋₇₀₃, MPO₂₄₈₋₂₆₂, MPO₄₄₄₋₄₅₈, MPO₅₁₃₋₅₂₇, MPO₉₇₋₁₁₁, MPO₆₁₆₋₆₃₀, MPO₄₆₂₋₄₇₆, MPO₆₁₇₋₆₃₁, MPO₇₁₄₋₇₂₈, PRTN3₄₄₋₅₈, PRTN3₂₃₄₋₂₄₈, PRTN3₅₉₋₇₃, PRTN3₁₁₇₋₁₃₁, PRTN3₁₆₄₋₁₇₈, PRTN3₇₁₋₈₅, PRTN3₂₄₁₋₂₅₅, PRTN3₅₉₋₇₃, PRTN3₁₈₃₋₁₉₇, PRTN3₆₂₋₇₆, PRTN3₁₁₈₋₁₃₂, PRTN3₂₃₉₋₂₅₃, or a fragment or an equivalent of each thereof; or
s) a Stiff Man Syndrome-relevant antigen derived from an antigen selected from one or more of the group: GAD₂₁₂₋₂₂₆, GAD₅₅₅₋₅₆₉, GAD₂₉₇₋₃₁₁, or a fragment or an equivalent of each thereof.

In certain aspects, the pMHC complex comprises:
a) a diabetes-relevant antigen derived from an antigen selected from one or more of the group: hInsB₁₀₋₁₈, hIGRP₂₂₈₋₂₃₆, hIGRP₂₆₅₋₂₇₃, IGRP₂₀₆₋₂₁₄, hIGRP₂₀₆₋₂₁₄, NRP-A7, NRP-I4, NRP-V7, YAI/D^{b}, INS B₁₅₋₂₃, PPI_{76-90(K88S)}, IGRP₁₃₋₂₅, GAD₅₅₅₋₅₆₇, GAD_{555-567(557I)}, IGRP₂₃₋₃₅, B₂₄-C₃₆, PPI₇₆₋₉₀, INS-I9, TUM, G6Pase, Pro-insulin_{L2-10}, Pro-insulin_{L3-11}, Pro-insulin_{L6-14}, Proinsulin_{B5-14}, Pro-insulin_{B10-18}, Pro-insulin_{B14-22}, Pro-insulin_{B15-24}, Pro-insulin_{B17-25}, Pro-insulin_{B18-27}, Pro-insulin_{B20-27}, Pro-insulin_{B21-29}, Pro-insulin_{B25-C1}, Pro-insulin_{B27-C5}, Pro-insulin_{C20-28}, Proinsulin_{C25-33}, Pro-insulin_{C29-A5}, Pro-insulin_{A1-10}, Pro-insulin_{A2-10}, Pro-insulin_{A12-20}, or a fragment or an equivalent of each thereof, and the MHC protein of the pMHC complex comprises all or part of HLA-DR or a fragment or an equivalent thereof;
b) a multiple sclerosis-relevant antigen derived from an antigen selected from one or more of the group: MOG₃₅₋₅₅, MOG₃₆₋₅₅, MAG₂₈₇₋₂₉₅, MAG₅₀₉₋₅₁₇, MAG₅₅₆₋₅₆₄, MBP₁₁₀₋₁₁₈, MOG₁₁₄₋₁₂₂, MOG₁₆₆₋₁₇₅, MOG₁₇₂₋₁₈₀, MOG₁₇₉₋₁₈₈, MOG₁₈₈₋₁₉₆, MOG₁₈₁₋₁₈₉, MOG₂₀₅₋₂₁₄, PLP₈₀₋₈₈, MAG₂₈₇₋₂₉₅, MAG₅₀₉₋₅₁₇, MAG₅₅₆₋₅₆₄, MOG₉₇₋₁₀₉ MOG_{97-109(E107S)}, MBP₈₉₋₁₀₁, PLP₁₇₅₋₁₉₂, PLP₉₄₋₁₀₈, MBP₈₆₋₉₈, PLP₅₄₋₆₈, PLP₂₄₉₋₂₆₃, MOG₁₅₆₋₁₇₀, MOG₂₀₁₋₂₁₅, MOG₃₈₋₅₂, MOG₂₀₃₋₂₁₇, PLP₂₅₀₋₂₆₄, MPB₁₃₋₃₂, MPB₈₃₋₉₉, MPB₁₁₁₋₁₂₉, MPB₁₄₆₋₁₇₀, MOG₂₂₃₋₂₃₇, MOG₆₋₂₀, PLP₈₈₋₁₀₂, PLP₁₃₉₋₁₅₄, or a fragment or an equivalent of each thereof, and the MHC protein of the pMHC complex comprises all or part of HLA-DR or a fragment or an equivalent thereof;
c) a Celiac Disease-relevant antigen derived from an antigen selected from one or more of the group: aGlia₅₇₋₆₈, aGlia₆₂₋₇₂, aGlia₂₁₇₋₂₂₉, or a fragment or an equivalent of each thereof, and the MHC protein of the pMHC complex comprises all or part of HLA-DQ or a fragment or an equivalent thereof;
d) a primary biliary cirrhosis-relevant antigen derived from an antigen selected from one or more of the group: PDC-E2₁₂₂₋₁₃₅, PDC-E2₂₄₉₋₂₆₂, PDC-E2₂₄₉₋₂₆₃, PDC-E2₆₂₉₋₆₄₃, PDC-E2₇₂₋₈₆, PDC-E2₃₅₃₋₃₆₇, PDC-E2₄₂₂₋₄₃₆, PDC-E2₆₂₉₋₆₄₃, PDC-E2₈₀₋₉₄, PDC-E2₃₅₃₋₃₆₇, PDC-E2₅₃₅₋₅₄₉, or a fragment or an equivalent of each thereof, and the MHC protein of the pMHC complex comprises all or part of HLA-DR or a fragment of an equivalent thereof;
e) a pemphigus folliaceus-relevant antigen and/or pemphigus vulgaris-relevant antigen, each of which is derived from an antigen selected from one or more of the group: DG1₂₁₆₋₂₂₉, DG3₉₇₋₁₁₁, DG3₂₅₁₋₂₆₅, DG3₄₄₁₋₄₅₅,DG3₃₅₁-₃₆₅, DG3₄₅₃₋₄₆₇, DG3₅₄₀₋₅₅₄, DG3₂₈₀₋₂₉₄, DG3₃₂₆₋₃₄₀, DG3₃₆₇₋₃₈₁, DG3₁₃₋₂₇, DG3₃₂₃₋₃₃₇, DG3₄₃₈₋₄₅₂, DG1₄₈₋₆₂, DG1₂₀₆₋₂₂₂, DG1₃₆₃₋₃₇₇, DG1₃₋₁₇, DG1₁₉₂₋₂₀₆, DG1₃₂₆₋₃₄₀, DG1₁₋₁₅, DG1₃₅₋₄₉, DG1₃₂₅₋₃₃₉, or a fragment or an equivalent of each thereof, and the MHC protein of the pMHC complex comprises all or part of HLA-DR or a fragment or an equivalent thereof;
f) a neuromyelitis optica spectrum disorder-relevant antigen derived from an antigen selected from one or more of the group: AQP4₁₂₉₋₁₄₃, AQP4₂₈₄₋₂₉₈, AQP4₆₃₋₇₆, AQP4₁₂₉₋₁₄₃, AQP4₃₉₋₅₃, or a fragment or an equivalent of each thereof, and the MHC protein of the pMHC complex comprises all or part of HLA-DR or a fragment or an equivalent thereof;
g) an allergic asthma-relevant antigen derived from an antigen selected from one or more of the group: DERP1₁₆₋₃₀, DERP1₁₇₁₋₁₈₅, DERP1₁₁₀₋₁₂₄, DERP-2₂₆₋₄₀, DERP-2 ₁₀₇₋₁₂₁, or a fragment or an equivalent of each thereof, and the MHC protein of the pMHC complex comprises all or part of a polypeptide of the group: HLA-DR, HLA-DP, or a fragment or an equivalent of each thereof;
h) an inflammatory bowel disease-relevant antigen derived from an antigen selected from one or more of the group: bacteroides integrase antigen₁₈₃₋₁₉₇, bacteroides integrase antigen₁₄₆₋₁₆₀, bacteroides integrase antigen₁₇₅₋₁₈₉, bacteroides integrase antigen₁₋₁₅, bacteroides integrase antigen₁₈₃₋₁₉₇, bacteroides integrase antigen₃₀₋₄₄, bacteroides integrase antigen₇₀₋₈₄, bacteroides integrase antigen₃₃₇₋₃₅₁, bacteroides integrase antigen₁₇₁₋₁₈₅, bacteroides integrase antigen₄₋₁₈, bacteroides integrase antigen₂₅₆₋₂₇₀, Fla-2/Fla-X₃₆₆₋₃₈₀, Fla-2/Fla-X₁₆₄₋₁₇₈, Fla-2/Fla-X₂₆₁₋₂₇₅, Fla-2/Fla-X₁₋₁₅, Fla-2/Fla-X₅₁₋₆₅, Fla-2/Fla-X₂₆₉₋₂₈₃, Fla-2/Fla-X₄₋₁₈, Fla-2/Fla-X₂₇₁₋₂₈₅, YIDX₇₈₋₉₂, YIDX₉₃₋₁₀₇, YIDX₉₈₋₁₁₂, YIDX₂₃₋₃₇, YIDX₇₈₋₉₂, YIDX₁₉₅₋₂₀₉, YIDX₂₂₋₃₆, YIDX₈₀₋₉₄, YIDX₁₀₁₋₁₁₅, or a fragment or an equivalent of each thereof, and the MHC protein of the pMHC complex comprises all or part of HLA-DR or a fragment or an equivalent thereof;
i) a systemic lupus erythematosus-relevant antigen derived from an antigen selected from one or more of the group: H4₇₁₋₉₄, H4₇₄₋₈₈, H4₇₆₋₉₀, H4₇₅₋₈₉, H4₇₈₋₉₂, H4₈₀₋₉₄, H2B₁₀₋₂₄, H2B₁₆₋₃₀, H1'₂₂₋₄₂, H1'₂₇₋₄₁, or a fragment or an equivalent of each thereof, and the MHC protein of the pMHC complex comprises all or part of a polypeptide of the group: I-A_{d}, HLA-DR, or a fragment or an equivalent of each thereof;
j) an atherosclerosis-relevant antigen derived from an antigen selected from one or more of the group: ApoB₃₅₀₁₋₃₅₁₆, ApoB₁₉₅₂₋₁₉₆₆, ApoB₉₇₈₋₉₉₃, ApoB₃₄₉₈₋₃₅₁₃, ApoB_{210A}, ApoB_{210B}, ApoB_{210C}, or a fragment or an equivalent of each thereof, and the MHC protein of the pMHC complex comprises all or part of I-A_{b} or a fragment or an equivalent thereof;
k) a COPD-relevant antigen and/or emphysema-relevant antigen, each of which is derived from an antigen selected from one or more of the group: elastin₈₉₋₁₀₃, elastin₆₉₈₋₇₁₂, elastin₈₋₂₂, elastin₉₄₋₁₀₈, elastin₁₃₋₂₇, elastin₆₉₅₋₇₀₉, elastin₅₆₃₋₅₇₇, elastin₅₅₈₋₅₇₂, elastin₆₉₈₋₇₁₂, elastin₅₆₆₋₅₈₀, elastin₆₄₅₋₆₅₉, or a fragment or an equivalent of each thereof, and the MHC protein of the pMHC complex comprises all or part of HLA-DR or a fragment or an equivalent thereof;
l) a psoriasis-relevant antigen derived from an antigen selected from one or more of the group: Cap18₆₄₋₇₈, Cap18₃₄₋₄₈, Cap18₄₇₋₆₁, Cap18₁₅₁₋₁₆₅, Cap18₁₄₉₋₁₆₃, Cap18₁₅₂₋₁₆₆, Cap18₁₃₁₋₁₄₅, Cap₁₈₂₄₋₃₈, ADMTSL5245₋₂₅₉, ADMTSL5₂₆₇₋₂₈₁, ADMTSL5₃₇₂₋₃₈₆, ADMTSL5₂₈₉₋₃₀₃, ADMTSL5₃₉₆₋₄₁₀, ADMTSL5₄₃₃₋₄₄₇, ADMTSL5₁₄₂₋₁₅₆, ADMTSL5₂₃₆₋₂₅₀, ADMTSL5₃₀₁₋₃₁₅, ADMTSL5₂₀₃₋₂₁₇, ADMTSL5₄₀₄₋₄₁₈, or a fragment or an equivalent of each thereof, and the MHC protein of the pMHC complex comprises all or part of HLA-DR or a fragment or an equivalent thereof;
m) an autoimmune hepatitis-relevant antigen derived from an antigen selected from one or more of the group: CYP2D6₁₉₃₋₂₀₇, CYP2D6₇₆₋₉₀, CYP2D6₂₉₃₋₃₀₇, CYP2D6₃₁₃₋₃₃₂, CYP2D6₃₉₃₋₄₁₂, CYP2D6₁₉₉₋₂₁₃, CYP2D6₄₅₀₋₄₆₄, CYP2D6₃₀₁₋₃₁₅, CYP2D6₄₅₂₋₄₆₆, CYP2D6₅₉₋₇₃, CYP2D6₁₃₀₋₁₄₄, CYP2D6₁₉₃₋₂₁₂, CYP2D6₃₀₅₋₃₂₄, CYP2D6₁₃₁₋₁₄₅, CYP2D6₂₁₆₋₂₃₀, CYP2D6₂₃₈₋₂₅₂, CYP2D6₁₉₉₋₂₁₃, CYP2D6₂₃₅₋₂₅₂, CYP2D6₂₉₃₋₃₀₇, CYP2D6₃₈₁₋₃₉₅, CYP2D6₄₂₉₋₄₄₃, SLA₃₃₄₋₃₄₈, SLA₁₉₆₋₂₁₀, SLA₁₁₅₋₁₂₉, SLA₃₇₃₋₃₈₆, SLA₁₈₆₋₁₉₇, SLA₃₁₇₋₃₃₁, SLA₁₇₁₋₁₈₅, SLA₄₁₇₋₄₃₁, SLA₃₅₉₋₃₇₃, SLA₂₁₅₋₂₂₉, SLA₁₁₁₋₁₂₅, SLA₁₁₀₋₁₂₄, SLA₂₉₉₋₃₁₃, SLA₃₄₂₋₃₅₆, SLA₄₉₋₆₃, SLA₁₁₉₋₁₃₃, SLA₂₆₀₋₂₇₄, SLA₂₆₋₄₀, SLA₈₆₋₁₀₀, SLA₃₃₁₋₃₄₅, or a fragment or an equivalent of each thereof, and the MHC protein of the pMHC complex comprises all or part of HLA-DR or a fragment or an equivalent thereof;
n) an uveitis-relevant antigen derived from an antigen selected from one or more of the group: arrestin₁₉₉₋₂₁₃, arrestin₇₇₋₉₁, arrestin₂₅₀₋₂₆₄, arrestin₁₇₂₋₁₈₆, arrestin₃₅₄₋₃₆₈, arrestin₂₃₉₋₂₅₃, arrestin₁₀₂₋₁₁₆, arrestin₅₉₋₇₃, arrestin₂₈₀₋₂₉₄, arrestin₂₉₁₋₃₀₆, arrestin₁₉₅₋₂₀₉, arrestin₂₀₀₋₂₁₄, or a fragment or an equivalent of each thereof, and the MHC protein of the pMHC complex comprises all or part of HLA-DR or a fragment or an equivalent thereof;
o) a Sjögren's Syndrome-relevant antigen derived from an antigen selected from one or more of the group: RO60₁₂₇₋₁₄₁, RO60₅₂₃₋₅₃₇, RO60₂₄₃₋₂₅₇, RO60₄₈₄₋₄₉₈, RO60₃₄₇₋₃₆₁, RO60₃₆₉₋₃₈₃, RO60₄₂₆₋₄₄₀, RO60₂₆₇₋₂₈₁, RO60₁₇₈₋₁₉₂, RO60₃₅₈₋₃₇₂, RO60₂₂₁₋₂₃₅, RO60₃₁₈₋₃₃₂, RO60₄₀₇₋₄₂₁, RO60₄₅₉₋₄₇₃, RO60₅₁₋₆₅, RO60₃₁₂₋₃₂₆, LA₂₄₁-₂₅₅, LA₁₀₁₋₁₁₅, LA₁₅₃₋₁₆₇, LA₁₇₈₋₁₉₂, LA₁₉₋₃₃, LA₃₇₋₅₁, LA₁₃₃₋₁₄₇, LA₅₀₋₆₄, LA₃₂₋₄₆, LA₁₅₃₋₁₆₇, LA₈₃₋₉₇, LA₁₃₆₋₁₅₀, LA₂₉₇₋₃₁₁, LA₅₉₋₇₃, LA₁₅₁₋₁₆₅, LA₈₆₋₁₀₀, LA₁₅₄₋₁₆₈, or a fragment or an equivalent of each thereof, and the MHC protein of the pMHC complex comprises all or part of a polypeptide of the group: HLA-DR, HLA-DP, or a fragment or an equivalent of each thereof;
p) a scleroderma-relevant antigen derived from an antigen selected from one or more of the group: TOP1₃₄₆₋₃₆₀, TOP1₄₂₀₋₄₃₄, TOP1₇₅₀₋₇₆₄, TOP1₄₁₉₋₄₃₃, TOP1₅₉₁₋₆₀₅, TOP1₆₉₅₋₇₀₉, TOP1₃₀₅₋₃₁₉, TOP1₃₄₆₋₃₆₀, TOP1₄₁₉₋₄₃₃, TOP1₄₂₅₋₄₃₉, TOP1₆₁₄₋₆₂₈, CENP-C₂₉₇₋₃₁₁, CENP-C₈₅₇₋₈₇₁, CENP-C₈₈₇₋₉₀₁, CENP-C₂₁₂₋₂₂₆, CENP-C₆₄₃₋₆₅₇, CENP-C₈₃₂₋₈₄₆, CENP-C₁₆₇₋₁₈₁, CENP-C₂₄₆₋₂₆₀, CENP-C₈₄₆₋₈₆₀, CENP-C₁₄₉₋₁₆₃, CENP-C₈₃₃₋₈₄₇, CENP-C₈₄₇₋₈₆₁, or a fragment or an equivalent of each thereof, and the MHC protein of the pMHC complex comprises all or part of HLA-DR or a fragment or an equivalent thereof;
q) an anti-phospholipid syndrome-relevant antigen derived from an antigen selected from one or more of the group: APOH₂₃₅₋₂₄₉, APOH₃₀₆₋₃₂₀, APOH₂₃₇₋₂₅₁, APOH₂₉₅₋₃₀₉, APOH₂₈₋₄₂, APOH₁₇₃₋₁₈₇, APOH₂₆₄₋₂₇₈, APOH₂₉₅₋₃₀₉, APOH₄₉₋₆₃, APOH₂₆₉₋₂₈₃, APOH₂₉₅₋₃₀₉, APOH₃₂₁₋₃₅₅, APOH₃₂₂₋₃₃₆, APOH₃₂₄₋₃₃₈, or a fragment or an equivalent of each thereof, and the MHC protein of the pMHC complex comprises all or part of HLA-DR or a fragment or an equivalent thereof;
r) an ANCA-associated vasculitis-relevant antigen derived from an antigen selected from one or more of the group: MPO₅₀₆₋₅₂₀, MPO₃₀₂₋₃₁₆, MPO₇₋₂₁, MPO₆₈₉₋₇₀₃, MPO₂₄₈₋₂₆₂, MPO₄₄₄₋₄₅₈, MPO₅₁₃₋₅₂₇, MPO₉₇₋₁₁₁, MPO₆₁₆₋₆₃₀, MPO₄₆₂₋₄₇₆, MPO₆₁₇₋₆₃₁, MPO₇₁₄₋₇₂₈, PRTN3₄₄₋₅₈, PRTN3₂₃₄₋₂₄₈, PRTN3₅₉₋₇₃, PRTN3₁₁₇₋₁₃₁, PRTN3₁₆₄₋₁₇₈, PRTN3₇₁₋₈₅, PRTN3₂₄₁₋₂₅₅, PRTN3₅₉₋₇₃, PRTN3₁₈₃₋₁₉₇, PRTN3₆₂₋₇₆, PRTN3₁₁₈₋₁₃₂, PRTN3₂₃₉₋₂₅₃, or a fragment or an equivalent of each thereof, and the MHC protein of the pMHC complex comprises all or part of HLA-DR or a fragment or an equivalent thereof; or
s) a Stiff Man Syndrome-relevant antigen derived from an antigen selected from one or more of the group: GAD₂₁₂₋₂₂₆, GAD₅₅₅₋₅₆₉, GAD₂₉₇₋₃₁₁, and the MHC protein of the pMHC complex comprises all or part of a polypeptide of the group: HLA-DR, HLA-DQ, or a fragment or an equivalent of each thereof.

In certain aspects, the pMHC complex is for the treatment of:
a) type I diabetes and the pMHC complex is selected from the group of: PPI_{76-90(K88S)}-HLA-DRB1*0401/DRA, IGRP₁₃₋₂₅-HLA-DRB1*0301/DRA, GAD₅₅₅₋₅₆₇-HLA-DRB1*0401/DRA, GAD_{555-567(557I)}-HLA-DRB1*0401/DRA, IGRP₂₃₋₃₅-HLA-DRB1*0401/DRA, B₂₄-C₃₆-HLA-DRB1*0301/DRA, or PPI₇₆₋₉₀-HLA-DRB1*0401/DRA;
b) multiple sclerosis and the pMHC complex is selected from the group of: MBP₈₆₋₉₈-HLA-DRB1*1501/DRA, MBP₈₉₋₁₀₁-HLA-DRB5*0101/DRA, MOG₃₈₋₅₂-HLA-DRB4*0101/DRA, MOG_{97-109(E107S)}-HLA-DRB1*0401/DRA, MOG₂₀₃₋₂₁₇-HLA-DRB3*0101/DRA, PLP₅₄₋₆₈-HLA-DRB3*0101/DRA, PLP₉₄₋₁₀₈-HLA-DRB1*0301/DRA, PLP₂₅₀₋₂₆₄-HLA-DRB4*0101/DRA, MPB₁₃₋₃₂-HLA-DRB5*0101/DRA, MPB₈₃₋₉₉-HLA-DRB5*0101/DRA, MPB₁₁₁₋₁₂₉-HLA-DRB5*0101/DRA, MPB₁₄₆₋₁₇₀-HLA-DRB5*0101/DRA, MOG₂₂₃₋₂₃₇-HLA-DRB3*0202/DRA, MOG₆₋₂₀-HLA-DRB5*0101/DRA, PLP₈₈₋₁₀₂-HLA-DRB3*0202/DRA, or PLP₁₃₉₋₁₅₄-HLA-DRB5*0101/DRA;
c) Celiac Disease and the pMHC complex is selected from the group of: aGlia₅₇₋₆₈- HLA-DQA1*0501/HLA-DQB1*0201, aGlia₆₂₋₇₂- HLA-DQA1*0501/HLA-DQB1*0201, aGlia₂₁₇₋₂₂₉-HLA-DQA1*0501/HLA-DQB1*0302, or aGlia₂₁₇₋₂₂₉-HLA-DQA1*03/HLA-DQB1*0302;
d) primary biliary cirrhosis and the pMHC complex is selected from the group of: PDC-E2₁₂₂₋₁₃₅-HLA-DRB4*0101/DRA, PDC-E2₂₄₉₋₂₆₂-HLA-DRB4*0101/DRA, PDC-E2₂₄₉₋₂₆₃-HLA-DRB1*0801/DRA, PDC-E2₆₂₉₋₆₄₃-HLA-DRB1*0801/DRA, PDC-E2₇₂₋₈₆-HLA-DRB3*0202/DRA, PDC-E2₃₅₃₋₃₆₇-HLA-DRB3*0202/DRA, PDC-E2₄₂₂₋₄₃₆-HLA-DRB3*0202/DRA, PDC-E2₆₂₉₋₆₄₃-HLA-DRB4*0101/DRA, PDC-E2₈₀₋₉₄-HLA-DRB5*0101/DRA, PDC-E2₃₅₃₋₃₆₇-HLA-DRB5*0101/DRA, or PDC-E2₅₃₅₋₅₄₉-HLA-DRB5*0101/DRA, mPDC-E2₁₆₆₋₁₈₁-I-A_{g7}, or mPDC-E2₈₂₋₉₆-I-A_{g7};
e) pemphigus folliaceus and/or pemphigus vulgaris and the pMHC complex is selected from the group of: DG1₂₁₆₋₂₂₉-HLA-DRB1*0101/DRA, DGb₁₆₋₂₂₉-HLA-DRB1*0102/DRA, DG3₉₇₋₁₁₁-HLA-DRB1*0402/DRA, DG3₂₅₁₋₂₆₅-HLA-DRB1*0402/DRA, DG3₂₅₁₋₂₆₅-HLA-DRB1*0401/DRA, DG3₄₄₁₋₄₅₅-HLA-DRB1*0402/DRA, DG3₃₅₁₋₃₆₅-HLA-DRB3*0202/DRA, DG3₄₅₃₋₄₆₇-HLA-DRB3*0202/DRA, DG3₅₄₀₋₅₅₄-HLA-DRB3*0202/DRA, DG3₂₈₀₋₂₉₄-HLA-DRB4*0101/DRA, DG3₃₂₆₋₃₄₀-HLA-DRB4*0101/DRA, DG3₃₆₇₋₃₈₁-HLA-DRB4*0101/DRA, DG3₁₃₋₂₇-HLA-DRB5*0101/DRA, DG3₃₂₃₋₃₃₇-HLA-DRB5*0101/DRA, DG3₄₃₈₋₄₅₂-HLA-DRB5*0101/DRA, DG1₄₈₋₆₂-HLA-DRB3*0202/DRA, DG1₂₀₆₋₂₂₂-HLA-DRB3*0202/DRA, DG1₃₆₃₋₃₇₇-HLA-DRB3*0202/DRA, DG1₃₋₁₇-HLA-DRB4*0101/DRA, DG1₁₉₂₋₂₀₆-HLA-DRB4*0101/DRA, DG1₃₂₆₋₃₄₀-HLA-DRB4*0101/DRA, DG1₁₋₁₅-HLA-DRB5*0101/DRA, DG1₃₅₋₄₉-HLA-DRB5*0101/DRA, or DG1₃₂₅₋₃₃₉-HLA-DRB5*0101/DRA;
f) neuromyelitis optica spectrum disorder and the pMHC complex is selected from the group of: AQP4₁₂₉₋₁₄₃-HLA-DRB1*0101/DRA, AQP4₂₈₄₋₂₉₈-HLA-DRB1*0301/DRA, AQP4₆₃₋₇₆-HLA-DRB1*0301/DRA, AQP4₁₂₉₋₁₄₃-HLA-DRB1*0401/DRA, or AQP4₃₉₋₅₃-HLA-DRB1*1501/DRA;
g) allergic asthma and the pMHC complex is selected from the group of: DERP-1₁₆₋₃₀-HLA-DRB1*0101/DRA, DERP-1₁₆₋₃₀ -HLA-DRB1*1501/DRA, DERP₁₁₇₁₋₁₈₅ - HLA-DRB1*1501/DRA, DERP-1₁₁₀₋₁₂₄-HLA-DPB1*0401/DRA, DERP-2₂₆₋₄₀ -HLA-DRB1*0101/DRA; DERP-2₂₆₋₄₀-HLA-DRB1*1501/DRA, or DERP-2₁₀₇₋₁₂₁-HLA-DRB1*0301/DRA;
h) inflammatory bowel disease and the pMHC complex is selected from the group of: bacteroides integrase antigen₁₈₃₋₁₉₇- HLA-DRB3*0101/DRA, bacteroides integrase antigen₁₄₆₋₁₆₀- HLA-DRB3*0101/DRA, bacteroides integrase antigen₁₇₅₋₁₈₉- HLA-DRB3 *0101/DRA, bacteroides integrase antigeni₋₁₅- HLA-DRB5*0101/DRA, bacteroides integrase antigen₁₈₃₋₁₉₇-HLA-DRB5*0101/DRA, bacteroides integrase antigen₁₈₃₋₁₉₇-HLA-DRB3*0101/DRA, bacteroides integrase antigen₃₀₋₄₄- HLA-DRB5*0101/DRA, bacteroides integrase antigen₇₀₋₈₄-HLA-DRB4*0101/DRA, bacteroides integrase antigen₃₃₇₋₃₅₁- HLA-DRB4*0101/DRA, bacteroides integrase antigen₁₇₁₋₁₈₅- HLA-DRB4*0101/DRA, bacteroides integrase antigen₄₋₁₈-HLA-DRB3*0202/DRA, bacteroides integrase antigen₁₇₁₋₁₈₅₅-HLA-DRB3*0202/DRA, bacteroides integrase antigen₂₅₆₋₂₇₀-HLA-DRB3 *0202/DRA, Fla-2/Fla-X₃₆₆₋₃₈₀- HLA-DRB3*0101/DRA, Fla-2/Fla-X₁₆₄₋₁₇₈- HLA-DRB3 *0101/DRA, Fla-2/Fla-X₂₆₁₋₂₇₅- HLA-DRB5*0101/DRA, Fla-2/Fla-X₁₋₁₅- HLA-DRB5*0101/DRA, Fla-2/Fla-X₅₁₋₆₅- HLA-DRB4*0101/DRA, Fla-2/Fla-X₂₆₉₋₂₈₃- HLA-DRB4*0101/DRA, Fla-2/Fla-X₄₋₁₈-HLA-DRB3*0202/DRA, Fla-2/Fla-X₂₆₁₋₂₇₅-HLA-DRB3*0202/DRA, Fla-2/Fla-X₂₇₁₋₂₈₅-HLA-DRB3*0202/DRA, YIDX₇₈₋₉₂- HLA-DRB3*0101/DRA, YIDX₇₈₋₉₂-HLA-DRB4*0101/DRA, YIDX₉₃₋₁₀₇- HLA-DRB3*0101/DRA, YIDX₉₈₋₁₁₂- HLA-DRB5*0101/DRA, YIDX₂₃₋₃₇- HLA-DRB5*0101/DRA, YIDX₇₈₋₉₂- HLA-DRB4*0101/DRA, YIDX₁₉₅₋₂₀₉- HLA-DRB4*0101/DRA, YIDX₂₂₋₃₆-HLA-DRB3*0202/DRA, YIDX₈₀₋₉₄-HLA-DRB3*0202/DRA, or YIDX₁₀₁₋₁₁₅-HLA-DRB3*0202/DRA;
i) COPD and/or emphysema and the pMHC complex is selected from the group of: elastin₈₉₋₁₀₃-HLA-DRB3*0101/DRA, elastin₆₉₈₋₇₁₂-HLA-DRB5*0101/DRA, elastin₈₋₂₂-HLA-DRB5*0101/DRA, elastin₉₄₋₁₀₈-HLA-DRB5*0101/DRA, elastin₁₃₋₂₇-HLA-DRB4*0101/DRA, elastin₆₉₅₋₇₀₉-HLA-DRB4*0101/DRA, elastin₅₆₃₋₅₇₇-HLA-DRB4*0101/DRA, elastin₅₅₈₋₅₇₂-HLA-DRB4*0101/DRA, elastin₆₉₈₋₇₁₂-HLA-DRB5*0101/DRA, elastin₅₆₆₋₅₈₀-HLA-DRB3*0202/DRA, or elastin₆₄₅₋₆₅₉-HLA-DRB3*0202/DRA;
j) psoriasis and the pMHC complex is selected from the group of: Cap18₆₄₋₇₈-HLA-DRB3*0101/DRA, Cap18₃₄₋₄₈-HLA-DRB3*0101/DRA, Cap18₄₇₋₆₁-HLA-DRB3*0101/DRA, Cap18₁₅₁₋₁₆₅-HLA -DRB4*0101/DRA, Cap18₁₄₉₋₁₆₃-HLA-DRB5*0101/DRA, Cap18₁₅₂₋₁₆₆-HLA-DRB5*0101/DRA, Cap18₁₃₁₋₁₄₅-HLA-DRB5*0101/DRA, Cap₁₈₂₄₋₃₈-BLA-DRB3*0202/DRA, ADMTSL5₂₄₅₋₂₅₉-HLA-DRB3*0101/DRA, ADMTSL5₂₆₇₋₂₈₁-HLA-DRB3*0101/DRA, ADMTSL5₃₇₂₋₃₈₆-HLA-DRB3*0101/DRA, ADMTSL5₂₈₉₋₃₀₃-HLA-DRB4*0101/DRA, ADMTSL5₃₉₆₋₄₁₀-HLA-DRB4*0101/DRA, ADMTSL5₄₃₃₋₄₄₇-HLA-DRB4*0101/DRA, ADMTSL5₁₄₂₋₁₅₆-HLA-DRB5*0101/DRA, ADMTSL5₂₃₆₋₂₅₀-HLA-DRB5*0101/DRA, ADMTSL5₃₀₁₋₃₁₅-HLA-DRB5*0101/DRA, ADMTSL5₂₀₃₋₂₁₇-HLA-DRB3*0202/DRA, ADMTSL5₄₀₄₋₄₁₈-HLA-DRB3*0202/DRA, or ADMTSL5₄₃₃₋₄₄₇-HLA-DRB3*0202/DRA;
k) autoimmune hepatitis and the pMHC complex is selected from the group of: CYP2D6₁₉₃₋₂₀₇-HLA-DRB1*0301/DRA, CYP2D6₇₆₋₉₀-HLA-DRB1*0301/DRA, CYP2D6₂₉₃₋₃₀₇-HLA-DRB1*0301/DRA, CYP2D6₃₁₃₋₃₃₂-HLA-DRB1*0301/DRA, CYP2D6₃₉₃₋₄₁₂-HLA-DRB1*0301/DRA, CYP2D6₁₉₉₋₂₁₃-HLA-DRB1*0401/DRA, CYP2D6₄₅₀₋₄₆₄-HLA-DRB1*0401/DRA, CYP2D6₃₀₁₋₃₁₅-HLA-DRB1*0401/DRA, CYP2D6₄₅₂₋₄₆₆-HLA-DRB1*0701/DRA, CYP2D6₅₉₋₇₃-HLA-DRB1*0701/DRA, CYP2D6₁₃₀₋₁₄₄-HLA-DRB1*0701/DRA, CYP2D6₁₉₃₋₂₁₂-HLA-DRB1*0701/DRA, CYP2D6₃₀₅₋₃₂₄-HLA-DRB1*0701/DRA, CYP2D6₁₃₁₋₁₄₅-HLA-DRB3*0202/DRA, CYP2D6₂₁₆₋₂₃₀-HLA-DRB3*0202/DRA, CYP2D6₂₃₈₋₂₅₂-HLA-DRB3*0202/DRA, CYP2D6₁₉₉₋₂₁₃-HLA-DRB4*0101/DRA, CYP2D6₂₃₅₋₂₅₂-HLA-DRB4*0101/DRA, CYP2D6₂₉₃₋₃₀₇-HLA-DRB4*0101/DRA, CYP2D6₂₃₈₋₂₅₂-HLA-DRB5*0101/DRA, CYP2D6₃₈₁₋₃₉₅-HLA-DRB5*0101/DRA, CYP2D6₄₂₉₋₄₄₃-HLA-DRB5*0101/DRA, SLA₃₃₄₋₃₄₈-HLA-DRB1*0301/DRA, SLA₁₉₆₋₂₁₀-HLA-DRB1*0301/DRA, SLA₁₁₅₋₁₂₉-HLA-DRB1*0301/DRA, SLA₃₇₃₋₃₈₆-HLA-DRB1*0301/DRA, SLA₁₈₆₋₁₉₇-HLA-DRB1*0301/DRA, SLA₃₁₇₋₃₃₁-HLA-DRB1*0401/DRA, SLA₁₇₁₋₁₈₅-HLA-DRB1*0401/DRA, SLA₄₁₇₋₄₃₁-HLA-DRB1*0401/DRA, SLA₃₅₉₋₃₇₃-HLA-DRB1*0701/DRA, SLA₂₁₅₋₂₂₉-HLA-DRB1*0701/DRA, SLA₁₁₁₋₁₂₅-HLA-DRB1*0701/DRA, SLA₁₁₀₋₁₂₄-HLA-DRB3*0202/DRA, SLA₂₉₉₋₃₁₃-HLA-DRB3*0202/DRA, SLA₃₄₂₋₃₅₆-HLA-DRB3*0202/DRA, SLA₄₉₋₆₃-HLA-DRB4*0101/DRA, SLA₁₁₉₋₁₃₃-HLA-DRB4*0101/DRA, SLA₂₆₀₋₂₇₄-HLA-DRB4*0101/DRA, SLA₂₆₋₄₀-HLA-DRB5*0101/DRA, SLA₈₆₋₁₀₀-HLA-DRB5*0101/DRA, or SLA₃₃₁₋₃₄₅-HLA-DRB5*0101/DRA;
l) uveitis and the pMHC complex is selected from the group of: arrestin₁₉₉₋₂₁₃-HLA-DRB3*0101/DRA, arrestin₇₇₋₉₁-HLA-DRB3*0101/DRA, arrestin₂₅₀₋₂₆₄-HLA-DRB3*0101/DRA, arrestin₁₇₂₋₁₈₆-HLA-DRB4*0101/DRA, arrestin₃₅₄₋₃₆₈-HLA-DRB4*0101/DRA, arrestin₂₃₉₋₂₅₃-HLA-DRB4*0101/DRA, arrestin₁₀₂₋₁₁₆-HLA-DRB5*0101/DRA, arrestin₅₉₋₇₃-HLA-DRB5*0101, arrestin₂₈₀₋₂₉₄-HLA-DRB5*0101, arrestin₂₉₁₋₃₀₆-HLA-DRB1*0301/DRA, arrestin₁₉₅₋₂₀₉-HLA-DRB3*0202/DRA, arrestin₁₉₉₋₂₁₃-HLA-DRB3*0202/DRA, or arrestin₂₀₀₋₂₁₄-HLA-DRB3*0202/DRA;
m) Jorgen Syndrome and the pMHC complex is selected from the group of: R060₁₂₇₋₁₄₁-HLA-DRB1*0301/DRA, RO60₅₂₃₋₅₃₇-HLA-DRB1*0301/DRA, RO60₂₄₃₋₂₅₇-HLA-DRB1*0301/DRA, RO60₄₈₄₋₄₉₈-HLA-DRB3*0101/DRA, RO60₃₄₇₋₃₆₁-HLA-DRB3*0101/DRA, RO60₃₆₉₋₃₈₃-HLA-DRB3*0101/DRA, RO60₄₂₆₋₄₄₀-HLA-DRB4*0101/DRA, RO60₂₆₇₋₂₈₁-HLA-DRB4*0101/DRA, RO60₁₇₈₋₁₉₂-HLA-DRB4*0101/DRA, RO60₃₅₈₋₃₇₂-HLA-DRB5*0101/DRA, RO60₃₅₈₋₃₇₂-HLA-DRB4*0101/DRA, RO60₂₂₁₋₂₃₅-HLA-DRB5*0101/DRA, RO60₂₂₁₋₂₃₅-HLA-DRB4*0101/DRA, RO60₃₁₈₋₃₃₂-HLA-DRB5*0101/DRA, RO60₃₁₈₋₃₃₂-HLA-DRB4*0101/DRA, RO60₄₀₇₋₄₂₁-HLA-DRB4*0101/DRA, RO60₄₀₇₋₄₂₁-HLA-DQA1*0501/HLA-DQB1*0201, RO60₄₅₉₋₄₇₃-HLA-DRB4*0101/DRA, RO60₄₅₉₋₄₇₃-HLA-DQA1*0501/HLA-DQB1*0201, RO60₃₁₈₋₃₃₂-HLA-DQA1*0501/HLA-DQB1*0201, RO60₅₁₋₆₅-HLA-DRB3*0202/DRA, RO60₃₁₂₋₃₂₆-HLA-DRB3*0202/DRA, RO60₃₄₇₋₃₆₁-HLA-DRB3*0202/DRA, LA₂₄₁₋₂₅₅-HLA-DRB1*0301/DRA, LA₁₀₁₋₁₁₅-HLA-DRB1*0301/DRA, LA₁₅₃₋₁₆₇-HLA-DRB1*0301/DRA, LA₁₇₈₋₁₉₂-HLA-DRB3*0101/DRA, LA₁₉₋₃₃-HLA-DRB3*0101/DRA, LA₃₇₋₅₁-HLA-DRB3*0101/DRA, LA₁₃₃₋₁₄₇-HLA-DRB4*0101/DRA, LA₅₀₋₆₄-HLA-DRB4*0101/DRA, LA₃₂₋₄₆-HLA-DRB4*0101/DRA, LA₁₅₃₋₁₆₇-HLA-DRB5*0101/DRA, LA₈₃₋₉₇-HLA-DRB5*0101/DRA, LA₁₃₆₋₁₅₀-HLA-DRB5*0101/DRA, LA₂₉₇₋₃₁₁-HLA-DQA1*0501/HLA-DQB1*0201, LA₅₉₋₇₃-HLA-DQA1*0501/HLA-DQB1*0201, LA₅₉₋₇₃-HLA-DRB4*0101/DRA, LA₁₅₁₋₁₆₅-HLA-DQA1*0501/HLA-DQB1*0201, LA₁₅₁₋₁₆₅-HLA-DRB4*0101/DRA, LA₂₉₇₋₃₁₁-HLA-DRB4*0101/DRA, LA₅₀₋₆₄-HLA-DRB3*0202/DRA, LA₈₆₋₁₀₀-HLA-DRB3*0202/DRA, or LA₁₅₄₋₁₆₈-HLA-DRB3 *0202/DRA;
n) scleroderma and the pMHC complex is selected from the group of: TOP1₃₄₆₋₃₆₀-HLA-DRB3*0101/DRA, TOP1₄₂₀₋₄₃₄-HLA-DRB3*0101/DRA, TOP1₇₅₀₋₇₆₄-HLA-DRB3*0101/DRA, TOP1₄₁₉₋₄₃₃-HLA-DRB4*0101/DRA, TOP1₅₉₁₋₆₀₅-HLA-DRB4*0101/DRA, TOP1₆₉₅₋₇₀₉-HLA-DRB4*0101/DRA, TOP1₃₀₅₋₃₁₉-HLA-DRB5*010/DRA, TOP1₃₄₆₋₃₆₀-HLA-DRB5*0101/DRA, TOP1₄₁₉₋₄₃₃-HLA-DRB5*0101/DRA, TOP1₄₂₀₋₄₃₄-HLA-DRB3*0202/DRA, TOP1₄₂₅₋₄₃₉-HLA-DRB3*0202/DRA, TOP1₆₁₄₋₆₂₈-HLA-DRB3*0202/DRA, CENP-C₂₉₇₋₃₁₁-HLA-DRB3*0101/DRA, CENP-C₈₅₇₋₈₇₁-HLA-DRB3*0101/DRA, CENP-C₈₈₇₋₉₀₁-HLA-DRB3*0101/DRA, CENP-C₂₁₂₋₂₂₆-HLA-DRB4*0101/DRA, CENP-C₆₄₃₋₆₅₇-HLA-DRB4*0101/DRA, CENP-C₈₃₂₋₈₄₆-HLA-DRB4*0101/DRA, CENP-C₁₆₇₋₁₈₁-HLA-DRB5*0101/DRA, CENP-C₂₄₆₋₂₆₀-HLA-DRB5*0101/DRA, CENP-C₈₄₆₋₈₆₀-HLA-DRB5*0101/DRA, CENP-C₁₄₉₋₁₆₃-HLA-DRB3*0202/DRA, CENP-C₈₃₃₋₈₄₇-HLA-DRB3*0202/DRA, or CENP-C₈₄₇₋₈₆₁-HLA-DRB3*0202/DRA;
o) anti-phospholipid syndrome and the pMHC complex is selected from the group of: APOH₂₃₅₋₂₄₀-HLA-DRB3*0101/DRA, APOH₃₀₆₋₃₂₀-HLA-DRB3*0101/DRA, APOH₂₃₇₋₂₅₁-HLA-DRB3*0101/DRA, APOH₂₉₅₋₃₀₉-HLA-DRB3*0101/DRA, APOH₂₈₋₄₂-HLA-DRB4*0101/DRA, APOH₁₇₃₋₁₈₇-HLA-DRB4*0101/DRA, APOH₂₆₄₋₂₇₈-HLA-DRB4*0101/DRA, APOH₂₉₅₋₃₀₉-HLA-DRB4*0101/DRA, APOH₄₉₋₆₃-HLA-DRB5*0101/DRA, APOH₂₆₉₋₂₈₃-HLA-DRB5*0101/DRA, APOH₂₉₅₋₃₀₉-HLA-DRB5*0101/DRA, APOH₃₂₁₋₃₅₅-HLA-DRB3*0202/DRA, APOH₃₂₂₋₃₃₆-HLA-DRB3*0202/DRA, or APOH₃₂₄₋₃₃₈-HLA-DRB3*0202/DRA;
p) ANCA-associated vasculitis and the pMHC complex is selected from the group of: MP0₅₀₆₋₅₂₀-HLA-DRB3*0101/DRA, MPO₃₀₂₋₃₁₆-HLA-DRB3*0101/DRA, MPO₇₋₂₁-HLA-DRB3*0101/DRA, MPO₆₈₉₋₇₀₃-HLA-DRB4*0101/DRA, MPO₂₄₈₋₂₆₂-HLA-DRB4*0101/DRA, MPO₄₄₄₋₄₅₈-HLA-DRB4*0101/DRA, MPO₅₁₃₋₅₂₇-HLA-DRB5*0101/DRA, MPO₉₇₋₁₁₁-HLA-DRB5*0101/DRA, MPO₆₁₆₋₆₃₀-HLA-DRB5*0101/DRA, MPO₄₆₂₋₄₇₆-HLA-DRB3*0202/DRA, MPO₆₁₇₋₆₃₁-HLA-DRB3*0202/DRA, MPO₇₁₄₋₇₂₈-HLA-DRB3*0202/DRA, PRTN3₄₄₋₅₈-HLA-DRB3*0101/DRA, PRTN3₂₃₄₋₂₄₈-HLA-DRB3*0101/DRA, PRTN3₅₉₋₇₃-HLA DRB3*0101/DRA, PRTN3₅₉₋₇₃-HLA-DRB5*0101/DRA, PRTN3₁₁₇₋₁₃₁-HLA-DRB4*0101/DRA, PRTN3₁₆₄₋₁₇₈-HLA-DRB4*0101/DRA, PRTN3₇₁₋₈₅-HLA-DRB4*0101/DRA, PRTN3₂₄₁₋₂₅₅-HLA-DRB5*0101/DRA, PRTN3₁₈₃₋₁₉₇-HLA-DRB5*0101/DRA, PRTN3₆₂₋₇₆-HLA-DRB3*0202/DRA, PRTN3₁₁₈₋₁₃₂-HLA-DRB3*0202/DRA, or PRTN3₂₃₉₋₂₅₃-HLA-DRB3*0202/DRA; or
q) Stiff Man Syndrome and the pMHC complex is selected from the group of: GAD₂₁₂₋₂₂₆-HLA-DRB1*0801/DRA, GAD₅₅₅₋₅₆₉-HLA-DRB1*0801/DRA, or GAD₂₉₇₋₃₁₁-HLA-DRB1*0301/DRA.

In some aspects, the pMHC complex is for the treatment of:
a) type I diabetes and the pMHC complex is selected from the group of: PPI_{76-90(K88S)}-HLA-DRB1*0401/DRA, IGRP₁₃₋₂₅-HLA-DRB1*0301/DRA, GAD₅₅₅₋₅₆₇-HLA-DRB1*0401/DRA, GAD_{555-567(557I)}-HLA-DRB1*0401/DRA, IGRP₂₃₋₃₅-HLA-DRB1*0401/DRA, or PPI₇₆₋₉₀-HLA-DRB1*0401/DRA;
b) multiple sclerosis and the pMHC complex is selected from the group of: MBP₈₆₋₉₈-HLA-DRB1*1501/DRA, MBP₈₉₋₁₀₁-HLA-DRB5*0101/DRA, MOG₃₈₋₅₂-HLA-DRB4*0101/DRA, MOG_{97-109(E107S)}-HLA-DRB1*0401/DRA, MOG₂₀₃₋₂₁₇-HLA-DRB3*0101/DRA, PLP₅₄₋₆₈-BLA-DRB3*0101/DRA, PLP₉₄₋₁₀₈-HLA-DRB1*0301/DRA, PLP₂₅₀₋₂₆₄-HLA-DRB4*0101/DRA, MPB₁₃₋₃₂-HLA-DRB5*0101/DRA, MPB₈₃₋₉₉-HLA-DRB5*0101/DRA, MPB₁₁₁₋₁₂₉-HLA-DRB5*0101/DRA, MPB₁₄₆₋₁₇₀-HLA-DRB5*0101/DRA, MOG₂₂₃₋₂₃₇-HLA-DRB3*0202/DRA, MOG₆₋₂₀-HLA-DRB5*0101/DRA, PLP₈₈₋₁₀₂-HLA-DRB3*0202/DRA, or PLP₁₃₉₋₁₅₄-HLA-DRB5*0101/DRA;
c) Celiac Disease and the pMHC complex is selected from the group of: aGlia₅₇₋₆₈- HLA-DQA1*0501/HLA-DQB1*0201, aGlia₆₂₋₇₂- HLA-DQA1*0501/HLA-DQB1*0201, or aGlia₂₁₇₋₂₂₉- HLA-DQA1*0501/HLA-DQB1 *0302;
d) primary biliary cirrhosis and the pMHC complex is selected from the group of: PDC-E2₁₂₂₋₁₃₅-HLA-DRB4*0101/DRA, PDC-E2₂₄₉₋₂₆₂-HLA-DRB4*0101/DRA, PDC-E2₂₄₉₋₂₆₃-HLA-DRB1*0801/DRA, PDC-E2₆₂₉₋₆₄₃-HLA-DRB1*0801/DRA, PDC-E2₇₂₋₈₆-HLA-DRB3*0202/DRA, PDC-E2₃₅₃₋₃₆₇-HLA-DRB3*0202/DRA, PDC-E2₄₂₂₋₄₃₆-HLA-DRB3*0202/DRA, PDC-E2₆₂₉₋₆₄₃-HLA-DRB4*0101/DRA, PDC-E2₈₀₋₉₄-HLA-DRB5*0101/DRA, PDC-E2₃₅₃₋₃₆₇-HLA-DRB5*0101/DRA, or PDC-E2₅₃₅₋₅₄₉-HLA-DRB5*0101/DRA;
e) pemphigus folliaceus and/or pemphigus vulgaris and the pMHC complex is selected from the group of: DG1₂₁₆₋₂₂₉-HLA-DRB1*0101/DRA, DG3₉₇₋₁₁₁-HLA-DRB1*0402/DRA, DG3₂₅₁₋₂₆₅-HLA-DRB1*0401/DRA, DG3₄₄₁₋₄₅₅-HLA-DRB1*0402/DRA, DG3₃₅₁₋₃₆₅-HLA-DRB3*0202/DRA, DG3₄₅₃₋₄₆₇-HLA-DRB3*0202/DRA, DG3₅₄₀₋₅₅₄-HLA-DRB3*0202/DRA, DG3₂₈₀₋₂₉₄-HLA-DRB4*0101/DRA, DG3₃₂₆₋₃₄₀-HLA-DRB4*0101/DRA, DG3₃₆₇₋₃₈₁-HLA-DRB4*0101/DRA, DG3₁₃₋₂₇-HLA-DRB5*0101/DRA, DG3₃₂₃₋₃₃₇-HLA-DRB5*0101/DRA, DG3₄₃₈₋₄₅₂-HLA-DRB5*0101/DRA, DG1₄₈₋₆₂-HLA-DRB3*0202/DRA, DG1₂₀₆₋₂₂₂-HLA-DRB3*0202/DRA, DG1₃₆₃₋₃₇₇-HLA-DRB3*0202/DRA, DG1₃₋₁₇-HLA-DRB4*0101/DRA, DG1₁₉₂₋₂₀₆-HLA-DRB4*0101/DRA, DG1₃₂₆₋₃₄₀-HLA-DRB4*0101/DRA, DG1₁₋₁₅-HLA-DRB5*0101/DRA, DG1₃₅₋₄₉-HLA-DRB5*0101/DRA, or DG1₃₂₅₋₃₃₉-HLA-DRB5*0101/DRA;
f) neuromyelitis optica spectrum disorder and the pMHC complex is selected from the group of: AQP4₂₈₄₋₂₉₈-HLA-DRB1*0301/DRA, AQP4₆₃₋₇₆-HLA-DRB1*0301/DRA, AQP4₁₂₉₋₁₄₃-HLA-DRB1*0401/DRA, or AQP4₃₉₋₅₃-HLA-DRB1*1501/DRA;
g) allergic asthma and the pMHC complex is selected from the group of: DERP-1₁₆₋₃₀-HLA-DRB1*0101/DRA, DERP-1₁₆₋₃₀ -HLA-DRB1*1501/DRA, DERP₁₁₇₁₋₁₈₅ - HLA-DRB1*1501/DRA, DERP-1₁₁₀₋₁₂₄-HLA-DPB1*0401/DRA, DERP-2₂₆₋₄₀ -HLA-DRB1*0101/DRA; DERP-2₂₆₋₄₀-HLA-DRB1*1501/DRA, or DERP-2₁₀₇₋₁₂₁-HLA-DRB1*0301/DRA;
h) inflammatory bowel disease and the pMHC complex is selected from the group of: bacteroides integrase antigen₁₋ₗ₅ - HLA-DRB5*0101/DRA, bacteroides integrase antigen₁₈₃₋₁₉₇-HLA-DRB3*0101/DRA, bacteroides integrase antigen₇₀₋₈₄- HLA-DRB4*0101/DRA, bacteroides integrase antigen₄₋₁₈-HLA-DRB3*0202/DRA, bacteroides integrase antigen₁₇₁₋₁₈₅-HLA-DRB3 *0202/DRA, bacteroides integrase antigen₂₅₆₋₂₇₀-HLA-DRB3 *0202/DRA, Fla-2/FlaX₃₆₆₋₃₈₀- HLA-DRB3*0101/DRA, Fla-2/Fla-X₂₆₁₋₂₇₅- HLA-DRB5*0101/DRA, Fla-2/Fla-X₅₁₋₆₅-HLA-DRB4*0101/DRA, Fla-2/Fla-X₄₋₁₈-HLA-DRB3*0202/DRA, Fla-2/Fla-X₂₆₁₋₂₇₅-HLA-DRB3*0202/DRA, Fla-2/Fla-X₂₇₁₋₂₈₅-HLA-DRB3*0202/DRA, YIDX₇₈₋₉₂-HLA-DRB3*0101/DRA, YIDX₇₈₋₉₂-HLA-DRB4*0101/DRA, YIDX₉₈₋₁₁₂-HLA-DRB5*0101/DRA, YIDX₂₂₋₃₆-HLA-DRB3*0202/DRA, YIDX₈₀₋₉₄-HLA-DRB3*0202/DRA, or YIDX₁₀₁₋₁₁₅-HLA-DRB3*0202/DRA;
i) emphysema and the pMHC complex is selected from the group of: elastin₈₉₋₁₀₃-HLA-DRB3*0101/DRA, elastin₆₉₈₋₇₁₂-HLA-DRB5*0101/DRA, elastin₅₅₈₋₅₇₂-HLA-DRB4*0101/DRA, elastin₅₆₆₋₅₈₀-HLA-DRB3*0202/DRA, or elastin₆₄₅₋₆₅₉-HLA-DRB3*0202/DRA;
j) psoriasis and the pMHC complex is selected from the group of: Cap18₆₄₋₇₈-HLA-DRB3*0101/DRA, Cap18₃₄₋₄₈-HLA-DRB3*0101/DRA, Cap18₄₇₋₆₁-HLA-DRB3*0101/DRA, Cap18₁₅₁₋₁₆₅-HLA -DRB4*0101/DRA, Cap18₁₄₉₋₁₆₃-HLA-DRB5*0101/DRA, Cap18₁₅₂₋₁₆₆-HLA-DRB5*0101/DRA, Cap18₁₃₁₋₁₄₅-HLA-DRB5*0101/DRA, Cap₁₈₂₄₋₃₈-HLA-DRB3*0202/DRA, ADMTSL5₂₄₅₋₂₅₉-HLA-DRB3*0101/DRA, ADMTSL5₂₆₇₋₂₈₁-HLA-DRB3*0101/DRA, ADMTSL5₃₇₂₋₃₈₆-HLA-DRB3*0101/DRA, ADMTSL5₂₈₉₋₃₀₃-HLA-DRB4*0101/DRA, ADMTSL5₃₉₆₋₄₁₀-HLA-DRB4*0101/DRA, ADMTSL5₄₃₃₋₄₄₇-HLA-DRB4*0101/DRA, ADMTSL5₁₄₂₋₁₅₆-HLA-DRB5*0101/DRA, ADMTSL5₂₃₆.₂₅₀-HLA-DRB5*0101/DRA, ADMTSL5₃₀₁₋₃₁₅-HLA-DRB5*0101/DRA, ADMTSL5₂₀₃₋₂₁₇-HLA-DRB3*0202/DRA, ADMTSL5₄₀₄₋₄₁₈-HLA-DRB3*0202/DRA, or ADMTSL5₄₃₃₋₄₄₇-HLA-DRB3*0202/DRA;
k) autoimmune hepatitis and the pMHC complex is selected from the group of: CYP2D6₁₉₃₋₂₀₇-HLA-DRB1*0301/DRA, CYP2D6₇₆₋₉₀-HLA-DRB1*0301/DRA, CYP2D6₂₉₃₋₃₀₇-HLA-DRB1*0301/DRA, CYP2D6₃₁₃₋₃₃₂-HLA-DRB1*0301/DRA, CYP2D6₃₉₃₋₄₁₂-HLA-DRB1*0301/DRA, CYP2D6₁₉₉₋₂₁₃-HLA-DRB1*0401/DRA, CYP2D6₄₅₀₋₄₆₄-HLA-DRB1*0401/DRA, CYP2D6₃₀₁₋₃₁₅-HLA-DRB1*0401/DRA, CYP2D6₄₅₂₋₄₆₆-HLA-DRB1*0701/DRA, CYP2D6₅₉₋₇₃-HLA-DRB1*0701/DRA, CYP2D6₁₃₀₋₁₄₄-HLA-DRB1*0701/DRA, CYP2D6₁₉₃₋₂₁₂-HLA-DRB1*0701/DRA, CYP2D6₃₀₅₋₃₂₄-HLA-DRB1*0701/DRA, CYP2D6₁₃₁₋₁₄₅-HLA-DRB3*0202/DRA, CYP2D6₂₁₆₋₂₃₀-HLA-DRB3*0202/DRA, CYP2D6₂₃₈₋₂₅₂-HLA-DRB3*0202/DRA, CYP2D6₁₉₉₋₂₁₃-HLA-DRB4*0101/DRA, CYP2D6₂₃₅₋₂₅₂-HLA-DRB4*0101/DRA, CYP2D6₂₉₃₋₃₀₇-HLA-DRB4*0101/DRA, CYP2D6₂₃₈₋₂₅₂-HLA-DRB5*0101/DRA, CYP2D6₃₈₁₋₃₉₅-HLA-DRB5*0101/DRA, CYP2D6₄₂₉₋₄₄₃-HLA-DRB5*0101/DRA, SLA₃₃₄₋₃₄₈-HLA-DRB1*0301/DRA, SLA₁₉₆₋₂₁₀-HLA-DRB1*0301/DRA, SLA₁₁₅₋₁₂₉-HLA-DRB1*0301/DRA, SLA₃₇₃₋₃₈₆-HLA-DRB1*0301/DRA, SLA₁₈₆₋₁₉₇-HLA-DRB1*0301/DRA, SLA₃₁₇₋₃₃₁-HLA-DRB1*0401/DRA, SLA ₁₇₁₋₁₈₅-HLA-DRB1*0401/DRA, SLA₄₁₇₋₄₃₁-HLA-DRB1*0401/DRA, SLA₃₅₉₋₃₇₃-HLA-DRB1*0701/DRA, SLA₂₁₅₋₂₂₉-HLA-DRB1*0701/DRA, SLA₁₁₁₋₁₂₅-HLA-DRB1*0701/DRA, SLA₁₁₀₋₁₂₄-HLA-DRB3*0202/DRA, SLA₂₉₉₋₃₁₃-HLA-DRB3*0202/DRA, SLA₃₄₂₋₃₅₆-HLA-DRB3*0202/DRA, SLA₄₉₋₆₃-HLA-DRB4*0101/DRA, SLA₁₁₉₋₁₃₃-HLA-DRB4*0101/DRA, SLA₂₆₀₋₂₇₄-HLA-DRB4*0101/DRA, SLA₂₆₋₄₀-HLA-DRB5*0101/DRA, SLA₈₆₋₁₀₀-HLA-DRB5*0101/DRA, or SLA₃₃₁₋₃₄₅-HLA-DRB5*0101/DRA;
l) uveitis and the pMHC complex is selected from the group of: arrestin₁₉₉₋₂₁₃-HLA-DRB3*0101/DRA, arrestin₇₇₋₉₁-HLA-DRB3*0101/DRA, arrestin₂₅₀₋₂₆₄-HLA-DRB3*0101/DRA, arrestin₁₇₂₋₁₈₆-HLA-DRB4*0101/DRA, arrestin₃₅₄₋₃₆₈-HLA-DRB4*0101/DRA, arrestin₂₃₉₋₂₅₃-HLA-DRB4*0101/DRA, arrestin₁₀₂₋₁₁₆-HLA-DRB5*0101/DRA, arrestin₅₉₋₇₃-HLA-DRB5*0101, arrestin₂₈₀₋₂₉₄-HLA-DRB5*0101, arrestin₂₉₁₋₃₀₆-HLA-DRB1*0301/DRA, arrestin₁₉₅₋₂₀₉-HLA-DRB3*0202/DRA, arrestin₁₉₉₋₂₁₃-HLA-DRB3*0202/DRA, or arrestin₂₀₀₋₂₁₄-HLA-DRB3*0202/DRA;
m) Jorgen Syndrome and the pMHC complex is selected from the group of: RO60₁₂₇₋₁₄₁-HLA-DRB1*0301/DRA, R060₅₂₃₋₅₃₇-HLA-DRB1*0301/DRA, RO60₂₄₃₋₂₅₇-HLA-DRB1*0301/DRA, RO60₄₈₄₋₄₉₈-HLA-DRB3*0101/DRA, RO60₃₄₇₋₃₆₁-HLA-DRB3*0101/DRA, RO60₃₆₉₋₃₈₃-HLA-DRB3*0101/DRA, RO60₄₂₆₋₄₄₀-HLA-DRB4*0101/DRA, RO60₂₆₇₋₂₈₁-HLA-DRB4*0101/DRA, RO60₁₇₈₋₁₉₂-HLA-DRB4*0101/DRA, RO60₃₅₈₋₃₇₂-HLA-DRB5*0101/DRA, RO60₂₂₁₋₂₃₅-HLA-DRB5*0101/DRA, RO60₃₁₃₋₃₃₂-HLA-DRB5*0101/DRA, RO60₅₁₋₆₅-HLA-DRB3*0202/DRA, RO60₃₁₂₋₃₂₆-HLA-DRB3*0202/DRA, RO60₃₄₇₋₃₆₁-HLA-DRB3*0202/DRA, LA₂₄₁₋₂₅₅-HLA-DRB1*0301/DRA, LA₁₀₁₋₁₁₅-HLA-DRB1*0301/DRA, LA₁₅₃₋₁₆₇-HLA-DRB1*0301/DRA, LA₁₇₈₋₁₉₂-HLA-DRB3*0101/DRA, LA₁₉₋₃₃-HLA-DRB3*0101/DRA, LA₃₇₋₅₁-HLA-DRB3*0101/DRA, LA₁₃₃₋₁₄₇-HLA-DRB4*0101/DRA, LA₅₀₋₆₄-HLA-DRB4*0101/DRA, LA₃₂₋₄₆-HLA-DRB4*0101/DRA, LA₁₅₃₋₁₆₇-HLA-DRB5*0101/DRA, LA₈₃₋₉₇-HLA-DRB5*0101/DRA, LA₁₃₆₋₁₅₀-HLA-DRB5*0101/DRA, LA₅₀₋₆₄-HLA-DRB3*0202/DRA, LA₈₆₋₁₀₀-HLA-DRB3*0202/DRA, or LA₁₅₄₋₁₆₈-HLA-DRB3*0202/DRA;
n) scleroderma and the pMHC complex is selected from the group of: TOP1₃₄₆₋₃₆₀-HLA-DRB3*0101/DRA, TOP1₄₂₀₋₄₃₄-HLA-DRB3*0101/DRA, TOP1₇₅₀₋₇₆₄-HLA-DRB3*0101/DRA, TOP1₄₁₉₋₄₃₃-HLA-DRB4*0101/DRA, TOP1₅₉₁₋₆₀₅-HLA-DRB4*0101/DRA, TOP1₆₉₅₋₇₀₉-HLA-DRB4*0101/DRA, TOP1₃₀₅₋₃₁₉-HLA-DRB5*0101/DRA, TOP1₃₄₆₋₃₆₀-HLA-DRB5*0101/DRA, TOP1₄₁₉₋₄₃₃-HLA-DRB5*0101/DRA, TOP1₄₂₀₋₄₃₄-HLA-DRB3*0202/DRA, TOP1₄₂₅₋₄₃₉-HLA-DRB3*0202/DRA, TOP1₆₁₄₋₆₂₈-HLA-DRB3*0202/DRA, CENP-C₂₉₇₋₃₁₁-HLA-DRB3*0101/DRA, CENP-C₈₅₇₋₈₇₁-HLA-DRB3*0101, CENP-C₈₈₇₋₉₀₁-HLA-DRB3*0101, CENP-C₂₁₂₋₂₂₆-HLA-DRB4*0101/DRA, CENP-C₆₄₃₋₆₅₇-HLA-DRB4*0101/DRA, CENP-C_{832-g46}-HLA-DRB4*0101/DRA, CENP-C₁₆₇₋₁₈₁-HLA-DRB5*0101/DRA, CENP-C₂₄₆₋₂₆₀-HLA-DRB5*0101/DRA, CENP-C₈₄₆₋₈₆₀-HLA-DRB5*0101/DRA, CENP-C₁₄₉₋₁₆₃-HLA-DRB3*0202/DRA, CENP-C₈₃₃₋₈₄₇-HLA-DRB3*0202/DRA, or CENP-C₈₄₇₋₈₆₁-HLA-DRB3*0202/DRA;
o) anti-phospholipid syndrome and the pMHC complex is selected from the group of: APOH₂₃₅₋₂₄₉-HLA-DRB3*0101/DRA, APOH₃₀₆₋₃₂₀-HLA-DRB3*0101/DRA, APOH₂₃₇₋₂₅₁-HLA-DRB3*0101/DRA, APOH₂₉₅₋₃₀₉-HLA-DRB3*0101/DRA, APOH₂₈₋₄₂-HLA-DRB4*0101/DRA, APOH₁₇₃₋₁₈₇-HLA-DRB4*0101/DRA, APOH₂₆₄₋₂₇₈-HLA-DRB4*0101/DRA, APOH₂₉₅₋₃₀₉-HLA-DRB4*0101/DRA, APOH₄₉₋₆₃-HLA-DRB5*0101/DRA, APOH₂₆₉₋₂₈₃-HLA-DRB5*0101/DRA, APOH₂₉₅₋₃₀₉-HLA-DRB5*0101/DRA, APOH₃₂₁₋₃₅₅-HLA-DRB3*0202/DRA, APOH₃₂₂₋₃₃₆-HLA-DRB3*0202/DRA, or APOH₃₂₄₋₃₃₈-HLA-DRB3*0202/DRA;
p) ANCA-associated vasculitis and the pMHC complex is selected from the group of: MPO₅₀₆₋₅₂₀-HLA-DRB3*0101/DRA, MPO₃₀₂₋₃₁₆-HLA-DRB3*0101/DRA, MPO₇₋₂₁-HLA-DRB3*0101/DRA, MPO₆₈₉₋₇₀₃-HLA-DRB4*0101/DRA, MPO₂₄₈₋₂₆₂-HLA-DRB4*0101/DRA, MPO₄₄₄₋₄₅₈-HLA-DRB4*0101/DRA, MPO₅₁₃₋₅₂₇-HLA-DRB5*0101/DRA, MPO₉₇₋₁₁₁-HLA-DRB5*0101/DRA, MPO₆₁₆₋₆₃₀-HLA-DRB5*0101/DRA, MPO₄₆₂₋₄₇₆-HLA-DRB3*0202/DRA, MPO₆₁₇₋₆₃₁-HLA-DRB3*0202/DRA, MPO₇₁₄₋₇₂₈-HLA-DRB3*0202/DRA, PRTN3₄₄₋₅₈-HLA-DRB3*0101/DRA, PRTN3₂₃₄₋₂₄₈-HLA-DRB3*0101/DRA, PRTN3₅₉₋₇₃-HLA DRB3*0101/DRA, PRTN3₅₉₋₇₃-HLA-DRB5*0101/DRA, PRTN3₁₁₇₋₁₃₁-HLA-DRB4*0101/DRA, PRTN3₁₆₄₋₁₇₈-HLA-DRB4*0101/DRA, PRTN3₇₁₋₈₅-HLA-DRB4*0101/DRA, PRTN3₂₄₁₋₂₅₅-HLA-DRB5*0101/DRA, PRTN3₁₈₃₋₁₉₇-HLA-DRB5*0101/DRA, PRTN3₆₂₋₇₆-HLA-DRB3*0202/DRA, PRTN3₁₁₈₋₁₃₂-HLA-DRB3*0202/DRA, or PRTN3₂₃₉₋₂₅₃-HLA-DRB3*0202/DRA; or
q) Stiff Man Syndrome and the pMHC complex is selected from the group of: GAD₂₁₂₋₂₂₆-HLA-DRB1*0801/DRA, GAD₅₅₅₋₅₆₉-HLA-DRB1*0801/DRA, or GAD₂₉₇₋₃₁₁-HLA-DRB1*0301/DRA.

Selection of the co-stimulatory molecule or molecules to be coupled to the pMHC/NP complex may also be similarly optimized and will largely depend on the nature of the immune cell population in need of differentiation or expansion. For instance, if the intent is to expand or differentiate T regulatory cell populations, relevant combinations may include, but are not limited to, co-stimulatory molecules and cytokines such as IL15-IL15Ra, IL-2, IL-10, IL-35, ICOS-L, IL2/Anti-IL2 mAb complex, TGF-beta, IL-21, ITE or ICOSL. In contrast, in certain embodiments, such as with certain types of cancers, an expansion and/or differentiation of the T regulatory phenotype may not be the desired response. Thus, alternative co-stimulatory molecules and cytokines would be optimized to the particular treatment.

### Polypeptides

In another aspect, provided herein are polypeptides comprising an MHC class II α1 domain, an MHC class II α2 domain, or a combination thereof; and at least one engineered protuberance. In some embodiments, the MHC class II α1 domain and the MHC class II α2 domain are derived from a human leukocyte antigen (HLA) molecule such as HLA-DR, HLA-DQ, or HLA-DP. In certain embodiments, the MHC class II α1 domain and the MHC class II α2 domain are derived from DRA, DQA1, or DPA1. In some embodiments, the MHC class II α1 domain or the MHC class II α2 domain are derived from DRA, DQA1, or DPA1.

In another aspect, provided herein are polypeptides comprising an MHC class II β1 domain, an MHC class II β2 domain, or a combination thereof; and at least one engineered protuberance. In some embodiments, the MHC class II β1 domain and the MHC class II β2 domain are derived from a human leukocyte antigen (HLA) molecule such as HLA-DR, HLA-DQ, or HLA-DP. In certain embodiments, the MHC class II β1 domain and the MHC class II β2 are derived from HLA-DRB1, HLA-DRB3, HLA-DRB4, HLA-DRB5, HLA-DQB1, or HLA-DPB1. In some embodiments, the MHC class II β1 domain or the MHC class II β2 are derived from HLA-DRB1, HLA-DRB3, HLA-DRB4, HLA-DRB5, HLA-DQB1, or HLA-DPB1.

In another aspect, provided herein are polypeptides comprising an MHC class II α1 domain, an MHC class II α2 domain, or a combination thereof; and at least one engineered cavity.

In another aspect, provided herein are polypeptides comprising an MHC class II β1 domain, an MHC class II β2 domain, or a combination thereof; and at least one engineered cavity.

In another aspect, provided herein are polypeptides comprising an MHC class II α1 domain and an MHC class II α2 domain; and at least one engineered protuberance.

In another aspect, provided herein are polypeptides comprising an MHC class II β1 domain and an MHC class II β2 domain; and at least one engineered protuberance.

In another aspect, provided herein are polypeptides comprising an MHC class II α1 domain and an MHC class II α2 domain; and at least one engineered cavity.

In another aspect, provided herein are polypeptides comprising an MHC class II β1 domain and an MHC class II β2 domain; and at least one engineered cavity.

In some embodiments, the polypeptide further comprises a C-terminal cysteine residue. In some embodiments of the disclosure and not of the invention, the polypeptide further comprises a biotinylation site. In some embodiments of the disclosure and not of the invention, the polypeptide further comprises a Strep tag.

In some embodiments, the polypeptide provided herein is encoded by a DNA sequence comprising any one of **SEQ ID NOS: 1-26, 64, or 65.** In some embodiments, the polypeptide is encoded by a DNA sequence comprising **SEQ ID NO: 1.** In some embodiments, the polypeptide is encoded by a DNA sequence comprising **SEQ ID NO: 2.** In some embodiments, the polypeptide is encoded by a DNA sequence comprising **SEQ ID NO: 3.** In some embodiments, the polypeptide is encoded by a DNA sequence comprising **SEQ ID NO: 4.** In some embodiments, the polypeptide is encoded by a DNA sequence comprising **SEQ ID NO: 25.** In some embodiments, the polypeptide is encoded by a DNA sequence comprising **SEQ ID NO: 26.**

In some embodiments, the polypeptide provided herein is encoded by any one of **SEQ ID NOS: 1-8.** In some embodiments, the polypeptide is encoded by **SEQ ID NO: 1.** In some embodiments, the polypeptide is encoded by **SEQ ID NO: 2.** In some embodiments, the polypeptide is encoded by **SEQ ID NO: 3.** In some embodiments, the polypeptide is encoded by **SEQ ID NO: 4.** In some embodiments, the polypeptide provided herein is encoded by any one of **SEQ ID NOS: 5-6.** In some embodiments, the polypeptide provided herein is encoded by any one of **SEQ ID NOS: 5-8.**

In some embodiments, the polypeptide is encoded by a DNA sequence comprising a DNA sequence with at least 80% identity with any one of **SEQ ID NOS: 1-26, 64, or 65.** This includes at least 81, 82, 83, 84, 85, 86, 87, 88, 89, 90, 91, 92, 93, 94, 95, 96, 97, 98, or 99% identity with any one of **SEQ ID NOS: 1-8.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with any one of **SEQ ID NOS: 1-26, 64, or 65.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with **SEQ ID NO: 1.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with **SEQ ID NO: 2.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with **SEQ ID NO: 3.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with **SEQ ID NO: 4.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with any one of **SEQ ID NO: 5-6.** In some embodiments, the polypeptide is encoded by a DNA sequence comprising a DNA sequence with at least 80, 81, 82, 83, 84, 85, 86, 87, 88, 89, 90, 91, 92, 93, 94, 95, 96, 97, 98, or 99% identity with any one of **SEQ ID NOS: 5-8.**

In some embodiments, the polypeptide is encoded by a DNA sequence comprising a DNA sequence with at least 85% identity with any one of **SEQ ID NOS: 1-26, 64, or 65.** In some embodiments, the polypeptide is encoded by a DNA sequence comprising a DNA sequence with at least 85% identity with **SEQ ID NO: 1.** In some embodiments, the polypeptide is encoded by a DNA sequence comprising a DNA sequence with at least 85% identity with **SEQ ID NO: 2.** In some embodiments, the polypeptide is encoded by a DNA sequence comprising a DNA sequence with at least 85% identity with **SEQ ID NO: 3.** In some embodiments, the polypeptide is encoded by a DNA sequence comprising a DNA sequence with at least 85% identity with **SEQ ID NO: 4.** In some embodiments, the polypeptide is encoded by a DNA sequence comprising a DNA sequence with at least 85% identity with any one of **SEQ ID NOS: 5-6.** In some embodiments, the polypeptide is encoded by a DNA sequence comprising a DNA sequence with at least 85% identity with any one of **SEQ ID NOS: 5-8.**

In some embodiments, the polypeptide is encoded by a DNA sequence comprising a DNA sequence with at least 90% identity with any one of **SEQ ID NOS: 1-26, 64, or 65.** In some embodiments, the polypeptide is encoded by a DNA sequence comprising a DNA sequence with at least 90% identity with **SEQ ID NO: 1.** In some embodiments, the polypeptide is encoded by a DNA sequence comprising a DNA sequence with at least 90% identity with **SEQ ID NO: 2.** In some embodiments, the polypeptide is encoded by a DNA sequence comprising a DNA sequence with at least 90% identity with **SEQ ID NO: 3.** In some embodiments, the polypeptide is encoded by a DNA sequence comprising a DNA sequence with at least 90% identity with **SEQ ID NO: 4.** In some embodiments, the polypeptide is encoded by a DNA sequence comprising a DNA sequence with at least 90% identity with any one of **SEQ ID NOS: 5-6.** In some embodiments, the polypeptide is encoded by a DNA sequence comprising a DNA sequence with at least 90% identity with any one of **SEQ ID NOS: 5-8.**

In some embodiments, the polypeptide is encoded by a DNA sequence comprising a DNA sequence with at least 95% identity with any one of **SEQ ID NOS: 1-26, 64, or 65.** In some embodiments, the polypeptide is encoded by a DNA sequence comprising a DNA sequence with at least 95% identity with **SEQ ID NO: 1.** In some embodiments, the polypeptide is encoded by a DNA sequence comprising a DNA sequence with at least 95% identity with **SEQ ID NO: 2.** In some embodiments, the polypeptide is encoded by a DNA sequence comprising a DNA sequence with at least 95% identity with **SEQ ID NO: 3.** In some embodiments, the polypeptide is encoded by a DNA sequence comprising a DNA sequence with at least 95% identity with **SEQ ID NO: 4.** In some embodiments, the polypeptide is encoded by a DNA sequence comprising a DNA sequence with at least 95% identity with any one of **SEQ ID NOS: 5-6.** In some embodiments, the polypeptide is encoded by a DNA sequence comprising a DNA sequence with at least 95% identity with any one of **SEQ ID NOS: 5-8.**

In some embodiments, the polypeptide provided herein is a polypeptide comprising an amino acid sequence of any one of **SEQ ID NOS: 27-63,** or a fragment thereof. In some embodiments, the polypeptide comprises an amino acid sequence of **SEQ ID NO: 35-50,** or a fragment thereof. In some embodiments, the polypeptide comprises an amino acid sequence of **SEQ ID NO: 51,** or a fragment thereof. In some embodiments, the polypeptide comprises an amino acid sequence of **SEQ ID NO: 52,** or a fragment thereof. In some embodiments, the polypeptide comprises an amino acid sequence of **SEQ ID NO: 53,** or a fragment thereof. In some embodiments, the polypeptide comprises an amino acid sequence of **SEQ ID NO: 54,** or a fragment thereof. In some embodiments, the polypeptide comprises an amino acid sequence of **SEQ ID NO: 55-59,** or a fragment thereof. As used herein, a "fragment" of an amino acid sequence or a polypeptide comprises at least 5 amino acids. In some embodiments, the fragment comprises an MHC class II α1 domain, an MHC class II α2 domain, or a combination thereof. In some embodiments, the fragment comprises an MHC class II α1 domain, an MHC class II α2 domain, an engineered protuberance described herein, or any combination thereof. In some embodiments, the fragment comprises an MHC class II α1 domain, an MHC class II α2 domain, an engineered cavity described herein, or any combination thereof. In some embodiments, the fragment comprises an MHC class II β1 domain, an MHC class II β2 domain, an engineered protuberance described herein, or any combination thereof. In some embodiments, the fragment comprises an MHC class II β1 domain, an MHC class II β2 domain, an engineered cavity described herein, or any combination thereof.

In some embodiments, the fragment comprises an MHC class II α1 domain, an MHC class II α2 domain, and an engineered protuberance described herein. In some embodiments, the fragment comprises an MHC class II α1 domain, an MHC class II α2 domain, and an engineered cavity described herein. In some embodiments, the fragment comprises an MHC class II β1 domain, an MHC class II β2 domain, and an engineered protuberance described herein. In some embodiments, the fragment comprises an MHC class II β1 domain, an MHC class II β2 domain, and an engineered cavity described herein.

In some embodiments, the polypeptide provided herein comprises an amino acid sequence having at least 80% identity with an amino acid sequence of any one of **SEQ ID NOS: 27-63,** or a fragment thereof. This includes at least 81, 82, 83, 84, 85, 86, 87, 88, 89, 90, 91, 92, 93, 94, 95, 96, 97, 98, or 99% identity with any one of **SEQ ID NOS: 27-63.** In some embodiments, the polypeptide comprises an amino acid sequence having at least 80, 81, 82, 83, 84, 85, 86, 87, 88, 89, 90, 91, 92, 93, 94, 95, 96, 97, 98, or 99% identity with an amino acid sequence of any one of **SEQ ID NOS: 35-50,** or a fragment thereof. In some embodiments, the polypeptide comprises an amino acid sequence having at least 80, 81, 82, 83, 84, 85, 86, 87, 88, 89, 90, 91, 92, 93, 94, 95, 96, 97, 98, or 99% identity with an amino acid sequence of **SEQ ID NO: 51,** or a fragment thereof. In some embodiments, the polypeptide comprises an amino acid sequence having at least 80, 81, 82, 83, 84, 85, 86, 87, 88, 89, 90, 91, 92, 93, 94, 95, 96, 97, 98, or 99% identity with an amino acid sequence of **SEQ ID NO: 52,** or a fragment thereof. In some embodiments, the polypeptide comprises an amino acid sequence having at least 80, 81, 82, 83, 84, 85, 86, 87, 88, 89, 90, 91, 92, 93, 94, 95, 96, 97, 98, or 99% identity with an amino acid sequence of **SEQ ID NO: 53,** or a fragment thereof. In some embodiments, the polypeptide comprises an amino acid sequence having at least 80, 81, 82, 83, 84, 85, 86, 87, 88, 89, 90, 91, 92, 93, 94, 95, 96, 97, 98, or 99% identity with an amino acid sequence of **SEQ ID NO: 54,** or a fragment thereof. In some embodiments, the polypeptide provided herein comprises an amino acid sequence having at least 80, 81, 82, 83, 84, 85, 86, 87, 88, 89, 90, 91, 92, 93, 94, 95, 96, 97, 98, or 99% identity with an amino acid sequence of any one of **SEQ ID NOS: 55-59,** or a fragment thereof.

In some embodiments, the polypeptide provided herein comprises an amino acid sequence having at least 85% identity with an amino acid sequence of any one of **SEQ ID NOS: 27-63,** or a fragment thereof. In some embodiments, the polypeptide provided herein comprises an amino acid sequence having at least 85% identity with an amino acid sequence of any one of **SEQ ID NOS: 35-50,** or a fragment thereof. In some embodiments, the polypeptide comprises an amino acid sequence having at least 85% identity with an amino acid sequence of **SEQ ID NO: 51,** or a fragment thereof. In some embodiments, the polypeptide comprises an amino acid sequence having at least 85% identity with an amino acid sequence of **SEQ ID NO: 52,** or a fragment thereof. In some embodiments, the polypeptide comprises an amino acid sequence having at least 85% identity with an amino acid sequence of **SEQ ID NO: 53,** or a fragment thereof. In some embodiments, the polypeptide comprises an amino acid sequence having at least 85% identity with an amino acid sequence of **SEQ ID NO: 54,** or a fragment thereof. In some embodiments, the polypeptide provided herein comprises an amino acid sequence having at least 85% identity with an amino acid sequence of any one of **SEQ ID NOS: 55-59,** or a fragment thereof.

In some embodiments, the polypeptide provided herein comprises an amino acid sequence having at least 90% identity with an amino acid sequence of any one of **SEQ ID NOS: 27-63,** or a fragment thereof. In some embodiments, the polypeptide provided herein comprises an amino acid sequence having at least 90% identity with an amino acid sequence of any one of **SEQ ID NOS: 35-50,** or a fragment thereof. In some embodiments, the polypeptide comprises an amino acid sequence having at least 90% identity with an amino acid sequence of **SEQ ID NO: 51,** or a fragment thereof. In some embodiments, the polypeptide comprises an amino acid sequence having at least 90% identity with an amino acid sequence of **SEQ ID NO: 52,** or a fragment thereof. In some embodiments, the polypeptide comprises an amino acid sequence having at least 90% identity with an amino acid sequence of **SEQ ID NO: 53,** or a fragment thereof. In some embodiments, the polypeptide comprises an amino acid sequence having at least 90% identity with an amino acid sequence of **SEQ ID NO: 54,** or a fragment thereof. In some embodiments, the polypeptide provided herein comprises an amino acid sequence having at least 90% identity with an amino acid sequence of any one of **SEQ ID NOS: 55-59,** or a fragment thereof.

In some embodiments, the polypeptide provided herein comprises an amino acid sequence having at least 95% identity with an amino acid sequence of any one of **SEQ ID NOS: 27-63,** or a fragment thereof. In some embodiments, the polypeptide provided herein comprises an amino acid sequence having at least 95% identity with an amino acid sequence of any one of **SEQ ID NOS: 35-50,** or a fragment thereof. In some embodiments, the polypeptide comprises an amino acid sequence having at least 95% identity with an amino acid sequence of **SEQ ID NO: 51,** or a fragment thereof. In some embodiments, the polypeptide comprises an amino acid sequence having at least 95% identity with an amino acid sequence of **SEQ ID NO: 52,** or a fragment thereof. In some embodiments, the polypeptide comprises an amino acid sequence having at least 95% identity with an amino acid sequence of **SEQ ID NO: 53,** or a fragment thereof. In some embodiments, the polypeptide comprises an amino acid sequence having at least 95% identity with an amino acid sequence of **SEQ ID NO: 54,** or a fragment thereof. In some embodiments, the polypeptide provided herein comprises an amino acid sequence having at least 95% identity with an amino acid sequence of any one of **SEQ ID NOS: 55-59,** or a fragment thereof.

In some embodiments, the polypeptide provided herein is a polypeptide comprising an amino acid sequence of any one of **SEQ ID NOS: 60-61,** or a fragment thereof. In some embodiments, the polypeptide comprises an amino acid sequence having at least 80, 81, 82, 83, 84, 85, 86, 87, 88, 89, 90, 91, 92, 93, 94, 95, 96, 97, 98, or 99% identity with an amino acid sequence of any one of **SEQ ID NOS: 60-61,** or a fragment thereof.

In some embodiments, four or more polypeptides described herein are covalently attached to a polymeric backbone to form a multimer. In some embodiments, the polymeric backbone is dextran or polyethylene glycol. In some embodiments, each of the polypeptides is attached to the polymeric backbone via a terminal cysteine residue on each of the polypeptides. In some embodiments, each of the polypeptides is attached to the polymeric backbone via a biotinylation site on each of the polypeptides.

In some embodiments, four or more polypeptides are covalently attached to avidin to form a multimer.

### Protuberance and/or Cavity

In some embodiments, the polypeptide comprises an engineered protuberance. In some embodiments, the protuberance comprises one or more non-naturally occurring amino acid residues. In some embodiments, the protuberance comprises one or more naturally occurring amino acid residues. In some embodiments, the protuberance comprises one or more amino acids selected from phenylalanine, arginine, tyrosine, tryptophan, and cysteine. In some embodiments, the protuberance comprises a phenylalanine residue. In some embodiments, the protuberance comprises an arginine residue. In some embodiments, the protuberance comprises a tyrosine residue. In some embodiments, the protuberance comprises a tryptophan residue. In some embodiments, the protuberance comprises a cysteine residue. In some embodiments, the protuberance comprises a cysteine residue and a tryptophan residue.

In some embodiments, the polypeptide comprises an engineered cavity. In some embodiments, the cavity comprises one or more non-naturally occurring amino acid residues. In some embodiments, the cavity comprises one or more naturally occurring amino acid residues. In some embodiments, the cavity comprises one or more amino acids selected from alanine, serine, threonine, valine, and cysteine. In some embodiments, the cavity comprises an alanine residue. In some embodiments, the cavity comprises a serine residue. In some embodiments, the cavity comprises a threonine residue. In some embodiments, the cavity comprises a valine residue. In some embodiments, the cavity comprises a cysteine residue. In some embodiments, the cavity comprises a cysteine residue, a serine residue, an alanine residue, and a valine residue.

In some embodiments, the protuberance or cavity are not located at an MHC class II domain on the polypeptide. In some embodiments, the protuberance or cavity are located at a C_{H}3 antibody constant domain on the polypeptide. In some embodiments, the protuberance is located at a C_{H}3 antibody constant domain on the polypeptide. In some embodiments, the cavity is located at a C_{H}3 antibody constant domain on the polypeptide.

In certain embodiments of the disclosure and not of the invention, the cavity comprises a nucleotide sequence with at least 80% identity to the nucleotide sequence set forth in **SEQ ID NO: 25.** In certain embodiments of the disclosure and not of the invention, the cavity comprises a nucleotide sequence with at least 85% identity to the nucleotide sequence set forth in **SEQ ID NO: 25.** In certain embodiments of the disclosure and not of the invention, the cavity comprises a nucleotide sequence with at least 90% identity to the nucleotide sequence set forth in **SEQ ID NO: 25.** In certain embodiments of the disclosure and not of the invention, the cavity comprises a nucleotide sequence with at least 95% identity to the nucleotide sequence set forth in **SEQ ID NO: 25.** In certain embodiments of the disclosure and not of the invention, the cavity comprises a nucleotide sequence with at least 98% identity to the nucleotide sequence set forth in **SEQ ID NO: 25.** In certain embodiments of the disclosure and not of the invention, the cavity comprises a nucleotide sequence set forth in **SEQ ID NO: 25.** In some embodiments of the disclosure and not of the invention, the cavity comprises a nucleotide sequence with at least 81, 82, 83, 84, 85, 86, 87, 88, 89, 90, 91, 92, 93, 94, 95, 96, 97, 98, or 99% identity to the nucleotide sequence set forth in **SEQ ID NO: 25.**

In certain embodiments of the disclosure, the cavity comprises an amino acid sequence with at least 80% identity to the amino acid sequence set forth in **SEQ ID NO: 51.** In certain embodiments of the disclosure, the cavity comprises an amino acid sequence with at least 85% identity to the amino acid sequence set forth in **SEQ ID NO: 51.** In certain embodiments, the cavity comprises an amino acid sequence with at least 90% identity to the amino acid sequence set forth in **SEQ ID NO: 51.** In certain embodiments, the cavity comprises an amino acid sequence with at least 95% identity to the amino acid sequence set forth in **SEQ ID NO: 51.** In certain embodiments, the cavity comprises an amino acid sequence with at least 98% identity to the amino acid sequence set forth in **SEQ ID NO: 51.** In certain embodiments, the cavity comprises an amino acid sequence set forth in **SEQ ID NO: 51.** In some embodiments of the disclosure, the cavity comprises a nucleotide sequence with at least 81, 82, 83, 84, 85, 86, 87, 88, 89, 90, 91, 92, 93, 94, 95, 96, 97, 98, or 99% identity to the amino acid sequence set forth in **SEQ ID NO: 51.**

In certain embodiments, the cavity is engineered into the C_{H}2 or C_{H}3 domain of an immunoglobulin molecule. In certain embodiments, the cavity is engineered into the C_{H}3 domain of an immunoglobulin molecule. In certain embodiments of the disclosure and not of the invention, the cavity is engineered into the C_{H}3 domain of an immunoglobulin molecule and comprises an amino acid sequence with at least 80% identity to the amino acid sequence set forth in **SEQ ID NO: 52.** In certain embodiments of the disclosure and not of the invention, the cavity is engineered into the C_{H}3 domain of an immunoglobulin molecule and comprises an amino acid sequence with at least 90% identity to the amino acid sequence set forth in **SEQ ID NO: 52.** In certain embodiments of the disclosure and not of the invention, the cavity is engineered into the C_{H}3 domain of an immunoglobulin molecule and comprises an amino acid sequence with at least 95% identity to the amino acid sequence set forth in **SEQ ID NO: 52.** In certain embodiments of the disclosure and not of the invention, the cavity is engineered into the C_{H}3 domain of an immunoglobulin molecule and comprises an amino acid sequence with at least 98% identity to the amino acid sequence set forth in **SEQ ID NO: 52.** In certain embodiments of the disclosure and not of the invention, the cavity is engineered into the C_{H}3 domain of an immunoglobulin molecule and comprises an amino acid sequence set forth in **SEQ ID NO: 52.** In some embodiments of the disclosure and not of the invention, the cavity is engineered into the C_{H}3 domain of an immunoglobulin molecule and comprises an amino acid sequence with at least 81, 82, 83, 84, 85, 86, 87, 88, 89, 90, 91, 92, 93, 94, 95, 96, 97, 98, or 99% identity to the amino acid sequence set forth in **SEQ ID NO: 52.**

In certain embodiments of the disclosure and not of the invention, the protuberance comprises nucleotide sequence with at least 80% identity to the nucleotide sequence set forth in **SEQ ID NO: 26.** In certain embodiments of the disclosure and not of the invention, the protuberance comprises a nucleotide sequence with at least 80% identity to the nucleotide sequence set forth in **SEQ ID NO: 26.** In certain embodiments of the disclosure and not of the invention, the protuberance comprises a nucleotide sequence with at least 90% identity to the nucleotide sequence set forth in **SEQ ID NO: 26.** In certain embodiments of the disclosure and not of the invention, the protuberance comprises a nucleotide sequence with at least 95% identity to the nucleotide sequence set forth in **SEQ ID NO: 26.** In certain embodiments of the disclosure and not of the invention, the protuberance comprises a nucleotide sequence with at least 98% identity to the nucleotide sequence set forth in **SEQ ID NO: 26.** In certain embodiments of the disclosure and not of the invention, the protuberance comprises a nucleotide sequence set forth in **SEQ ID NO: 26.** In some embodiments of the disclosure and not of the invention, the protuberance comprises a nucleotide sequence with at least 81, 82, 83, 84, 85, 86, 87, 88, 89, 90, 91, 92, 93, 94, 95, 96, 97, 98, or 99% identity to the nucleotide sequence set forth in **SEQ ID NO: 26.**

In certain embodiments of the disclosure, the protuberance comprises an amino acid sequence with at least 80% identity to the amino acid sequence set forth in **SEQ ID NO: 53.** In certain embodiments of the disclosure, the protuberance comprises an amino acid sequence with at least 80% identity to the amino acid sequence set forth in **SEQ ID NO: 53.** In certain embodiments, the protuberance comprises an amino acid sequence with at least 90% identity to the amino acid sequence set forth in **SEQ ID NO: 53.** In certain embodiments, the protuberance comprises an amino acid sequence with at least 95% identity to the amino acid sequence set forth in **SEQ ID NO: 53.** In certain embodiments, the protuberance comprises an amino acid sequence with at least 98% identity to the amino acid sequence set forth in **SEQ ID NO: 53.** In certain embodiments, the protuberance comprises an amino acid sequence set forth in **SEQ ID NO: 53.** In some embodiments of the disclosure, the protuberance comprises an amino acid sequence with at least 81, 82, 83, 84, 85, 86, 87, 88, 89, 90, 91, 92, 93, 94, 95, 96, 97, 98, or 99% identity to the amino acid sequence set forth in **SEQ ID NO: 53.**

In certain embodiments, the protuberance is engineered into the C_{H}2 or C_{H}3 domain of an immunoglobulin molecule. In certain embodiments, the protuberance is engineered into the C_{H}3 domain of an immunoglobulin molecule. In certain embodiments of the disclosure, the protuberance is engineered into the C_{H}3 domain of an immunoglobulin molecule and comprises an amino acid sequence with at least 80% identity to the amino acid sequence set forth in **SEQ ID NO: 54.** In certain embodiments of the disclosure, the protuberance is engineered into the C_{H}3 domain of an immunoglobulin molecule and comprises an amino acid sequence with at least 90% identity to the amino acid sequence set forth in **SEQ ID NO: 54.** In certain embodiments of the disclosure, the protuberance is engineered into the C_{H}3 domain of an immunoglobulin molecule and comprises an amino acid sequence with at least 95% identity to the amino acid sequence set forth in **SEQ ID NO: 54.** In certain embodiments of the disclosure, the protuberance is engineered into the C_{H}3 domain of an immunoglobulin molecule and comprises an amino acid sequence with at least 98% identity to the amino acid sequence set forth in **SEQ ID NO: 54.** In certain embodiments of the disclosure, the protuberance is engineered into the C_{H}3 domain of an immunoglobulin molecule and comprises an amino acid sequence set forth in **SEQ ID NO: 54.** In some embodiments of the disclosure, the protuberance is engineered into the C_{H}3 domain of an immunoglobulin molecule and comprises an amino acid sequence with at least 81, 82, 83, 84, 85, 86, 87, 88, 89, 90, 91, 92, 93, 94, 95, 96, 97, 98, or 99% identity to the amino acid sequence set forth in **SEQ ID NO: 54.**

A Protuberance and a cavity can be engineered by specific mutation in the C_{H}3 domain of an IgG₁fused to either an MHC class II alpha or beta domain. In certain embodiments, the protuberance comprise a S354C mutation (EU antibody numbering scheme) in the C_{H}3 region of an IgG₁ molecule. In certain embodiments, the protuberance comprise a T366W mutation (EU antibody numbering scheme) in the C_{H}3 region of an IgG₁ molecule. In certain embodiments, the protuberance comprise a S354C and T366W mutation (EU antibody numbering scheme) in the C_{H}3 region of an IgG₁ molecule. In certain embodiments, the protuberance comprise a S354C and T366W mutation (EU antibody numbering scheme) in the C_{H}3 region of an IgG₁ molecule. In certain embodiments, the protuberance consists of a S354C and T366W mutation (EU antibody numbering scheme) in the C_{H}3 region of an IgG₁ molecule. In certain embodiments, the cavity comprises a Y349C mutation (EU antibody numbering scheme) in the C_{H}3 region of an IgG₁ molecule. In certain embodiments, the cavity comprises a T366S mutation (EU antibody numbering scheme) in the C_{H}3 region of an IgG₁ molecule. In certain embodiments, the cavity comprises a L368A mutation (EU antibody numbering scheme) in the C_{H}3 region of an IgG₁ molecule. In certain embodiments, the cavity comprises a Y407V mutation (EU antibody numbering scheme) in the C_{H}3 region of an IgG₁ molecule. In certain embodiments, the cavity comprises a Y349C, T366S, L368A, andY407V mutation (EU antibody numbering scheme) in the C_{H}3 region of an IgG₁ molecule. In certain embodiments, the cavity consists of a Y349C, T366S, L368A, andY407V mutation (EU antibody numbering scheme) in the C_{H}3 region of an IgG₁ molecule.

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, an IgG C_{H}2 domain, and an IgG C_{H}3 domain, wherein the protuberance is formed by mutations in the IgG C_{H}3 domain and (ii) the second polypeptide comprises an MHC class II β1 domain, an MHC class II β2 domain, an IgG C_{H}2 domain, and an IgG C_{H}3 domain, wherein the cavity is formed by mutations in the IgG C_{H}3 domain.

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II β1 domain, an MHC class II β2 domain, an IgG C_{H}2 domain, and an IgG C_{H}3 domain, wherein the protuberance is formed by mutations in the IgG C_{H}3 domain; and (ii) the second polypeptide comprises an MHC class II α1 domain, an MHC class II α2 domain, an IgG C_{H}2 domain, and an IgG C_{H}3 domain, wherein the cavity is formed by mutations in the IgG C_{H}3 domain.

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, an IgG C_{H}2 domain, and an IgG C_{H}3 domain, wherein the protuberance is formed by mutations in the IgG C_{H}3 domain corresponding to S354C and T366W (EU numbering); and (ii) the second polypeptide comprises an MHC class II β1 domain, an MHC class II β2 domain, an IgG C_{H}2 domain, and an IgG C_{H}3 domain, wherein the cavity is formed by mutations in the IgG C_{H}3 domain corresponding to Y349C, T366S, L368A and Y407V (EU numbering).

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II β1 domain, an MHC class II β2 domain, an IgG C_{H}2 domain, and an IgG C_{H}3 domain, wherein the protuberance is formed by mutations in the IgG C_{H}3 domain corresponding to S354C and T366W (EU numbering); and (ii) the second polypeptide comprises an MHC class II α1 domain, an MHC class II α2 domain, an IgG C_{H}2 domain, and an IgG C_{H}3 domain, wherein the cavity is formed by mutations in the IgG C_{H}3 domain corresponding to Y349C, T366S, L368A and Y407V (EU numbering).

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide consists of an MHC class II α1 domain, an MHC class II α2 domain, an IgG C_{H}2 domain, and an IgG C_{H}3 domain, wherein the protuberance is formed by mutations in the IgG C_{H}3 domain and (ii) the second polypeptide consists of an MHC class II β1 domain, an MHC class II β2 domain, an IgG CH2 domain, and an IgG C_{H}3 domain, wherein the cavity is formed by mutations in the IgG C_{H}3 domain.

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide consists of an MHC class II β1 domain, an MHC class II β2 domain, an IgG C_{H}2 domain, and an IgG C_{H}3 domain, wherein the protuberance is formed by mutations in the IgG C_{H}3 domain; and (ii) the second polypeptide consists of an MHC class II α1 domain, an MHC class II α2 domain, an IgG C_{H}2 domain, and an IgG C_{H}3 domain, wherein the cavity is formed by mutations in the IgG C_{H}3 domain.

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide consists of an MHC class II α1 domain, an MHC class II α2 domain, an IgG C_{H}2 domain, and an IgG C_{H}3 domain, wherein the protuberance is formed by mutations in the IgG C_{H}3 domain corresponding to S354C and T366W (EU numbering); and (ii) the second polypeptide consists of an MHC class II β1 domain, an MHC class II β2 domain, an IgG C_{H}2 domain, and an IgG C_{H}3 domain, wherein the cavity is formed by mutations in the IgG C_{H}3 domain corresponding to Y349C, T366S, L368A and Y407V (EU numbering).

In one embodiment, the isolated heterodimers comprise at least one first polypeptide and at least one second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide consists of an MHC class II β1 domain, an MHC class II β2 domain, an IgG C_{H}2 domain, and an IgG C_{H}3 domain, wherein the protuberance is formed by mutations in the IgG C_{H}3 domain corresponding to S354C and T366W (EU numbering); and (ii) the second polypeptide consists of an MHC class II α1 domain, an MHC class II α2 domain, an IgG C_{H}2 domain, and an IgG C_{H}3 domain, wherein the cavity is formed by mutations in the IgG C_{H}3 domain corresponding to Y349C, T366S, L368A and Y407V (EU numbering).

### Methods of Preparation

Provided herein are methods of preparing a heterodimer described herein. This disclosure contemplates methods of producing heterodimers wherein the heterodimers comprise a first polypeptide comprising an MHC class II α1 domain and an MHC class II α2 domain, and a second polypeptide comprising an MHC class II β1 domain and an MHC class II β2 domain. In some embodiments, each polypeptide further comprises a C_{H}2 and C_{H}3 domain. In some embodiments, the C_{H}3 domain further comprises either an engineered knob (protuberance) or an engineered hole (cavity). If the knob is associated with the polypeptide comprising the MHC class II α1 and α2 domain, then the hole is associated with the polypeptide comprising the MHC class II β1 and β2 domain. The configuration may be switched with the knob associated with the polypeptide comprising the MHC class II β1 and β2 domain, and the hole associated with the polypeptide comprising MHC class II α1 and α2 domain. The heterodimer also comprises an immunologically relevant polypeptide associated in the binding groove that is formed between the MHC class II alpha and beta chains. The polypeptide may be a part of either of the first or second polypeptide chain associated therewith by a flexible amino acid linker.

In some embodiments of the disclosure, the polypeptides of the heterodimers are encoded by polynucleotides that are introduced into a eukaryotic cell line. In certain embodiments of the disclosure, the eukaryotic ell line is a mammalian cell line. In certain embodiments of the disclosure, the eukaryotic ell line is a CHO cell line (Chinese hamster ovary). The polynucleotide may be introduced by methods known in the art such as viral transduction (retroviral or lentiviral) or by producing stable cell line which comprises a copy of the polynucleotide integrated into the genome. After expression the heterodimers are assembled in the cell and secreted into the culture medium. At this stage the culture medium is subjected to at least one further purification step. In certain embodiments of the disclosure, this purification step comprises any one or more of centrifugation, ultracentrifugation, dialysis, filtration, chromatography, or column chromatography. In certain embodiments of the disclosure, since the polypeptides comprise a C_{H}2 and C_{H}3 domain of a human immunoglobulin, then the chromatography step comprises affinity chromatography using Protein A, Protein G, Protein L, or any combination thereof. In certain embodiments of the disclosure, the chromatography step comprises affinity chromatography using Protein A. In certain embodiments of the disclosure, the chromatography step comprises affinity chromatography using Protein G. In certain embodiments of the disclosure, the chromatography step comprises affinity chromatography using protein A/G. In certain embodiments of the disclosure, the chromatography step consists of affinity chromatography using Protein A. In certain embodiments of the disclosure, the chromatography step consists of affinity chromatography using Protein G. In certain embodiments of the disclosure, the chromatography step consists of affinity chromatography using protein A/G. In certain embodiments of the disclosure, the chromatography step does not utilize an affinity reagent other than Protein A, Protein G, Protein L or a combination thereof (this does not include non-affinity based chromatography steps such as desalting or size exclusion chromatography).

In some embodiments of the disclosure, are methods of preparing a heterodimer comprising a first polypeptide and a second polypeptide, wherein the first polypeptide and the second polypeptide meet at an interface, wherein the interface of the first polypeptide comprises an engineered protuberance which is positionable in an engineered cavity in the interface of the second polypeptide; and (i) the first polypeptide comprises an MHC class II α1 domain, an MHC class II α2 domain, or a combination thereof; and the second polypeptide comprises an MHC class II β1 domain, an MHC class II β2 domain, or a combination thereof; or (ii) the first polypeptide comprises an MHC class II β1 domain, an MHC class II β2 domain, or a combination thereof; and the second polypeptide comprises an MHC class II α1 domain, an MHC class II α2 domain, or a combination thereof; comprising the steps of: (a) culturing a host cell comprising nucleic acid encoding the first polypeptide and second polypeptide including the interfaces thereof, wherein the nucleic acid encoding the interface of the first polypeptide has been altered from nucleic acid encoding an original interface of the first polypeptide to encode the protuberance or the nucleic acid encoding the interface of the second polypeptide has been altered from nucleic acid encoding an original interface of the second polypeptide to encode the cavity, or both, and wherein the culturing is such that the first polypeptide and second polypeptide are expressed; and (b) recovering the heterodimer from the host cell culture. In some embodiments, the nucleic acid encoding the first polypeptide has been altered from the original nucleic acid to encode the protuberance and the nucleic acid encoding the second polypeptide has been altered from the original nucleic acid to encode the cavity. In some embodiments of the disclosure, step (a) is preceded by a step wherein each of one or more nucleic acid encoding an original amino acid residue from the interface of the first polypeptide is replaced with nucleic acid encoding an import amino acid residue, wherein the protuberance comprises one or more import residues. In further embodiments, one or more import residues have a larger side chain volume than the original amino acid residue. In some embodiments of the disclosure, step (a) is preceded by a step wherein each of one or more nucleic acid encoding an original amino acid residue in the interface of the second polypeptide is replaced with nucleic acid encoding an import amino acid residue, wherein the cavity comprises one or more import residues. In further embodiments, one or more import residues have a smaller side chain volume than the original amino acid residue.

In some embodiments, the import residue is selected from phenylalanine, arginine, tyrosine, tryptophan, alanine, serine, threonine, valine, and cysteine. In some embodiments, the import residue is arginine. In some embodiments, the import residue is phenylalanine. In some embodiments, the import residue is tyrosine. In some embodiments, the import residue is tryptophan. In some embodiments, the import residue is alanine. In some embodiments, the import residue is serine. In some embodiments, the import residue is threonine. In some embodiments, the import residue is valine. In some embodiments, the import residue is not cysteine. In some embodiments, the import residue is cysteine. In some embodiments, the import residues are cysteine and tryptophan. In some embodiments, the import residues are cysteine, serine, alanine, and valine.

Also provided herein are methods of preparing a heterodimer-nanoparticle conjugate described herein. In some embodiments of the disclosure, the methods comprise linking at least one heterodimer described herein to a nanoparticle, wherein the nanoparticle is non-liposomal and/or has a solid core. In some embodiments of the disclosure, the methods comprise linking at least one heterodimer described herein to a nanoparticle, wherein the nanoparticle is non-liposomal and has a solid core. In some embodiments of the disclosure, the methods comprise linking at least one heterodimer described herein to a nanoparticle, wherein the nanoparticle is non-liposomal or has a solid core. In some embodiments of the disclosure, the methods comprise linking at least one heterodimer described herein to a nanoparticle, wherein the nanoparticle is non-liposomal and has a solid gold core. In some embodiments of the disclosure, the methods comprise linking at least one heterodimer described herein to a nanoparticle, wherein the nanoparticle is non-liposomal and has a solid iron oxide core. In some embodiments of the disclosure, the methods comprise linking at least one heterodimer described herein to a nanoparticle, wherein the nanoparticle is non-liposomal and has an iron oxide core; and the linking step comprises covalently linking the at least one heterodimer to the nanoparticle via a linker.

### Methods of treating diseases

Provided herein are methods of treating disease using compositions comprising, consisting of, or consisting essentially of heterodimers disclosed herein and/or heterodimer-nanoparticle conjugates disclosed herein. In some embodiments, the disease is an autoimmune disease or disorder.

The isolated heterodimers or isolated heterodimer-nanoparticle complexes of the current disclosure are useful for reprogramming/differentiating autoreactive T cells into T regulatory or TR1 cells. In certain embodiments, the TR1 cells express IL-10. In certain embodiments, the TR1 cells secrete IL-10. In certain embodiments, the TR1 cells express CD49b. In certain embodiments, the TR1 cells express LAG-3. T-cells that have these phenotypic characteristics are useful to treat inflammatory or autoimmune conditions of individuals.

"Autoimmune disease or disorder" includes diseases or disorders arising from and directed against an individual's own tissues or organs or manifestation thereof or a condition resulting there from. In one embodiment, it refers to a condition that results from, or is aggravated by, the production by T cells that are reactive with normal body tissues and antigens. Examples of autoimmune diseases or disorders include, but are not limited to arthritis (rheumatoid arthritis such as acute arthritis, chronic rheumatoid arthritis, gout or gouty arthritis, acute gouty arthritis, acute immunological arthritis, chronic inflammatory arthritis, degenerative arthritis, type II collagen-induced arthritis, infectious arthritis, Lyme arthritis, proliferative arthritis, psoriatic arthritis, Still's disease, vertebral arthritis, and juvenile-onset rheumatoid arthritis, osteoarthritis, arthritis chronica progrediente, arthritis deformans, polyarthritis chronica primaria, reactive arthritis, and ankylosing spondylitis), inflammatory hyperproliferative skin diseases, psoriasis (such as plaque psoriasis, gutatte psoriasis, pustular psoriasis, and psoriasis of the nails), atopy (including atopic diseases such as hay fever and Job's syndrome), dermatitis (including contact dermatitis, chronic contact dermatitis, exfoliative dermatitis, allergic dermatitis, allergic contact dermatitis, dermatitis herpetiformis, nummular dermatitis, seborrheic dermatitis, non-specific dermatitis, primary irritant contact dermatitis, and atopic dermatitis), x-linked hyper IgM syndrome, allergic intraocular inflammatory diseases, urticaria (such as chronic allergic urticaria and chronic idiopathic urticaria, including chronic autoimmune urticaria), myositis, polymyositis/dermatomyositis, juvenile dermatomyositis, toxic epidermal necrolysis, scleroderma (including systemic scleroderma), sclerosis (such as systemic sclerosis; multiple sclerosis (MS) such as spino-optical MS, primary progressive MS (PPMS), and relapsing remitting MS (RRMS); progressive systemic sclerosis, atherosclerosis, arteriosclerosis, sclerosis disseminata, and ataxic sclerosis), neuromyelitis optica spectrum disorder (NMO, also known as Devic's Disease or Devic's Syndrome), inflammatory bowel disease (IBD) (for example, Crohn's disease; autoimmune-mediated gastrointestinal diseases; colitis such as ulcerative colitis, colitis ulcerosa, microscopic colitis, collagenous colitis, colitis polyposa, necrotizing enterocolitis, and transmural colitis; and autoimmune inflammatory bowel disease), bowel inflammation, pyoderma gangrenosum, erythema nodosum, primary sclerosing cholangitis, respiratory distress syndrome (including adult or acute respiratory distress syndrome (ARDS)), meningitis, inflammation of all or part of the uvea, iritis, choroiditis, an autoimmune hematological disorder, rheumatoid spondylitis, rheumatoid synovitis, hereditary angioedema, cranial nerve damage as in meningitis, herpes gestationis, pemphigoid gestationis, pruritis scroti, autoimmune premature ovarian failure, sudden hearing loss due to an autoimmune condition, IgE-mediated diseases such as anaphylaxis and allergic and atopic rhinitis, encephalitis such as Rasmussen's encephalitis and limbic and/or brainstem encephalitis, uveitis (such as anterior uveitis, acute anterior uveitis, granulomatous uveitis, nongranulomatous uveitis, phacoantigenic uveitis, posterior uveitis, or autoimmune uveitis), glomerulonephritis (GN) with and without nephrotic syndrome (such as chronic or acute glomerulonephritis such as primary GN, immune-mediated GN, membranous GN (membranous nephropathy), idiopathic membranous GN or idiopathic membranous nephropathy, membrano- or membranous proliferative GN (MPGN), including Type I and Type II, and rapidly progressive GN, or proliferative nephritis), autoimmune polyglandular endocrine failure, balanitis including balanitis circumscripta plasmacellularis, balanoposthitis, erythema annulare centrifugum, erythema dyschromicum perstans, erythema multiform, granuloma annulare, lichen nitidus, lichen sclerosus et atrophicus, lichen simplex chronicus, lichen spinulosus, lichen planus, lamellar ichthyosis, epidermolytic hyperkeratosis, premalignant keratosis, pyoderma gangrenosum, allergic conditions and responses, allergic reaction, eczema (including allergic or atopic eczema, asteatotic eczema, dyshidrotic eczema, and vesicular palmoplantar eczema), asthma (such as asthma bronchiale, bronchial asthma, and auto-immune asthma), conditions involving infiltration of T cells and chronic inflammatory responses, immune reactions against foreign antigens such as fetal A-B-O blood groups during pregnancy, chronic pulmonary inflammatory disease, autoimmune myocarditis, leukocyte adhesion deficiency, lupus (including lupus nephritis, lupus cerebritis, pediatric lupus, non-renal lupus, extra-renal lupus, discoid lupus and discoid lupus erythematosus, alopecia lupus, systemic lupus erythematosus (SLE) such as cutaneous SLE or subacute cutaneous SLE, neonatal lupus syndrome (NLE), and lupus erythematosus disseminatus), Type I diabetes, Type II diabetes, and latent autoimmune diabetes in adults (or Type 1.5 diabetes). Also contemplated are immune responses associated with acute and delayed hypersensitivity mediated by cytokines and T-lymphocytes, sarcoidosis, granulomatosis (including lymphomatoid granulomatosis, Wegener's granulomatosis, or agranulocytosis), vasculitides (including vasculitis, large-vessel vasculitis (including polymyalgia rheumatica and giant cell (Takayasu's) arteritis), medium-vessel vasculitis (including Kawasaki's disease and polyarteritis nodosa/periarteritis nodosa), microscopic polyarteritis, immunovasculitis, CNS vasculitis, cutaneous vasculitis, hypersensitivity vasculitis, necrotizing vasculitis such as systemic necrotizing vasculitis, and ANCA-associated vasculitis (such as Churg-Strauss vasculitis or syndrome (CSS) and ANCA-associated small-vessel vasculitis)), temporal arteritis, aplastic anemia, autoimmune aplastic anemia, Coombs positive anemia, Diamond Blackfan anemia, hemolytic anemia, immune hemolytic anemia including autoimmune hemolytic anemia (AIHA), Addison's disease, autoimmune neutropenia, pancytopenia, leukopenia, diseases involving leukocyte diapedesis, CNS inflammatory disorders, Alzheimer's disease, Parkinson's disease, multiple organ injury syndrome (such as those secondary to septicemia, trauma, or hemorrhage), antigen-antibody complex-mediated diseases, anti-glomerular basement membrane disease, anti-phospholipid antibody syndrome, anti-phospholipid syndrome, allergic neuritis, Behcet's disease/syndrome, Castleman's syndrome, Goodpasture's syndrome, Reynaud's syndrome, Sjogren's syndrome, Stevens-Johnson syndrome, pemphigoid such as pemphigoid bullous and skin pemphigoid, pemphigus (including pemphigus vulgaris, pemphigus foliaceus, pemphigus mucus-membrane pemphigoid, and pemphigus erythematosus), autoimmune polyendocrinopathies, Reiter's disease or syndrome, thermal injury, preeclampsia, an immune complex disorder such as immune complex nephritis, antibody-mediated nephritis, polyneuropathies, chronic neuropathy such as IgM polyneuropathies or IgM-mediated neuropathy, autoimmune or immune-mediated thrombocytopenia such as idiopathic thrombocytopenic purpura (ITP) including chronic or acute ITP, acquired thrombocytopenic purpura, scleritis such as idiopathic cerato-scleritis, episcleritis, autoimmune disease of the testis and ovary including autoimmune orchitis and oophoritis, primary hypothyroidism, hypoparathyroidism, autoimmune endocrine diseases (including thyroiditis (such as autoimmune thyroiditis, Hashimoto's disease, chronic thyroiditis (Hashimoto's thyroiditis), or subacute thyroiditis), autoimmune thyroid disease, idiopathic hypothyroidism, or Grave's disease), polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis or encephalomyelitis allergica and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS), sensory neuropathy, multifocal motor neuropathy, Sheehan's syndrome, autoimmune hepatitis, chronic hepatitis, lupoid hepatitis, giant cell hepatitis, chronic active hepatitis or autoimmune chronic active hepatitis, lymphoid interstitial pneumonitis (LIP), bronchiolitis obliterans (non-transplant) vs NSIP, Guillain-Barre syndrome, Berger's disease (IgA nephropathy), idiopathic IgA nephropathy, linear IgA dermatosis, acute febrile neutrophilic dermatosis, subcorneal pustular dermatosis, transient acantholytic dermatosis, cirrhosis such as primary biliary cirrhosis and pneumonocirrhosis, autoimmune enteropathy syndrome, Celiac or Coeliac disease, celiac sprue (gluten enteropathy), refractory sprue, idiopathic sprue, cryoglobulinemia, amylotrophic lateral sclerosis (ALS; Lou Gehrig's disease), coronary artery disease, autoimmune ear disease such as autoimmune inner ear disease (AIED), autoimmune hearing loss, polychondritis such as refractory or relapsed or relapsing polychondritis, pulmonary alveolar proteinosis, Cogan's syndrome/nonsyphilitic interstitial keratitis, Bell's palsy, Sweet's disease/syndrome, rosacea autoimmune, zoster-associated pain, amyloidosis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, channelopathies of the CNS, autism, inflammatory myopathy, focal or segmental or focal segmental glomerulosclerosis (FSGS), endocrine ophthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases and chronic inflammatory demyelinating polyneuropathy, Dressler's syndrome, alopecia greata, alopecia totalis, CREST syndrome (calcinosis, Raynaud's phenomenon, esophageal dysmotility, sclerodactyly, and telangiectasia), male and female autoimmune infertility (e.g., due to anti-spermatozoan antibodies) mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrosing alveolitis, interstitial lung disease, transfusion reaction, leprosy, malaria, parasitic diseases such as leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, pulmonary fibrosis, idiopathic pulmonary fibrosis, cystic fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, iridocyclitis (acute or chronic), or Fuchs' cyclitis, Henoch-Schonlein purpura, human immunodeficiency virus (HIV) infection, SCID, acquired immune deficiency syndrome (AIDS), echovirus infection, sepsis, endotoxemia, pancreatitis, thyroxicosis, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, chorioiditis, giant cell polymyalgia, chronic hypersensitivity pneumonitis, keratoconjunctivitis sicca, epidemic keratoconjunctivitis, idiopathic nephritic syndrome, minimal change nephropathy, benign familial and ischemia-reperfusion injury, transplant organ reperfusion, retinal autoimmunity, joint inflammation, bronchitis, chronic obstructive airway/pulmonary disease, silicosis, aphthae, aphthous stomatitis, arteriosclerotic disorders, asperniogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensoneural hearing loss, haemoglobinuria paroxysmatica, hypogonadism, ileitis regionalis, leucopenia, mononucleosis infectiosa, traverse myelitis, primary idiopathic myxedema, nephrosis, ophthalmia symphatica, orchitis granulomatosa, pancreatitis, polyradiculitis acuta, pyoderma gangrenosum, Quervain's thyreoiditis, acquired splenic atrophy, non-malignant thymoma, vitiligo, toxic-shock syndrome, food poisoning, conditions involving infiltration of T cells, leukocyte-adhesion deficiency, immune responses associated with acute and delayed hypersensitivity mediated by cytokines and T-lymphocytes, diseases involving leukocyte diapedesis, multiple organ injury syndrome, antigen-antibody complex-mediated diseases, antiglomerular basement membrane disease, allergic neuritis, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, autoimmune polyglandular syndrome type I, adult-onset idiopathic hypoparathyroidism (AOIH), cardiomyopathy such as dilated cardiomyopathy, epidermolisis bullosa acquisita (EBA), hemochromatosis, myocarditis, nephrotic syndrome, primary sclerosing cholangitis, purulent or nonpurulent sinusitis, acute or chronic sinusitis, ethmoid, frontal, maxillary, or sphenoid sinusitis, an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils, anaphylaxis, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia syndrome, angiectasis, autoimmune disorders associated with collagen disease, rheumatism, neurological disease, lymphadenitis, reduction in blood pressure response, vascular dysfunction, tissue injury, cardiovascular ischemia, hyperalgesia, renal ischemia, cerebral ischemia, and disease accompanying vascularization, allergic hypersensitivity disorders, glomerulonephritides, reperfusion injury, ischemic re-perfusion disorder, reperfusion injury of myocardial or other tissues, lymphomatous tracheobronchitis, inflammatory dermatoses, dermatoses with acute inflammatory components, multiple organ failure, bullous diseases, renal cortical necrosis, acute purulent meningitis or other central nervous system inflammatory disorders, ocular and orbital inflammatory disorders, granulocyte transfusion-associated syndromes, cytokine-induced toxicity, narcolepsy, acute serious inflammation, chronic intractable inflammation, pyelitis, endarterial hyperplasia, peptic ulcer, valvulitis, emphysema, alopecia areata, adipose tissue inflammation/diabetes type II, obesity associated adipose tissue inflammation/insulin resistance, and endometriosis.

In some embodiments, the autoimmune disorder or disease may include, but is not limited to, diabetes mellitus Type I and Type II, pre-diabetes, transplantation rejection, multiple sclerosis, a multiple-sclerosis related disorder, premature ovarian failure, scleroderma, Sjogren's disease/syndrome, lupus, vitiligo, alopecia (baldness), polyglandular failure, Grave's disease, hypothyroidism, polymyosititis, pemphigus, Crohn's disease, colitis, autoimmune hepatitis, hypopituitarism, myocarditis, Addison's disease, autoimmune skin diseases, uveitis, pernicious anemia, hypoparathyroidism, and/or rheumatoid arthritis. Other indications of interest include, but are not limited to, asthma, allergic asthma, primary biliary cirrhosis, cirrhosis, Neuromyelitis Optica Spectrum Disorder (Devic's disease, opticospinal multiple sclerosis (OSMS)), Pemphigus vulgaris, inflammatory bowel disease (IBD), arthritis, Rheumatoid arthritis, systemic lupus erythematosus (SLE), Celiac disease, psoriasis, autoimmune cardiomyopathy, idiopathic dilated cardiomyopathy (IDCM), a Myasthyenia Gravis, Uveitis, Ankylosing Spondylitis, Immune Mediated Myopathies, prostate cancer, anti-phospholipid syndrome (ANCA+), atherosclerosis, dermatomyositis, chronic obstructive pulmonary disease (COPD), emphysema, spinal cord injury, traumatic injury, a tobacco-induced lung destruction, ANCA-associated vasculitis, psoriasis, sclerosing cholangitis, primary sclerosing cholangitis, and diseases of the central and peripheral nervous systems.

In some embodiments, the autoimmune disorder or disease may include, but is not limited to, type I diabetes, multiple sclerosis, Celiac Disease, primary biliary cirrhosis, pemphigus, pemphigus folliaceus, pemphigus vulgaris, neuromyelitis optica spectrum disorder, arthritis (including rheumatoid arthritis), allergic asthma, inflammatory bowel disease (including Crohn's disease and ulcerative colitis), systemic lupus erythematosus, atherosclerosis, chronic obstructive pulmonary disease, emphysema, psoriasis, autoimmune hepatitis, uveitis, Sjogren's Syndrome, scleroderma, anti-phospholipid syndrome, ANCA-associated vasculitis, and Stiff Man Syndrome. In a further aspect, the disease-relevant antigen is a tumor- or cancer-relevant antigen.

In certain embodiments, the isolated heterodimers or isolated heterodimer-nanoparticle complexes of the current disclosure are included in a pharmaceutical composition comprising one or more pharmaceutically acceptable excipients, carriers, and diluents. In certain embodiments, the isolated heterodimers or isolated heterodimer-nanoparticle complexes of the current disclosure are administered suspended in a sterile solution. In certain embodiments, the solution comprises 0.9% NaCl. In certain embodiments, the solution further comprises one or more of: buffers, for example, acetate, citrate, histidine, succinate, phosphate, bicarbonate and hydroxymethylaminomethane (Tris); surfactants, for example, polysorbate 80 (Tween 80), polysorbate 20 (Tween 20), and poloxamer 188; polyol/disaccharide/polysaccharides, for example, glucose, dextrose, mannose, mannitol, sorbitol, sucrose, trehalose, and dextran 40; amino acids, for example, glycine or arginine; antioxidants, for example, ascorbic acid, methionine; or chelating agents, for example, EGTA or EGTA. In certain embodiments, the isolated heterodimers or isolated heterodimer-nanoparticle complexes of the current disclosure are shipped/stored lyophilized and reconstituted before administration. In certain embodiments, the lyophilized isolated heterodimers or isolated heterodimer-nanoparticle complex formulations comprise a bulking agent such as mannitol, sorbitol, sucrose, trehalose, or dextran 40. The lyophilized formulation can be contained in a vial comprised of glass. The isolated heterodimers or isolated heterodimer-nanoparticle complexes, when formulated, whether reconstituted or not, can be buffered at a certain pH, generally less than 7.0. In certain embodiments, the pH can be between 4.5 and 6.5, 4.5 and 6.0, 4.5 and 5.5, 4.5 and 5.0, or 5.0 and 6.0. In certain embodiments, isolated heterodimers or isolated heterodimer-nanoparticle complexes can be formulated for intravenous injection. In certain embodiments, isolated heterodimers or isolated heterodimer-nanoparticle complexes can be formulated for oral ingestion. In certain embodiments, isolated heterodimers or isolated heterodimer-nanoparticle complexes can be formulated for parenteral, intramuscular, or intra tissue injection. In certain embodiments, isolated heterodimers or isolated heterodimer-nanoparticle complexes can be formulated and/or administered without any immunological adjuvant or other compound or polypeptide intended to increase or decrease an immune response.

Also provided herein are methods of detecting and/or monitoring a population of immune cells, preferably T cells comprising administering a labeled antigen-MHC complex where a subject has received heterodimers disclosed herein and/or heterodimer-nanoparticle conjugates disclosed herein.

In certain aspects of the disclosure, provided herein are methods to detect a population of T_{R}1 cells and/or effector T cells in an antigen specific manner in a subject that has received heterodimers disclosed herein and/or heterodimer-nanoparticle conjugates disclosed herein. The method comprises, alternatively consists of, or yet further consists essentially of, contacting a sample suspected of comprising the T_{R}1 cells with an effective amount of labeled pMHC complex to form a multimer complex, and detecting any multimer complex, thereby detecting the population of T_{R}1 cells. In some embodiments, the method further comprises, alternatively further consists of, or yet further consists essentially of staining any T cell population using a labeled multimer complex. In some embodiments, the step of detecting the population of T_{R}1 cells comprises flow cytometry to detect any multimer complex. In some embodiments of the disclosure and not of the invention, the method further comprises, or alternatively consists of, or yet further consists essentially of administering the complex or composition to the subject.

In certain aspects of the disclosure, provided herein are methods to detect a population of T_{R}1 cells and/or effector T cells in an antigen specific manner in a subject that has received heterodimers disclosed herein and/or heterodimer-nanoparticle conjugates disclosed herein. The method comprises, alternatively consists of, or yet further consists essentially of any one of the following assays: cytokine ELISPOT assay, a multimer-guided epitope analysis, or a multimer-pull-down assay. In some embodiments of the disclosure and not of the invention, the method further comprises, alternatively further consists of, or yet further consists essentially of administering heterodimers disclosed herein and/or heterodimer-nanoparticle conjugates disclosed herein.

In other aspects of the disclosure, provided herein are methods to monitor the expansion of a population of antigen-specific T_{R}1 and/or effector T cells in a subject. The method comprises, alternatively consists of, or yet further consists essentially of: a) administering to a subject an effective amount of heterodimers disclosed herein and/or heterodimer-nanoparticle conjugates disclosed herein, wherein the disease-relevant antigen of the pMHC complex is selected to expand the antigen-specific T_{R}1 and/or effector T cells; b) isolating a suitable sample from the subject suspected of containing the population; c) contacting the sample with an effective amount of labeled pMHC complex to form a multimer complex, and detecting any multimer complex; and d) quantifying the number of antigen-specific T_{R}1 and/or effector T cells in the population. In some embodiments, the method further comprises, alternatively further consists of, or yet further consists essentially of staining any multimer complex. In some embodiments, the step of quantifying the number of antigen-specific T_{R}1 and/or effector T cells comprises flow cytometry and/or ELISA. In some embodiments of the disclosure and not of the invention, the method further comprises, alternatively further consists of, or yet further consists essentially of administering heterodimers disclosed herein and/or heterodimer-nanoparticle conjugates disclosed herein.

There are many types of immunoassays that can be implemented. Immunoassays encompassed by the present disclosure include, but are not limited to, those described in U.S. Patent 4,367,110 (double monoclonal antibody sandwich assay) and U.S. Patent 4,452,901 (western blot). Other assays include immunoprecipitation of labeled ligands and immunocytochemistry, both *in vitro* and *in vivo.*

One method for quantifying the number of circulating antigen-specific immune cells is the tetramer assay. In this assay, a specific epitope is bound to synthetic multimeric forms of fluorescently labeled MHC molecules. Since immune cells recognize antigens in the form of short peptides bound to MHC molecules, cells with the appropriate T cell receptor will bind to the labeled tetramers and can be quantified by flow cytometry. Although this method is less time-consuming than an ELISPOT assay, the multimer assay measures only binding, not function. Not all cells that bind a particular antigen necessarily become activated. However, correlation between ELISPOT, multimer, and cytotoxicity assays has been demonstrated.

Immunoassays generally are binding assays. Certain immunoassays, including the various types of enzyme linked immunosorbent assays (ELISAs), radioimmunoassays (RIA), or bead based assays, such as Luminex^{®} technology, are known in the art. Immunohistochemical detection using tissue sections is also useful.

In one example of ELISA, the antibodies or antigens are immobilized on a selected surface, such as a well in a polystyrene microtiter plate, dipstick, or column support. Then, a test composition suspected of containing the desired antigen or antibody, such as a clinical sample, is added to the wells. After binding and washing to remove non-specifically bound immune complexes, the bound antigen or antibody may be detected. Detection is generally achieved by the addition of another antibody specific for the desired antigen or antibody that is linked to a detectable label. This type of ELISA is known as a "sandwich ELISA." Detection also may be achieved by the addition of a second antibody specific for the desired antigen, followed by the addition of a third antibody that has binding affinity for the second antibody, with the third antibody being linked to a detectable label. Variations on ELISA techniques are known to those of skill in the art.

Competition ELISAs are also possible in which test samples compete for binding with known amounts of labeled antigens or antibodies. The amount of reactive species in the unknown sample is determined by mixing the sample with the known labeled species before or during incubation with coated wells. The presence of reactive species in the sample acts to reduce the amount of labeled species available for binding to the well and thus reduces the ultimate signal.

Irrespective of the format employed, ELISAs have certain features in common, such as coating, incubating or binding, washing to remove non-specifically bound species, and detecting the bound immune complexes.

Antigen or antibodies may also be linked to a solid support, such as in the form of plate, beads, dipstick, membrane, or column matrix, and the sample to be analyzed is applied to the immobilized antigen or antibody. In coating a plate with either antigen or antibody, one will generally incubate the wells of the plate with a solution of the antigen or antibody, either overnight or for a specified period. The wells of the plate will then be washed to remove incompletely-adsorbed material. Any remaining available surfaces of the wells are then "coated" with a nonspecific protein that is antigenically neutral with regard to the test antisera. These include bovine serum albumin (BSA), casein, and solutions of milk powder. The coating allows for blocking of nonspecific adsorption sites on the immobilizing surface and thus reduces the background caused by nonspecific binding of antisera onto the surface.

In ELISAs, it is more customary to use a secondary or tertiary detection means rather than a direct procedure. Thus, after binding of the antigen or antibody to the well, coating with a non reactive material to reduce background, and washing to remove unbound material, the immobilizing surface is contacted with the clinical or biological sample to be tested under conditions effective to allow immune complex (antigen/antibody) formation. Detection of the immune complex then requires a labeled secondary binding ligand or antibody, or a secondary binding ligand or antibody in conjunction with a labeled tertiary antibody or third binding ligand.

Additionally, flow cytometry may be used to detect and quantitate particular cell subtypes according to cell surface markers. Common means of detection and quantitation via flow cytometry include the use of fluorescent labeled beads that bind to cell surface markers specific to each immune cell subtype, *e.g.* CD 4 specific beads, to select for CD 4+ T cells, *etc.*

Compositions described herein may be conventionally administered parenterally, by injection, for example, intravenously, subcutaneously, or intramuscularly. Intravenously administered compositions may include stabilizers, excipients, and preservatives. pMHC-nanoparticle compositions may be suspended in sterile saline, buffered saline, or phosphate buffered saline. Additionally, in certain embodiments, intravenous formulations comprise dextrose, glucose, mannitol, pH buffers, or sodium bicarbonate. Additional formulations which are suitable for other modes of administration include oral formulations. Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders and contain about 10% to about 95% of active ingredient, preferably about 25% to about 70%. The preparation of an aqueous composition that contains an antigen-MHC-nanoparticle complex that modifies the subject's immune condition will be known to those of skill in the art in light of the present disclosure. In certain embodiments, a composition may be inhaled (e.g., U.S. Patent No. 6,651,655). In one embodiment, the antigen-heterodimer-nanoparticle complex (*i.e.,* antigen-pMHC-NP complex) is administered systemically. In specific embodiments, the pMHC-NP complex described herein or the compositions comprising a plurality of pMHC-NP complexes described herein are administered intravenously.

### EXAMPLES

### Example 1. Expression of peptide-MHC (pMHC) complexes with a leucine zipper.

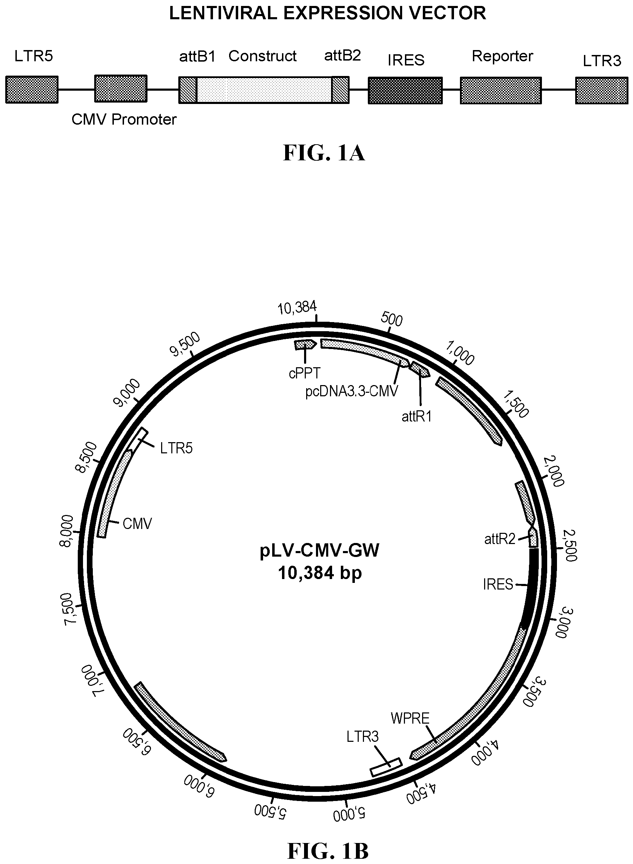

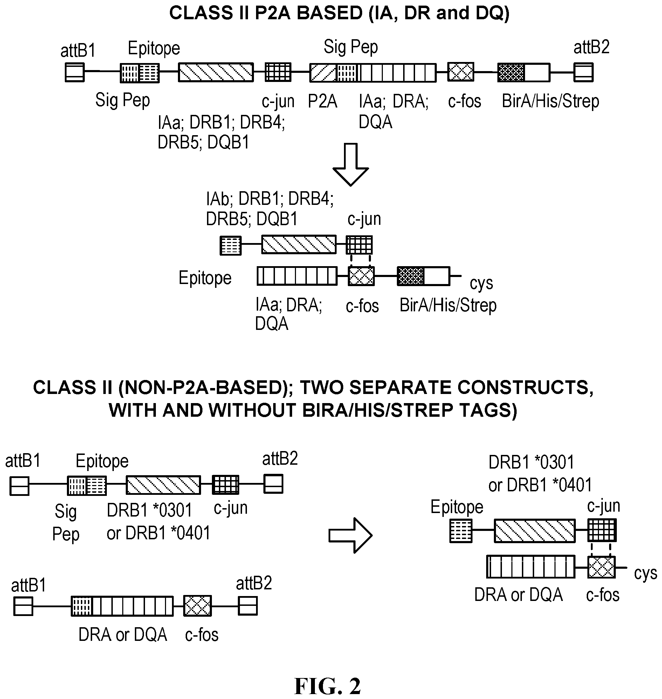

To express pMHC complexes in CHO-S cells, DNA fragments encoding a pMHC complex (in different configurations, see **FIG. 2**) are cloned into the pLV/CMV-GW lentiviral expression vector (**FIGS. 1A** and **1B**). Both the alpha and beta chains of the pMHC complex are encoded by a single ORF, either as two separate chains separated by a P2A ribosomal skipping sequence (**FIG. 2****, top**) or as two separate chains cloned in two different vectors (**FIG. 2****, bottom**). The pMHC-encoding fragment is cloned upstream of an IRES-EGFP cassette. The expressed beta chain product consists of a leader sequence, the epitope sequence, a GS linker, followed by the extracellular domain of the beta chain and a C-Jun fragment (40 a.a.). The expressed alpha chain contains a leader sequence, the extracellular domain of the alpha chain, a C-fos fragment (40 a.a.), a BirA biotinylation site (14 a.a.) and a 6X Histidine and/or Strep tags with a C-terminal cysteine. A preferred vector design (**FIG. 2****, bottom**) encodes both chains on separate plasmids.

## Claims

1. A composition comprising at least one isolated heterodimer conjugated to a nanoparticle,
wherein the nanoparticle is non-liposomal and/or has a solid core and
wherein the isolated heterodimer comprises at least one first polypeptide, at least one second polypeptide and an autoimmune disease-relevant antigen, wherein the first polypeptide and the second polypeptide meet at an interface, wherein the interface of the first polypeptide comprises an engineered protuberance which is positionable in an engineered cavity in the interface of the second polypeptide,
wherein the engineered protuberance comprises an amino acid sequence that is at least 90% identical to the amino acid sequence of SEQ ID NO: 53,
wherein the engineered cavity comprises an amino acid sequence that is at least 90% identical to the amino acid sequence of SEQ ID NO: 51; wherein
(i) the first polypeptide comprises a soluble MHC class II α1 domain, a soluble MHC class II α2 domain, or a combination thereof; and the second polypeptide comprises a soluble MHC class II β1 domain, a soluble MHC class II β2 domain, or a combination thereof; or
(ii) the first polypeptide comprises a soluble MHC class II β1 domain, a soluble MHC class II β2 domain, or a combination thereof; and the second polypeptide comprises a soluble MHC class II α1 domain, a soluble MHC class II α2 domain, or a combination thereof; and
wherein the autoimmune disease-relevant antigen is a polypeptide which is at least 11 amino acids in length connected to the MHC class II α1 domain or the MHC class II β1 domain by a flexible linker; and
wherein neither the first polypeptide nor the second polypeptide comprises a leucine zipper domain, or an extraneous biotinylation site or purification tag.

2. The composition of claim 1, wherein the engineered protuberance comprises an amino acid sequence that is at least 95% identical to the amino acid sequence of SEQ ID NO: 53 or SEQ ID NO: 54.

3. The composition of claim 2, wherein the engineered protuberance comprises an amino acid sequence that is at least 98% identical to the amino acid sequence of SEQ ID NO: 53 or SEQ ID NO: 54.

4. The composition of claim 3, wherein the engineered protuberance comprises an amino acid sequence that is the amino acid sequence of SEQ ID NO: 53 or SEQ ID NO. 54.

5. The composition of any one of claims 1 to 4, wherein the engineered cavity comprises an amino acid sequence that is at least 95% identical to the amino acid sequence of SEQ ID NO: 51 or SEQ ID NO. 52.

6. The composition of claim 5, wherein the engineered cavity comprises an amino acid sequence that is at least 98% identical to the amino acid sequence of SEQ ID NO: 51 or SEQ ID NO. 52.

7. The composition of claim 6, wherein the engineered cavity comprises an amino acid sequence that is the amino acid sequence of SEQ ID NO: 51 or SEQ ID NO. 52.

8. The composition of any one of claims 1 to 7, wherein the solid core is a gold, iron, or iron oxide core.

9. The composition of any one of claims 1 to 8, wherein the solid core has a diameter of less than 100 nanometers.

10. The composition of any one of claims 1 to 9, wherein the at least one heterodimer is covalently linked to the nanoparticle, optionally through a linker comprising polyethylene glycol (PEG).

11. The composition of claim 10, wherein the polyethylene glycol is functionalized with maleimide.

12. The composition of claim 10 or claim 11, wherein the polyethylene glycol is less than 5kD.

13. A pharmaceutical composition comprising the composition of any one of claims 1 to 12, and a pharmaceutical excipient, stabilizer, or diluent.

14. The pharmaceutical composition of claim 13, for use in a method of treating an autoimmune disease or inflammatory condition.

## Patentansprüche

1. Zusammensetzung, umfassend mindestens ein isoliertes Heterodimer, das an ein Nanopartikel konjugiert ist,
wobei das Nanopartikel nicht liposomal ist und/oder einen festen Kern aufweist und
wobei das isolierte Heterodimer mindestens ein erstes Polypeptid, mindestens ein zweites Polypeptid und ein für Autoimmunkrankheiten relevantes Antigen umfasst, wobei das erste Polypeptid und das zweite Polypeptid an einer Grenzfläche aufeinandertreffen, wobei die Grenzfläche des ersten Polypeptids eine konstruierte Protuberanz umfasst, die in einer konstruierten Kavität in der Grenzfläche des zweiten Polypeptids positionierbar ist,
wobei die konstruierte Protuberanz eine Aminosäuresequenz umfasst, die zu mindestens 90 % mit der Aminosäuresequenz von SEQ ID Nr. 53 identisch ist,
wobei die konstruierte Kavität eine Aminosäuresequenz umfasst, die zu mindestens 90 % mit der Aminosäuresequenz von SEQ ID Nr. 51 identisch ist; wobei
(i) das erste Polypeptid eine lösliche α1-Domäne der MHC-Klasse II, eine lösliche α2-Domäne der MHC-Klasse II oder eine Kombination davon umfasst; und das zweite Polypeptid eine lösliche β1-Domäne der MHC-Klasse II, eine lösliche β2-Domäne der MHC-Klasse II oder eine Kombination davon umfasst; oder
(ii) das erste Polypeptid eine lösliche β1-Domäne der MHC-Klasse II, eine lösliche β2-Domäne der MHC-Klasse II oder eine Kombination davon umfasst; und das zweite Polypeptid eine lösliche α1-Domäne der MHC-Klasse II, eine lösliche α2-Domäne der MHC-Klasse II oder eine Kombination davon umfasst; und
wobei das für Autoimmunkrankheiten relevante Antigen ein Polypeptid ist, das mindestens 11 Aminosäuren lang ist, durch einen flexiblen Linker mit der α1-Domäne der MHC-Klasse II oder der β1-Domäne der MHC-Klasse II verbunden; und
wobei weder das erste Polypeptid noch das zweite Polypeptid eine Leucin-Zipper-Domäne oder eine freme Biotinylierungsstelle oder ein fremdes Reinigungstag umfasst.

2. Zusammensetzung nach Anspruch 1, wobei die konstruierte Protuberanz eine Aminosäuresequenz umfasst, die zu mindestens 95 % mit der Aminosäuresequenz von SEQ ID Nr. 53 oder SEQ ID Nr. 54 identisch ist.

3. Zusammensetzung nach Anspruch 2, wobei die konstruierte Protuberanz eine Aminosäuresequenz umfasst, die zu mindestens 98 % mit der Aminosäuresequenz von SEQ ID Nr. 53 oder SEQ ID Nr. 54 identisch ist.

4. Zusammensetzung nach Anspruch 3, wobei die konstruierte Protuberanz eine Aminosäuresequenz umfasst, welche die Aminosäuresequenz von SEQ ID Nr. 53 oder SEQ ID Nr. 54 ist.

5. Zusammensetzung nach einem der Ansprüche 1 bis 4, wobei die konstruierte Kavität eine Aminosäuresequenz umfasst, die zu mindestens 95 % mit der Aminosäuresequenz von SEQ ID Nr. 51 oder SEQ ID Nr. 52 identisch ist.

6. Zusammensetzung nach Anspruch 5, wobei die konstruierte Kavität eine Aminosäuresequenz umfasst, die zu mindestens 98 % mit der Aminosäuresequenz von SEQ ID Nr. 51 oder SEQ ID Nr. 52 identisch ist.

7. Zusammensetzung nach Anspruch 6, wobei die konstruierte Kavität eine Aminosäuresequenz umfasst, welche die Aminosäuresequenz von SEQ ID Nr. 51 oder SEQ ID Nr. 52 ist.

8. Zusammensetzung nach einem der Ansprüche 1 bis 7, wobei der feste Kern ein Gold-, Eisen- oder Eisenoxidkern ist.

9. Zusammensetzung nach einem der Ansprüche 1 bis 8, wobei der feste Kern einen Durchmesser von weniger als 100 Nanometer aufweist.

10. Zusammensetzung nach einem der Ansprüche 1 bis 9, wobei das mindestens eine Heterodimer kovalent mit dem Nanopartikel verknüpft ist, gegebenenfalls durch einen Linker, umfassend Polyethylenglykol (PEG).

11. Zusammensetzung nach Anspruch 10, wobei das Polyethylenglykol mit Maleinimid funktionalisiert ist.

12. Zusammensetzung nach Anspruch 10 oder Anspruch 11, wobei das Polyethylenglykol weniger als 5 kD beträgt.

13. Pharmazeutische Zusammensetzung, umfassend die Zusammensetzung nach einem der Ansprüche 1 bis 12 und einen pharmazeutischen Hilfsstoff, einen pharmazeutischen Stabilisator oder ein pharmazeutisches Verdünnungsmittel.

14. Pharmazeutische Zusammensetzung nach Anspruch 13 zur Verwendung in einem Verfahren zur Behandlung einer Autoimmunkrankheit oder eines Entzündungszustands.

## Revendications

1. Composition comprenant au moins un hétérodimère isolé conjugué à une nanoparticule,
dans laquelle la nanoparticule est non liposomale et/ou possède un noyau solide et
dans laquelle l'hétérodimère isolé comprend au moins un premier polypeptide, au moins un second polypeptide et un antigène pertinent pour une maladie auto-immune, dans laquelle le premier polypeptide et le second polypeptide se rencontrent au niveau d'une interface, dans laquelle l'interface du premier polypeptide comprend une protubérance artificielle qui peut être positionnée dans une cavité artificielle dans l'interface du second polypeptide,
dans laquelle la protubérance modifiée comprend une séquence d'acides aminés qui est au moins à 90 % identique à la séquence d'acides aminés de SEQ ID N° 53,
dans laquelle la cavité conçue comprend une séquence d'acides aminés qui est au moins à 90 % identique à la séquence d'acides aminés de SEQ ID N° 51 ; dans laquelle
(i) le premier polypeptide comprend un domaine α1 soluble du CMH de classe II, un domaine α2 soluble du CMH de classe II ou une combinaison de ceux-ci ; et le second polypeptide comprend un domaine β1 soluble du CMH de classe II, un domaine β2 soluble du CMH de classe II ou une combinaison de ceux-ci ; ou
(ii) le premier polypeptide comprend un domaine β1 soluble du CMH de classe II, un domaine β2 soluble du CMH de classe II ou une combinaison de ceux-ci ; et le second polypeptide comprend un domaine α1 soluble du CMH de classe II, un domaine α2 soluble du CMH de classe II ou une combinaison de ceux-ci ; et
dans laquelle l'antigène pertinent pour la maladie auto-immune est un polypeptide qui a au moins 11 acides aminés de longueur connecté au domaine α1 de classe II du CMH ou au domaine β1 de classe II du CMH par un lieur flexible ; et
dans laquelle ni le premier polypeptide ni le second polypeptide ne comprennent un domaine de fermeture éclair à leucine, ni un site de biotinylation étranger ni une étiquette de purification.

2. Composition selon la revendication 1, dans laquelle la protubérance modifiée comprend une séquence d'acides aminés qui est au moins à 95 % identique à la séquence d'acides aminés de SEQ ID N° 53 ou SEQ ID N° 54.

3. Composition selon la revendication 2, dans laquelle la protubérance modifiée comprend une séquence d'acides aminés qui est au moins à 98 % identique à la séquence d'acides aminés de SEQ ID N° 53 ou SEQ ID N° 54.

4. Composition selon la revendication 3, dans laquelle la protubérance modifiée comprend une séquence d'acides aminés qui est la séquence d'acides aminés de SEQ ID N° 53 ou SEQ ID N° 54.

5. Composition selon l'une quelconque des revendications 1 à 4, dans laquelle la cavité modifiée comprend une séquence d'acides aminés qui est au moins à 95 % identique à la séquence d'acides aminés de SEQ ID N° 51 ou SEQ ID N° 52.

6. Composition selon la revendication 5, dans laquelle la protubérance modifiée comprend une séquence d'acides aminés qui est au moins à 98 % identique à la séquence d'acides aminés de SEQ ID N° 51 ou SEQ ID N° 52.

7. Composition selon la revendication 6, dans laquelle la protubérance modifiée comprend une séquence d'acides aminés qui est la séquence d'acides aminés de SEQ ID N° 51 ou SEQ ID N° 52.

8. Composition selon l'une quelconque des revendications 1 à 7, dans laquelle le noyau solide est un noyau d'or, de fer ou d'oxyde de fer.

9. Composition selon l'une quelconque des revendications 1 à 8, dans laquelle le noyau solide a un diamètre inférieur à 100 nanomètres.

10. Composition selon l'une quelconque des revendications 1 à 9, dans laquelle l'au moins un hétérodimère est lié de manière covalente à la nanoparticule, éventuellement par l'intermédiaire d'un lieur comprenant du polyéthylène glycol (PEG).

11. Composition selon la revendication 10, dans laquelle le polyéthylène glycol est fonctionnalisé avec du maléimide.

12. Composition selon la revendication 10 ou la revendication 11, dans laquelle le polyéthylène glycol est inférieur à 5 kD.

13. Composition pharmaceutique comprenant la composition selon l'une quelconque des revendications 1 à 12, et un excipient, un stabilisateur ou un diluant pharmaceutique.

14. Composition pharmaceutique selon la revendication 13, pour utilisation dans un procédé de traitement d'une maladie auto-immune ou d'un état inflammatoire.
